# EUROPEAN PATENT SPECIFICATION

(11) **EP 2 925 744 B1**
(45) Date of publication and mention of the grant of the patent: **30.12.2020**
(21) Application number: 13859400.7
(22) Date of filing: 26.11.2013
(51) Int. Cl.: A61K 31/505, C07D 403/04, A61K 31/506, A61P 35/00

(54) **PYRIMIDINE-2,4-DIAMINE DERIVATIVES FOR TREATMENT OF CANCER**
PYRIMIDIN-2,4-DIAMINDERIVATE ZUR BEHANDLUNG VON KREBS
DÉRIVÉS DE PYRIMIDINE-2,4-DIAMINE UTILISABLES EN VUE DU TRAITEMENT DU CANCER

(30) Priority: 27.11.2012 SE 1251332; 27.11.2012 US 201261797022 P
(43) Date of publication of application: 07.10.2015
(73) Proprietor: Thomas Helledays Stiftelse För Medicinsk Forskning, 113 34 Stockholm (SE)
(72) Inventor: SCOBIE, Martin, S-754 49 Uppsala (SE); HELLEDAY, Thomas, S-182 79 Stocksund (SE); KOOLMEISTER, Tobias, S-115 37 Stockholm (SE); JACQUES, Sylvain, F-67350 Uberach (FR); DESROSES, Matthieu, SE-741 43 Knivsta (SE); JACQUES-CORDONNIER, Marie-Caroline, 67350 Uberach (FR)
(74) Representative: Brann AB
(86) International application number: PCT/SE2013/051387
(87) International publication number: WO 2014/084778

(56) References cited:
- WO-A1-2004/080979
- WO-A1-2009/105220
- WO-A1-2011/056916
- WO-A1-2011/100285
- WO-A2-02/096867
- WO-A2-2006/078886
- GB-A- 681 712
- US-A1- 2008 194 577
- US-A1- 2011 275 611
- MEDINA J R ET AL: "Structure-Based Design of Potent and Selective 3-Phosphoinositide-Dependent Kinase-1 (PDK1) Inhibitors", JOURNAL OF MEDICINAL CHEMISTRY, AMERICAN CHEMICAL SOCIETY, US, vol. 54, no. 6, 24 March 2011 (2011-03-24) , pages 1871-1895, XP002736564, ISSN: 0022-2623, DOI: 10.1021/JM101527U [retrieved on 2011-02-22]
- JINHO LEE ET AL: "Discovery of a novel class of 2-aminopyrimidines as CDK1 and CDK2 inhibitors", BIOORGANIC & MEDICINAL CHEMISTRY LETTERS, PERGAMON, AMSTERDAM, NL, vol. 21, no. 14, 23 May 2011 (2011-05-23), pages 4203-4205, XP028234380, ISSN: 0960-894X, DOI: 10.1016/J.BMCL.2011.05.081 [retrieved on 2011-05-27]
- SHI ZHI-CAI ET AL: "Modulation of peripheral serotonin levels by novel tryptophan hydroxylase inhibitors for the potential treatment of functional gastrointestinal disorders", JOURNAL OF MEDICINAL CHEMISTRY, AMERICAN CHEMICAL SOCIETY, US, vol. 51, no. 13, 10 July 2008 (2008-07-10) , pages 3684-3687, XP002494320, ISSN: 0022-2623, DOI: 10.1021/JM800338J [retrieved on 2008-06-17]
- SUNDURU N ET AL: "Discovery of new 1,3,5-triazine scaffolds with potent activity against Mycobacterium tuberculosis H37Rv", EUROPEAN JOURNAL OF MEDICINAL CHEMISTRY, EDITIONS SCIENTIFIQUE ELSEVIER, PARIS, FR, vol. 45, no. 8, 1 August 2010 (2010-08-01) , pages 3335-3345, XP027473628, ISSN: 0223-5234, DOI: 10.1016/J.EJMECH.2010.04.017 [retrieved on 2010-04-20]
- KATIYAR S B ET AL: "Syntheses of 2,4,6-trisubstituted pyrimidine derivatives as a new class of antifilarial topoisomerase II inhibitors", BIOORGANIC & MEDICINAL CHEMISTRY LETTERS, PERGAMON, AMSTERDAM, NL, vol. 15, no. 1, 3 January 2005 (2005-01-03), pages 47-50, XP027800876, ISSN: 0960-894X [retrieved on 2005-01-03]
- HARALD ENGELHARDT ET AL: "Bispyrimidines as Potent Histamine H 4 Receptor Ligands: Delineation of Structure-Activity Relationships and Detailed H 4 Receptor Binding Mode", JOURNAL OF MEDICINAL CHEMISTRY, vol. 56, no. 11, 13 June 2013 (2013-06-13) , pages 4264-4276, XP55238885, US ISSN: 0022-2623, DOI: 10.1021/jm301886t
- KUMAR A ET AL: "A Novel and Convenient Synthesis of 2-Amino-4-(N-alkyl-N-arylamino)-pyrimidine s using Polarized Ketene S,S- and S,N-Acetals", SYNTHESIS, GEORG THIEME VERLAG, STUTTGART, DE, vol. 9, 1 January 1980 (1980-01-01), pages 748-751, XP002432317, ISSN: 0039-7881, DOI: 10.1055/S-1980-29200
- DATABASE CA [Online] CHEMICAL ABSTRACTS SERVICE, COLUMBUS, OHIO, US; VISHWAKARMA, J. N. ET AL: "Reactions of polarized ketene S,N-acetals with guanidine: a facile general route to novel 5,6-substituted 2-amino-4-N-alkyl/aryl/N- azacycloalkylaminopyrimidines. Part XLIV", XP002759721, retrieved from STN Database accession no. 1986:406471 & VISHWAKARMA, J. N. ET AL: "Reactions of polarized ketene S,N-acetals with guanidine: a facile general route to novel 5,6-substituted 2-amino-4-N-alkyl/aryl/N- azacycloalkylaminopyrimidines. Part XLIV", INDIAN JOURNAL OF CHEMISTRY, SECTION B: ORGANIC CHEMISTRY INCLUDING MEDICINAL CHEMISTRY , 24B(5), 466-71 CODEN: IJSBDB; ISSN: 0376-4699, 1985,
- DATABASE CA [Online] CHEMICAL ABSTRACTS SERVICE, COLUMBUS, OHIO, US; TANI, HIDEO ET AL: "Pyrimidine derivatives", XP002759722, retrieved from STN Database accession no. 1975:140168 & TANI, HIDEO ET AL: "Pyrimidine derivatives", JPN. TOKKYO KOHO, 12 PP. CODEN: JAXXAD, 1974,
- DATABASE CA [Online] CHEMICAL ABSTRACTS SERVICE, COLUMBUS, OHIO, US; TANI, HIDEO ET AL: "2,4,6-Trisubstituted pyrimidines", XP002759723, retrieved from STN Database accession no. 1975:140173 & TANI, HIDEO ET AL: "2,4,6-Trisubstituted pyrimidines", JPN. TOKKYO KOHO, 12 PP. CODEN: JAXXAD, 1974,
- RAI PRIYAMVADA ED - RAI PRIYAMVADA: "Human Mut T Homolog 1 (MTH1): a roadblock for the tumor-suppressive effects of oncogenic RAS-induced ROS", SMALL GTPASES 2012 APR-JUN,, vol. 3, no. 2, 1 April 2012 (2012-04-01), pages 120-125, XP002688058, ISSN: 2154-1256
- LINDA M SVENSSON ET AL: "Crystal structure of human MTH1 and the 8-oxo-dGMP product complex", FEBS LETTERS, ELSEVIER, AMSTERDAM, NL, vol. 585, no. 16, 13 July 2011 (2011-07-13), pages 2617-2621, XP028261613, ISSN: 0014-5793, DOI: 10.1016/J.FEBSLET.2011.07.017 [retrieved on 2011-07-21]
- DATABASE REGISTRY [Online] XP055260721 Retrieved from STN Database accession no. 634582-11-9 & DATABASE REGISTRY 06 January 2004 STN Database accession no. 634195-10-1 & DATABASE REGISTRY 05 January 2004 STN Database accession no. 634195-07-6

## Description

### Field of the Invention

The invention relates to novel compounds, compositions and methods for treatment of cancer. In particular, the invention relates to novel compounds, compositions and methods for the treatment of cancers through inhibition of MTH1.

### Background of the Invention

The listing or discussion of an apparently prior-published document in this specification should not necessarily be taken as an acknowledgement that the document is part of the state of the art or is common general knowledge.

### Background

Dysfuncitonal redox regulation of cellular signalling and an increased ROS (Reactive oxygen species) tension have been demonstrated to play a crucial role in cancer etiology, progression and metastasis (Zhang et al., Antioxid Redox Signal 15(11)2011:2876-2908). ROSmediates tumor-promoting characteristics, such as e.g. unrestrained proliferation, survival signaling, increased migration, angiogenesis. ROS are generated during cell metabolism and are highly reactive with macromolecules such as DNA, proteins and lipids. Exposure of nucleic acids to ROS can create more than 20 oxidatively modified nucleotides, of which 8-oxo-7,8-dihydroxyguanine (8-oxo-dG) is most abundant. 8-oxo-dG plays a pivotal role in mutagenesis (Sekiguchi and Tsuzuki., Oncogene 21(58)2002:8895-906) . To protect themselves from carcinogenic effects, mammalian cells are armed with a set of repair enzymes to remove the oxidized nucleotides to maintain genome integrity. One of these protective enzymes is MTH1 (MutT homologue 1,8-oxo-dGTPase, NUDT1). Interestingly, MTH1 is upregulated in various cancer forms, suggesting that the cancer cell rely on MTH1 function to survive the increased DNA lesion (Human Proteinatlas, Koketsu et al., Hepatogastroenterology, 51(57)2004:638-41). Suppression of MTH1 level and activity by using RNAi technology, leads to reduced cancer cell survival, premature senescence and DNA strand breaks (Rai et al, PNAS, 106(1)2009:169-174), Helleday et al unpublished data). Interestingly, lung cancers which spontaneously form in OGG-/- mice are prevented from forming in crosses with the MTH1-/- mice, suggesting that MTH1 is required for lung cancer cells to survive (Sakumi et al., Cancer Res 63, 2003: 902). We have observed that downregulation of MTH1 protein levels in human colon cancer tumors in xenograft mice model reduced tumor growth and significantly shrinked the tumour (Helleday et al, unpublished data).
In tumour cells, reducing the capacity to eliminate oxidised dNTPs by inhibiting MTH1 activity, will reduce cancer cell survival and hence be a promising novel anticancer therapy, either as monotherapy in cancer forms with high oxidative stress levels and/or in combination with radiotherapy and chemotherapy drugs.

### Shortcomings and complications with current treatment

Today's treatment of cancer is not effective for all patients with diagnosed disease also including a large proportion of patients that experience adverse effects from treatments with existing therapies or where resistance to on-going therapy is developed over time.

### Prior art

Engelhardt, H. et al. Journal of Medicinal Chemistry (2013), 56(11), 4264-4276 and US patent application US 2010/0016344 disclose certain 6-aryl-2,4-diaminopyrimidines having an additional pyrimidine appendage as histamine H4 receptor modulators. The compounds are claimed to be useful for a various diseases including cancer pain, but their use in the treatment of cancer as such is neither disclosed or suggested.
International patent application WO 2013/066839 discloses 6-(3-pyridyl)-(2,4-diaminopyrimidines as HDAC inhibitors. However, the substituent on the 4-amino group contains a prerequisite 5-trifluoromethyl-1,2,4-oxadiazol-3-yl group.
2,4-Diaminopyrimidines substituted in the 6-position with 3-aminoindazoles have been described in international patent application WO 2010/059658. Although also indazoles without the amino groups are mentioned, it is evident from the examples that the 3-amino substituent on the indazole is required for activity. The same document also describes 2,4-diaminopyrimidines substituted in the 6-position by a 3-cyano-2-fluorophenyl group. However, these compounds are merely precursors to the 3-aminoindazoles mentioned above and there is no disclosure or suggestions in the document that they possess any anti-cancer activity.

International patent application WO 2006/078886 describes 2,4-diaminopyrimidines substituted in the 6-position by an aryl group as wnt modulators. The 4-aryl group is lacking any substituents or must be substituted in the 3-position by methoxy. The document does not disclose or suggest compounds with any other substituent-pattern, nor does it mention or suggest the use of such compounds in the treatment of cancer. Moreover, in all examples the 4-amino group of the pyrimidine is substituted by either 1,3-benzodioxol-5-ylmethyl or by 4-hydroxy-phenethyl. Several scientific publications describes the use of one of the compounds (*N*4-(1,3-benzodioxol-5-ylmethyl)-6-(3-methoxyphenyl)-2,4-pyrimidinediamine) as a tool to investigate the wnt-pathway.
International patent application WO 86/04583 describes aziridinyl substituted anti-neoplastic compounds. There is only one compound that have both a 6-aryl substituent and a 4-*N*-alkyl group attached to 2-aminopyrimidine core. The compound has besides the aziridinyl group a fluorine in the 5-position of the pyrimidine ring. Both the aziridinyl and the fluorine are implied to be important for the activity and there is nothing that suggests that anti-neoplastic activity can be obtained without at least one of these substituents.
British patent application GB 681712 describes 2,4-diaminopyrimidines substituted in the 6-position by an aryl group for use in the treatment of cancer, but in only one example the aryl is phenyl and the 4-amino group is substituted by an alkyl. In this compound the phenyl is unsubstituted and the alkyl is methyl. There is no disclosure in this documents of compounds in which the 6-phenyl may be substituted by other groups than chloro or nitro in the para-position, that the the 6-phenyl may contain more than one substituent or that the 4-alkylamino-group is larger than methyl or may carry substituents.

Two publications from the group of H. Junjappa (Indian Journal Chemistry (1985), 24B 466; Synthesis (1980), 748) describes the synthesis of certain 2-amino-4-(*N-*alkylamino)-6-arylpyrimidines. The publications do not mention or suggest the use of the synthesized compounds in the treatment of cancer.

There are numerous 2-amino-4-(*N*-alkylamino)-6-arylpyrimidines that are, or that at some point have been stated to be, commercially available but that do not have any ascribed pharmaceutical use, nor any other use, ascribed to them.

MTH1 inhibitors have been described in Streib, M. et al. Angewandte Chemie, Int,. Ed. (2013), Vol. 52. The compounds are organometallic and are not 2-amino-4-(*N*-alkylamino)-6-arylpyrimidines.

### Summary of the invention

Although the finding of oncogenes and development of new anticancer treatments and diagnosis have improved the life length of cancer patients, there is still a high medical need to find more effective and less toxic treatments for e.g. breast cancer, leukemia, colon or lung cancer. Our preliminary data suggests that MTH1 inhibitors have the potential to be very effective against cancer forms with dysfunctional redox status, with minimal general toxic effects. MTH1 inhibition may also be a suitable adjuvant therapy to be used in conjunction with radiotherapies or other chemotherapeutic approaches.

The present invention aims at providing new treatments for cancer that can be achieved by inhibition of MTH1

### Brief description of the drawings

Figure 1. Effect on cell survival following MTH1 siRNA depletion in various human cancer and normal cell lines.
Figure 2. MTH1 inhibitor reduce cell survival in various cancer cell lines, with less effect on normal immortalised cells (VH10 and BJ hTERT)

### Detailed description of the Invention

In an embodiment of the invention there is provided a compound of formula I, 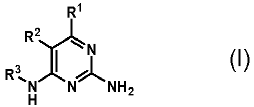 or a pharmaceutically acceptable salt thereof, wherein:
R¹ represents heteroaryl selected from benzofuran-3-yl, benzothiophen-3-yl, dihydrobenzofuran-7-yl, indol-3-yl, indol-4-yl, indol-5-yl, isoquinolin-4-yl, pyridin-3-yl, pyridin-4-yl, pyrrol-2-yl and quinolin-5-yl, which heteroaryl is connected to the pyrimidine of formula I via a carbon atom of the heteroaryl ring, and which heteroaryl is optionally substituted by one or more substituents selected from Y¹, or -C₁₋₆alkyl optionally substituted by one or more Y²; or aryl represented by 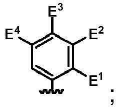
E³ and E⁴ represent hydrogen; and
E¹ represents -F and E² represent -F, -Cl, -CH₃ or -CF₃; or
E¹ represents -Cl and E² represents -F, -Cl, -CH₃ or -CF₃; or
E¹ represents -CH₃ and E² represents -F, -Cl, -CH₃, -CF₃, or -CN;
R² represents hydrogen;
R³ represents -C₂₋₆alkyl substituted by Z¹;
each Y¹ independently represents halogen, -CN, -C(O)R⁸, -C(O)N(R^{b})R^{c}, -C(O)OR^{d}, -N(R^{e})R^{f}, -N(R^{g})C(O)R^{h}, -N(Rⁱ)C(O)OR^{j}, -N(R^{k})C(O)N(R^{l})R^{m}, -NO₂, -N(Rⁿ)S(O)₂R^{o}, -OR^{p}, -OC(O)R^{q}, -OS(O)₂R^{r}, -S(O)ₘR^{s}, -S(O)₂N(R^{t})R^{u}, 3- to 12-membered heterocycloalkyl optionally substituted by one or more substituents selected from W², 6- to 14-membered aryl optionally substituted by one or more substituents selected from W³, or 5- to 10-membered heteroaryl containing one or more heteroatoms selected from oxygen, nitrogen and/or sulfur optionally substituted by one or more substituents selected from W³;
each R^{a}, R^{b}, R^{c}, R^{d}, R^{e}, R^{g}, R^{h}, Rⁱ, R^{k}, R^{l}, R^{m}, Rⁿ, R^{p}, R^{q}, R^{s}, R^{t} and R^{u} independently represents hydrogen, -C₁₋₆ alkyl optionally substituted by one or more substituents selected from W¹, 3- to 12-membered heterocycloalkyl optionally substituted by one or more substituents selected from W², 6- to 14-membered aryl optionally substituted by one or more substituents selected from W³, or 5- to 10-membered heteroaryl containing one or more heteroatoms selected from oxygen, nitrogen and/or sulfur optionally substituted by one or more substituents selected from W³; or
any two R^{b} and R^{c}, R^{e} and R^{f} , R^{l} and R^{m} and/or R^{t} and R^{u} are linked together to form, along with the nitrogen atom to which they are attached, a 3- to 8-membered monocyclic or bicyclic ring, which ring optionally contains one or two further heteroatoms and which ring optionally is substituted by one or more substituents selected from W², C₁₋₃alkyl optionally substituted by one or more substituents selected from W¹, and =O;
each R^{f}, R^{j}, R^{o} and R^{r} independently represent -C₁₋₆ alkyl optionally substituted by one or more substituents selected from W¹, 3- to 12-membered heterocycloalkyl optionally substituted by one or more substituents selected from W², 6- to 14-membered aryl optionally substituted by one or more substituents selected from W³ or 5- to 10-membered heteroaryl containing one or more heteroatoms selected from oxygen, nitrogen and/or sulfur optionally substituted by one or more substituents selected from W³;
each Y² independently represents halogen, -CN, -C(O)R^{b1}, -C(O)N(R^{c1})R^{d1}, -C(O)OR^{e1}, -N(R^{f1})R^{g1}, -N(R^{h1})C(O)Rⁱ¹, -N(R^{j1})C(O)OR^{k1}, -N(R^{l1})C(O)N(R^{m1})Rⁿ¹, -N(R^{o1})S(O)₂R^{p1}, -OR^{q1}, -OC(O)R^{r1}, -OS(O)₂R^{s1}, -S(O)ₘR^{t1}, -S(O)₂N(R^{u1})R^{v1}, 3- to 12-membered heterocycloalkyl optionally substituted by one or more substituents selected from W¹, 6- to 14-membered aryl optionally substituted by one or more substituents selected from W², 5- to 10-membered heteroaryl containing one or more heteroatoms selected from oxygen, nitrogen and/or sulfur optionally substituted by one or more substituents selected from W²;
each R^{b1}, R^{c1}, R^{d1}, R^{e1}, R^{f1}, R^{h1}, Rⁱ¹, R^{j1}, R^{l1}, R^{m1}, Rⁿ¹, R^{o1}, R^{q1}, R^{r1}, R^{t1}, R^{u1} and R^{v1} independently represents hydrogen, C₁₋₆ alkyl optionally substituted by one or more substituents selected from W¹, 3- to 12-membered heterocycloalkyl optionally substituted by one or more substituents selected from W², 6- to 14-membered aryl optionally substituted by one or more substituents selected from W³, or 5- to 10-membered heteroaryl containing one or more heteroatoms selected from oxygen, nitrogen and/or sulfur optionally substituted by one or more substituents selected from W³; or
any two R^{c1} and R^{d1}, R^{f1} and R^{g1}, R^{m1} and Rⁿ¹ and/or R^{u1} and R^{v1} are linked together to form, along with the nitrogen atom to which they are attached, a 3- to 8-membered monocyclic or bicyclic ring, which ring optionally contains one or two further heteroatoms and which ring optionally is substituted by one or more substituents selected from W², C₁₋₃alkyl optionally substituted by one or more substituents selected from W¹, and =O;
each R^{g1}, R^{k1}, R^{p1} and R^{s1} independently represent -C₁₋₆ alkyl optionally substituted by one or more substituents selected from W¹, 3- to 12-membered heterocycloalkyl optionally substituted by one or more substituents selected from W², 6- to 14-membered aryl optionally substituted by one or more substituents selected from W³, or 5- to 10-membered heteroaryl containing one or more heteroatoms selected from oxygen, nitrogen and/or sulfur optionally substituted by one or more substituents selected from W³;
Z¹ represents -C(O)N(R^{c2})R^{d2}, -N(R^{f2})R^{g2}, -N(R^{h2})C(O)Rⁱ², -N(R^{l2})C(O)N(R^{m2})Rⁿ²,-N(R^{o2})S(O)₂R^{p2}, -OR^{q2}, -S(O)ₘR^{t2}, -S(O)₂N(R^{u2})R^{v2}, or aryl represented by 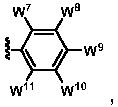
R^{c2}, R^{f2}, R^{h2}, R^{l2}, R^{m2}, R^{o2}, and R^{u2} represents hydrogen; and
R^{d2}, R^{g2}, Rⁱ², Rⁿ² R^{p2}, R^{q2}, R^{t2} and R^{v2} represents 6- to 14-membered aryl optionally substituted by one or more substituents selected from W⁶, or 5- to 10-membered heteroaryl containing one or more heteroatoms selected from oxygen, nitrogen and/or sulfur optionally substituted by one or more substituents selected from W⁶;
each W¹ independently represents halogen, -CN, -N(R^{f3})R^{g3}, -N(R^{h3})C(O)Rⁱ³,-N(R^{j3})C(O)OR^{k3}, -N(R^{l3})C(O)N(R^{m3})Rⁿ³, -N(R^{o3})S(O)₂R^{p3}, -OR^{q3}, -OC(O)R^{r3},-OS(O)₂R^{s3}, -S(O)ₘR^{t3}, -S(O)₂N(R^{u3})R^{v3}, 3- to 12-membered heterocycloalkyl optionally substituted by one or more substituents selected from G¹, 6- to 14-membered aryl optionally substituted by one or more substituents selected from G², 5- to 10-membered heteroaryl containing one or more heteroatoms selected from oxygen, nitrogen and/or sulfur optionally substituted by one or more substituents selected from G², or =O;
each W², W³, and W⁶ independently represents halogen, -R^{a3}, -CN, -C(O)R^{b3}, -C(O)N(R^{c3})R^{d3}, -C(O)OR^{e3}, -N(R^{f3})R^{g3}, -N(R^{h3})C(O)Rⁱ³, -N(R^{j3})C(O)OR^{k3}, -N(R^{l3})C(O)N(R^{m3})Rⁿ³, -N(R^{o3})S(O)₂R^{p3}, -OR^{q3}, -OC(O)R^{r3}, -OS(O)₂R^{s3}, -S(O)ₘR^{t3}, -S(O)₂N(R^{u3})R^{v3}, 3- to 12-membered heterocycloalkyl optionally substituted by one or more substituents selected from G¹, 6- to 14-membered aryl optionally substituted by one or more substituents selected from G², 5- to 10-membered heteroaryl containing one or more heteroatoms selected from oxygen, nitrogen and/or sulfur optionally substituted by one or more substituents selected from G², or =O;
W⁷ and W¹¹ represent hydrogen;
each W⁸ and W¹⁰ independently represents halogen, -R^{a3}, -CN, -C(O)R^{b3}, -C(O)N(R^{c3})R^{d3} -C(O)OR^{e3}, -N(R^{f3})R^{g3}, -N(R^{h3})C(O)Rⁱ³, -N(R^{j3})C(O)OR^{k3}, -N(R^{l3})C(O)N(R^{m3})Rⁿ³, -N(R^{o3})S(O)₂R^{p3}, -OR^{q3}, -OC(O)R^{r3}, -OS(O)₂R^{s3}, -S(O)ₘR^{t3}, -S(O)₂N(R^{u3})R^{v3}, 3- to 12-membered heterocycloalkyl optionally substituted by one or more substituents selected from G¹, 6- to 14-membered aryl optionally substituted by one or more substituents selected from G², or 5- to 10-membered heteroaryl containing one or more heteroatoms selected from oxygen, nitrogen and/or sulfur optionally substituted by one or more substituents selected from G²;
W⁹ represents halogen, -R^{a3}, -CN, -C(O)R^{b3}, -C(O)N(R^{c3})R^{d3}, -C(O)OR^{e3},-N(R^{f3})R^{g3}, -N(R^{h3})C(O)Rⁱ³, -N(R^{j3})C(O)OR^{k3}, -N(R^{l3})C(O)N(R^{m3})Rⁿ³,-N(R^{o3})S(O)₂R^{p3}, -OR^{q3x}, -OC(O)R^{r3}, -OS(O)₂R^{s3}, -S(O)ₘR^{t3}, -S(O)₂N(R^{u3})R^{v3}, 3- to 12-membered heterocycloalkyl optionally substituted by one or more substituents selected from G¹, 6- to 14-membered aryl optionally substituted by one or more substituents selected from G², or 5- to 10-membered heteroaryl containing one or more heteroatoms selected from oxygen, nitrogen and/or sulfur optionally substituted by one or more substituents selected from G²;
each R^{a3}, R^{b3}, R^{c3}, R^{d3}, R^{e3}, R^{f3}, R^{h3}, Rⁱ³, R^{j3}, R^{l3}, R^{m3}, Rⁿ³, R^{o3}, R^{q3}, R^{r3}, R^{t3}, R^{u3} and R^{v3} independently represents hydrogen or -C₁₋₆ alkyl optionally substituted by one or more G³; or
any two R^{c3} and R^{d3}, R^{f3} and R^{g3}, R^{m3} and Rⁿ³ and/or R^{u3} and R^{v3} are linked together to form, along with the nitrogen atom to which they are attached, a 4- to 6-membered ring, which ring optionally contains one further heteroatom and which ring optionally is substituted by one or more G²;
each R^{g3}, R^{k3}, R^{p3}, and R^{s3} independently represent C₁₋₆ alkyl optionally substituted by one or more G³;
R^{q3x} represents C₂₋₆ alkyl optionally substituted by one or more G³;
each G¹ and G² independently represents halogen, -R^{a4}, -CN, -C(O)R^{b4}, -C(O)N(R^{c4})R^{d4}, -C(O)OR^{e4}, -N(R^{f4})R^{g4}, -N(R^{h4})C(O)Rⁱ⁴, -N(R^{j4})C(O)OR^{k4}, -N(R^{l4})C(O)N(R^{m4})Rⁿ⁴, -N(R^{o4})S(O)₂R^{p4}, -OR^{q4}, -OC(O)R^{r4}, -OS(O)₂R^{s4}, -S(O)ₘR^{t4}, -S(O)₂N(R^{u4})R^{v4}, or =O;
G³ represents halogen, -CN, -C(O)R^{b4}, -C(O)N(R^{c4})R^{d4}, -C(O)OR^{e4}, -N(R^{f4})R^{g4}, -N(R^{h4})C(O)Rⁱ⁴, -N(R^{j4})C(O)OR^{k4}, -N(R^{l4})C(O)N(R^{m4})Rⁿ⁴, -N(R^{o4})S(O)₂R^{p4}, -OR^{q4}, -OC(O)R^{r4}, -OS(O)₂R^{s4}, -S(O)ₘR^{t4}, -S(O)₂N(R^{u4})R^{v4}, or =O;
each R^{a4}, R^{b4}, R^{c4}, R^{d4}, R^{e4}, R^{f4}, R^{h4}, Rⁱ⁴, R^{j4}, R^{l4}, R^{m4}, Rⁿ⁴, R^{o4}, R^{q4}, R^{r4}, R^{t4}, R^{u4} and R^{v4} independently represents hydrogen or -C₁₋₆ alkyl optionally substituted by one or more -F; or
any two R^{c4} and R^{d4}, R^{f4} and R^{g4}, R^{m4} and Rⁿ⁴ and/or R^{u4} and R^{v4} are linked together to form, along with the nitrogen atom to which they are attached, a 3- to 6-membered ring, which ring optionally is substituted by one or more -F, -CH₃, -CH₂CH₃, -CHF₂, -CF₃, -CH₂CF₃, or =O;
each R^{g4}, R^{k4}, R^{p4} and R^{s4} independently represents C₁₋₆ alkyl optionally substituted by one or more -F; and
each m independently represents 0, 1 or 2;
which compounds may be referred to herein as "the compounds of the invention".

Pharmaceutically-acceptable salts include acid addition salts and base addition salts. Such salts may be formed by conventional means, for example by reaction of a free acid or a free base form of a compound of formula I with one or more equivalents of an appropriate acid or base, optionally in a solvent, or in a medium in which the salt is insoluble, followed by removal of said solvent, or said medium, using standard techniques (e.g. *in vacuo,* by freeze-drying or by filtration). Salts may also be prepared by exchanging a counter-ion of a compound of the invention in the form of a salt with another counter-ion, for example using a suitable ion exchange resin. For the avoidance of doubt, solvates are also included within the scope of the invention.

Compounds of the invention may contain double bonds and may thus exist as E (*e*n*tgegen*) and *Z* (*zusammen*) geometric isomers about each individual double bond. All such isomers and mixtures thereof are included within the scope of the invention.

Compounds of the invention may also exhibit tautomerism. All tautomeric forms and mixtures thereof are included within the scope of the invention.

Compounds of the invention may also contain one or more asymmetric carbon atoms and may therefore exhibit optical and/or diastereoisomerism. Diastereoisomers may be separated using conventional techniques, e.g. chromatography or fractional crystallisation. The various stereoisomers may be isolated by separation of a racemic or other mixture of the compounds using conventional, e.g. fractional crystallisation or HPLC, techniques. Alternatively the desired optical isomers may be made by reaction of the appropriate optically active starting materials under conditions which will not cause racemisation or epimerisation (i.e. a 'chiral pool' method), by reaction of the appropriate starting material with a 'chiral auxiliary' which can subsequently be removed at a suitable stage, by derivatisation (i.e. a resolution, including a dynamic resolution), for example with a homochiral acid followed by separation of the diastereomeric derivatives by conventional means such as chromatography, or by reaction with an appropriate chiral reagent or chiral catalyst all under conditions known to the skilled person. All stereoisomers and mixtures thereof are included within the scope of the invention.

Unless otherwise specified, C_{1-q} alkyl groups (where q is the upper limit of the range) defined herein may be straight-chain or, when there is a sufficient number (i.e. a minimum of two or three, as appropriate) of carbon atoms, be branched-chain, and/or cyclic (so forming a C_{3-q}-cycloalkyl group). When there is a sufficient number (i.e. a minimum of four) of carbon atoms, such groups may also be part cyclic. Such alkyl groups may also be saturated or, when there is a sufficient number (i.e. a minimum of two) of carbon atoms, be unsaturated (forming, for example, a C_{2-q} alkenyl or a C_{2-q} alkynyl group).

Unless otherwise specified, C_{1-q} alkylene groups (where q is the upper limit of the range) defined herein may (in a similar manner to the definition of C_{1-q} alkyl) be straight-chain or, when there is a sufficient number (i.e. a minimum of two or three, as appropriate) of carbon atoms, be branched-chain, and/or cyclic (so forming a C_{3-q}-cycloalkylene group). When there is a sufficient number (i.e. a minimum of four) of carbon atoms, such groups may also be part cyclic. Such alkylene groups may also be saturated or, when there is a sufficient number (i.e. a minimum of two) of carbon atoms, be unsaturated (forming, for example, a C_{2-q}alkenylene or a C_{2-q}alkynylene group). Particular alkylene groups that may be mentioned include those that are straight-chained and saturated.

The term "halo", when used herein, includes fluoro, chloro, bromo and iodo (for example, fluoro and chloro).

Heterocycloalkyl groups that may be mentioned include non-aromatic monocyclic and bicyclic heterocycloalkyl groups (which groups may further be bridged) in which at least one (e.g. one to four) of the atoms in the ring system is other than carbon (i.e. a heteroatom), and in which the total number of atoms in the ring system is between three and twelve (e.g. between five and ten and, most preferably, between three and eight, e.g. a 5- or 6-membered heterocycloalkyl group). Further, such heterocycloalkyl groups may be saturated or unsaturated containing one or more double and/or triple bonds, forming for example a C_{2-q} (e.g. C_{4-q}) heterocycloalkenyl (where q is the upper limit of the range) or a C_{7-q} heterocycloalkynyl group. C_{2-q} heterocycloalkyl groups that may be mentioned include 7-azabicyclo-[2.2.1]heptanyl, 6-azabicyclo[3.1.1]heptanyl, 6-azabicyclo[3.2.1]-octanyl, 8-azabicyclo[3.2.1]octanyl, aziridinyl, azetidinyl, dihydropyranyl, dihydropyridyl, dihydropyrrolyl (including 2,5-dihydropyrrolyl), dioxolanyl (including 1,3-dioxolanyl), dioxanyl (including 1,3-dioxanyl and 1,4-dioxanyl), dithianyl (including 1,4-dithianyl), dithiolanyl (including 1,3-dithiolanyl), imidazolidinyl, imidazolinyl, 7-oxabicyclo[2.2.1]heptanyl, 6-oxabicyclo[3.2.1]-octanyl, oxetanyl, oxiranyl, piperazinyl, piperidinyl, pyranyl, pyrazolidinyl, pyrrolidinonyl, pyrrolidinyl, pyrrolinyl, quinuclidinyl, sulfolanyl, 3-sulfolenyl, tetrahydropyranyl, tetrahydrofuryl, tetrahydropyridyl (such as 1,2,3,4-tetrahydropyridyl and 1,2,3,6-tetrahydropyridyl), thietanyl, thiiranyl, thiolanyl, thiomorpholinyl, trithianyl (including 1,3,5-trithianyl), tropanyl and the like. Substituents on heterocycloalkyl groups may, where appropriate, be located on any atom in the ring system including a heteroatom. Further, in the case where the substituent is another cyclic compound, then the cyclic compound may be attached through a single atom on the heterocycloalkyl group, forming a so-called "spiro"-compound. The point of attachment of heterocycloalkyl groups may be *via* any atom in the ring system including (where appropriate) a heteroatom (such as a nitrogen atom), or an atom on any fused carbocyclic ring that may be present as part of the ring system. Heterocycloalkyl groups may also be in the *N*- or *S-*oxidised form. At each occurrence when mentioned herein, a heterocycloalkyl group is preferably a 3- to 8-membered heterocycloalkyl group (e.g. a 5- or 6-membered heterocycloalkyl group).

The term "aryl", when used herein, includes C₆₋₁₄ (e.g. C₆₋₁₀) aromatic groups. Such groups may be monocyclic or bicyclic and, when bicyclic, be either wholly or partly aromatic. C₆₋₁₀ aryl groups that may be mentioned include phenyl, naphthyl, 1,2,3,4-tetrahydronaphthyl, indanyl, and the like (e.g. phenyl, naphthyl and the like). For the avoidance of doubt, the point of attachment of substituents on aryl groups may be *via* any carbon atom of the ring system.

The term "heteroaryl" (or heteroaromatic), when used herein, includes 5- to 10-membered heteroaromatic groups containing one or more heteroatoms selected from oxygen, nitrogen and/or sulfur. Such heteroaryl group may comprise one, or two rings, of which at least one is aromatic. Substituents on heteroaryl/heteroaromatic groups may, where appropriate, be located on any atom in the ring system including a heteroatom. The point of attachment of heteroaryl/heteroaromatic groups may be *via* any atom in the ring system including (where appropriate) a heteroatom. Bicyclic heteroaryl/heteroaromatic groups may comprise a benzene ring fused to one or more further aromatic or non-aromatic heterocyclic rings, in which instances, the point of attachment of the polycyclic heteroaryl/heteroaromatic group may be *via* any ring including the benzene ring or the heteroaryl/heteroaromatic or heterocycloalkyl ring. Examples of heteroaryl/heteroaromatic groups that may be mentioned include pyridinyl, pyrrolyl, furanyl, thiophenyl, oxadiazolyl, thiadiazolyl, thiazolyl, oxazolyl, pyrazolyl, triazolyl, tetrazolyl, isoxazolyl, isothiazolyl, imidazolyl, imidazopyrimidinyl, pyrimidinyl, indolyl, azaindolyl, pyrazinyl, indazolyl, pyrimidinyl, quinolinyl, isoquinolinyl, benzofuranyl, benzothiophenyl, benzoimidazolyl, benzoxazolyl, benzothiazolyl and benzotriazolyl. The oxides of heteroaryl/ heteroaromatic groups are also embraced within the scope of the invention (e.g. the N-oxide). As stated above, heteroaryl includes polycyclic (e.g. bicyclic) groups in which one ring is aromatic (and the other may or may not be aromatic). Hence, other heteroaryl groups that may be mentioned include e.g. benzo[1,3]dioxolyl, benzo[1,4]dioxinyl, indolinyl, *5H,6H,7H*-pyrrolo[1,2-*b*]pyrimidinyl, 1,2,3,4-tetra-hydroquinolinyl and the like.

Heteroatoms that may be mentioned include phosphorus, silicon, boron and, preferably, oxygen, nitrogen and sulfur.

For the avoidance of doubt, in cases in which the identity of two or more substituents in a compound of the invention may be the same, the actual identities of the respective substituents are not in any way interdependent.

For the avoidance of doubt, when R¹ is defined as 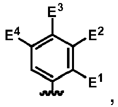 it is connected to the rest of formula I by the bond interrupted by the wiggly line, and formula I can thus be represented by

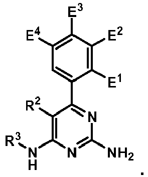

Likewise, when R³ is -C₂₋₆ alkyl substituted by Z¹, and Z¹ is represented by 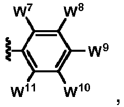 then, if e.g. R³ is C₂alkyl, then formula I can be represented by

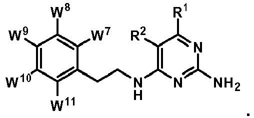

The present invention also embraces isotopically-labeled compounds of the present invention which are identical to those recited herein, but for the fact that one or more atoms are replaced by an atom having an atomic mass or mass number different from the atomic mass or mass number usually found in nature (or the most abundant one found in nature). All isotopes of any particular atom or element as specified herein are contemplated within the scope of the compounds of the invention. Hence, the compounds of the invention also include deuterated compounds, i.e. in which one or more hydrogen atoms are replaced by the hydrogen isotope deuterium.

All individual features (e.g. preferred features) mentioned herein may be taken in isolation or in combination with any other feature (including preferred features) mentioned herein (hence, preferred features may be taken in conjunction with other preferred features, or independently of them).

The skilled person will appreciate that compounds of the invention that are the subject of this invention include those that are stable. That is, compounds of the invention include those that are sufficiently robust to survive isolation from e.g. a reaction mixture to a useful degree of purity.

Compounds of formula I where R¹ represents heteroaryl are those where R¹ represents benzofuran-3-yl, benzothiophen-3-yl, dihydrobenzofuran-7-yl, indol-3-yl, indol-4-yl, indol-5-yl, isoquinolin-4-yl, pyridin-3-yl, pyridin-4-yl, pyrrol-2-yl and quinolin-5-yl.

For example, compounds of formula I that may be mentioned include those in which R¹ represents indol-3-yl, indol-4-yl or indol-5-yl, where the indolyl is optionally substituted on the nitrogen with -S(O)₂Ar^{x}, where Ar^{x} is aryl or heteroaryl, preferably optionally substituted phenyl, e.g. unsubstituted phenyl or phenyl substituted in the 4-position by -F, -CI, -CH₃ or -CF₃.

Preferred compounds of formula I that may be mentioned include those in which R¹ is represented by 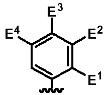 where:
E³ and E⁴ represent hydrogen; and
E¹ represents -F and E² represent -F, -CI, -CH₃ or -CF₃; or
E¹ represents -Cl and E² represents -F, -CI, -CH₃ or -CF₃; or
E¹ represents -CH₃ and E² represents -F, -CI, -CH₃, -CF₃ or -CN.

Preferred compounds of formula I that may be mentioned are those where Z¹ represents -OR^{q2} and R^{q2} represents aryl optionally substituted by one or more substituents selected from W⁶, or heteroaryl optionally substituted by one or more substituents selected from W⁶.

More preferred compounds of formula I that may be mentioned are those where Z¹ represents -N(R^{f2})R^{g2}, R^{f2} represents hydrogen and R^{g2} represents aryl optionally substituted by one or more substituents selected from W⁶, or heteroaryl optionally substituted by one or more substituents selected from W⁶.

Further preferred compounds of formula I that may be mentioned are those where Z¹ represents -N(R^{o2})S(O)₂R^{p2}, R^{o2} represents hydrogen and R^{p2} represents aryl optionally substituted by one or more substituents selected from W⁶, or heteroaryl optionally substituted by one or more substituents selected from W⁶.

Preferred compounds of formula I where Z¹ represents 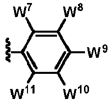 that may be mentioned are those where:
(a) each W⁸ and W¹⁰ are independently selected from -F, -CI, -CH₃, -CN, -CF₃, or more preferably hydrogen and W⁹ is selected from -R^{a3}, -CN, -C(O)R^{b3}, -C(O)N(R^{c3})R^{d3}, -C(O)OR^{e3}, -N(R^{f3})R^{g3}, -N(R^{h3})C(O)Rⁱ³, -N(R^{j3})C(O)OR^{k3}, -N(R^{l3})C(O)N(R^{m3})Rⁿ³, -N(R^{o3})S(O)₂R^{p3}, -OR^{q3x}, -OC(O)R^{r3}, -OS(O)₂R^{s3}, -S(O)ₘR^{t3}, -S(O)₂N(R^{u3})R^{v3}, heterocycloalkyl optionally substituted by one or more substituents selected from G¹, aryl optionally substituted by one or more substituents selected from G², or heteroaryl optionally substituted by one or more substituents selected from G²; and
(b) each W⁹ and W¹⁰ are independently selected from -F, -CI, -CH₃, -CN, -CF₃, or more preferably hydrogen and W⁸ is selected from -R^{a3}, -CN, -C(O)R^{b3}, -C(O)N(R^{c3})R^{d3}, -C(O)OR^{e3}, -N(R^{f3})R^{g3}, -N(R^{h3})C(O)Rⁱ³, -N(R^{j3})C(O)OR^{k3}, -N(R^{l3})C(O)N(R^{m3})Rⁿ³, -N(R^{o3})S(O)₂R^{p3}, -OR^{q3x}, -OC(O)R^{r3}, -OS(O)₂R^{s3}, -S(O)ₘR^{t3}, -S(O)₂N(R^{u3})R^{v3}, heterocycloalkyl optionally substituted by one or more substituents selected from G¹, aryl optionally substituted by one or more substituents selected from G², or heteroaryl optionally substituted by one or more substituents selected from G².

For example, particular compounds of formula I that may be mentioned include those wherein W⁸ and W¹⁰ represent hydrogen and W⁹ represents -F, -CI, -CH₃, cyclopropyl, -CF₃, -CN, -NH₂, -N(CH₃)₂, -N(H)C(O)CH₃, -N(H)C(O)OC(CH₃)₃, -SO₂CH₃, -SO₂NH₂, -S(O)₂N(CH₃)₂, -S(O)₂-4-morpholinyl, 4-methylpiperazin-1-yl, 4-methylpiperidin-1-ylmethyl or 1,2,3-thiadiazol-4-yl; or
W⁹ and W¹⁰ represent hydrogen and W⁸ represents -F, -Cl, -CN, -CH₃, -NMe₂, -S(O)₂NH₂ or -S(O)₂NMe₂.

More particularly preferred compounds of formula I that may be mentioned are those where W⁶ represents -F, -CI, -Br, -CH₃, cyclopropyl, -CF₃, -CN, -N(CH₃)₂, -SO₂CH₃, -SO₂NH₂ and -SO)₂N(CH₃)₂.

In one embodiment, the compound according to the invention is selected from the compounds of the Examples described herein.

As discussed hereinbefore, compounds of the invention are indicated as pharmaceuticals. According to a further aspect of the invention there is provided a compound of the invention, as hereinbefore defined, for use as a pharmaceutical.

In another aspect of the invention the use of a compound of the invention, as hereinbefore defined, is provided for the manufacture of a medicament for the treatment of cancer.

Although compounds of the invention may possess pharmacological activity as such, certain pharmaceutically-acceptable (e.g. "protected") derivatives of compounds of the invention may exist or be prepared which may not possess such activity, but may be administered parenterally or orally and thereafter be metabolised in the body to form compounds of the invention. Such compounds (which may possess some pharmacological activity, provided that such activity is appreciably lower than that of the "active" compounds to which they are metabolised) may therefore be described as "prodrugs" of compounds of the invention.

By "prodrug of a compound of the invention", we include compounds that form a compound of the invention, in an experimentally-detectable amount, within a predetermined time, following enteral or parenteral administration (e.g. oral or parenteral administration). All prodrugs of the compounds of the invention are included within the scope of the invention.

Furthermore, certain compounds of the invention may possess no or minimal pharmacological activity as such, but may be administered parenterally or orally, and thereafter be metabolised in the body to form compounds of the invention that possess pharmacological activity as such. Such compounds (which also includes compounds that may possess some pharmacological activity, but that activity is appreciably lower than that of the "active" compounds of the invention to which they are metabolised), may also be described as "prodrugs".

Thus, the compounds of the invention are useful because they possess pharmacological activity, and/or are metabolised in the body following oral or parenteral administration to form compounds, which possess pharmacological activity.

It is stated herein that the compounds of the invention may be useful in the treatment of cancer. For the purposes of this specification, and for the avoidance of doubt, the term "treatment" includes treatment *per se,* prevention and prophylaxis.

In an alternative embodiment, compounds of the invention may be useful in the the treatment of cancer.

Preferably the cancer is selected from the group comprising: Soft Tissue Cancers: sarcoma (angiosarcoma, fibrosarcoma, rhabdomyosarcoma, liposarcoma), myxoma, rhabdomyoma, fibroma, lipoma and teratoma; Lung: bronchogenic carcinoma (squamous cell, undifferentiated small cell, undifferentiated large cell, adenocarcinoma), alveolar (bronchiolar) carcinoma, bronchial adenoma, sarcoma, lymphoma, chondromatous hamartoma, mesothelioma; Gastrointestinal: esophagus (squamous cell carcinoma, adenocarcinoma, leiomyosarcoma, lymphoma), stomach (carcinoma, lymphoma, leiomyosarcoma), pancreas (ductal adenocarcinoma, insulinoma, glucagonoma, gastrinoma, carcinoid tumors, vipoma), small bowel (adenocarcinoma, lymphoma, carcinoid tumors, Kaposi's sarcoma, leiomyoma, hemangioma, lipoma, neurofibroma, fibroma), large bowel (adenocarcinoma, tubular adenoma, villous adenoma, hamartoma, leiomyoma); Genitourinary tract: kidney (adenocarcinoma, Wilm's tumor [nephroblastoma], lymphoma, leukemia), bladder and urethra (squamous cell carcinoma, transitional cell carcinoma, adenocarcinoma), prostate (adenocarcinoma, sarcoma), testis (seminoma, teratoma, embryonal carcinoma, teratocarcinoma, choriocarcinoma, sarcoma, interstitial cell carcinoma, fibroma, fibroadenoma, adenomatoid tumors, lipoma); Liver: hepatoma (hepatocellular carcinoma), cholangiocarcinoma, hepatoblastoma, angiosarcoma, hepatocellular adenoma, hemangioma; Bone: osteogenic sarcoma (osteosarcoma), fibrosarcoma, malignant fibrous histiocytoma, chondrosarcoma, Ewing's sarcoma, malignant lymphoma (reticulum cell sarcoma), multiple myeloma, malignant giant cell tumor chordoma, osteochronfroma (osteocartilaginous exostoses), benign chondroma, chondroblastoma, chondromyxofibroma, osteoid osteoma and giant cell tumors; Nervous system: skull (osteoma, hemangioma, granuloma, xanthoma, osteitis deformans), meninges (meningioma, meningiosarcoma, gliomatosis), brain (astrocytoma, medulloblastoma, glioma, ependymoma, germinoma [pinealoma], glioblastoma multiform, oligodendroglioma, schwannoma, retinoblastoma, congenital tumors), spinal cord neurofibroma, meningioma, glioma, sarcoma); Gynecological: uterus (endometrial carcinoma), cervix (cervical carcinoma, pre-tumor cervical dysplasia), ovaries (ovarian carcinoma [serous cystadenocarcinoma, mucinous cystadenocarcinoma, unclassified carcinoma], granulosa-thecal cell tumors, Sertoli- Leydig cell tumors, dysgerminoma, malignant teratoma), vulva (squamous cell carcinoma, intraepithelial carcinoma, adenocarcinoma, fibrosarcoma, melanoma), vagina (clear cell carcinoma, squamous cell carcinoma, botryoid sarcoma (embryonal rhabdomyosarcoma], fallopian tubes (carcinoma); Hematologic: blood and bone marrow (myeloid leukemia [acute and chronic], acute lymphoblastic leukemia, chronic lymphocytic leukemia, myeloproliferative diseases, multiple myeloma, myelodysplastic syndrome), Hodgkin's disease, non-Hodgkin's lymphoma [malignant lymphoma]; Skin: malignant melanoma, basal cell carcinoma, squamous cell carcinoma, Kaposi's sarcoma, moles dysplastic nevi, lipoma, angioma, dermatofibroma, keloids, psoriasis; neurofibromatosis and Adrenal glands: neuroblastoma. The term "cancerous cell" as provided herein, includes a cell afflicted by any one of the above identified conditions.

In certain embodiments of the present invention, the cancer is a solid tumor cancer.

In certain embodiments of the present invention, the cancer is selected from pancreatic cancer, ovarian cancer and colorectal cancer.

In certain embodiments of the present invention, the cancer is selected from colorectal cancer (including Ras mutations), small cell lung cancer, non-small cell lung cancer, and glioma.

In certain embodiments of the present invention, the cancer is selected from non-small cell lung cancer, ovarian cancer, metastatic breast cancer, pancreatic cancer, hepatobiliary cancer (including hepatocellular cancer, bile duct cancer and cholangiocarcinoma), and gastric cancer.

In certain embodiments of the present invention, the cancer is selected from colorectal cancer (including Ras mutations), small cell lung cancer, non-small cell lung cancer, ovarian cancer, hepatobiliary cancer (including hepatocellular cancer, bile duct cancer and cholangiocarcinoma), gastric cancer, testicular cancer, and head and neck squamous cell carcinoma.

In certain embodiments of the present invention, the cancer is selected from leukemia (including acute myeloid leukemia, acute lymphoblastic leukemia, chronic myeloid leukemia, and chronic lymphoid leukemia), lymphoma (including mantle cell lymphoma, Hodgkin's lymphoma and non-Hodgkin's lymphoma), and prostate cancer
Compounds of the invention will normally be administered orally, intravenously, subcutaneously, buccally, rectally, dermally, nasally, tracheally, bronchially, sublingually, intranasally, topically, by any other parenteral route or *via* inhalation, in a pharmaceutically acceptable dosage form.

Compounds of the invention may be administered alone, but are preferably administered by way of known pharmaceutical compositions/formulations, including tablets, capsules or elixirs for oral administration, suppositories for rectal administration, sterile solutions or suspensions for parenteral or intramuscular administration, and the like.

Compounds of the invention (i.e. compounds that inhibit MTH1) may be administered in the form of tablets or capsules, e.g., time-release capsules that are taken orally. Alternatively, the compounds of the invention may be in a liquid form and may be taken orally or by injection. The compounds of the invention may also be in the form of suppositories, or, creams, gels, and foams e.g. that can be applied to the skin. In addition, they may be in the form of an inhalant that is applied nasally.

Such compositions/formulations may be prepared in accordance with standard and/or accepted pharmaceutical practice.

According to a further aspect of the invention there is thus provided a pharmaceutical composition/formulation including a compound of the invention, as hereinbefore defined, optionally in admixture with a pharmaceutically acceptable adjuvant, diluent and/or carrier. Such compositions/formulations may be of use in the treatment, prevention and/or prophylaxis of cancer and diseases which benefit by inhibition of MTH1.

Depending on e.g. potency and physical characteristics of the compound of the invention (i.e. active ingredient), pharmaceutical formulations that may be mentioned include those in which the active ingredient is present in at least 1% (or at least 10%, at least 30% or at least 50%) by weight. That is, the ratio of active ingredient to the other components (i.e. the addition of adjuvant, diluent and carrier) of the pharmaceutical composition is at least 1:99 (or at least 10:90, at least 30:70 or at least 50:50) by weight.

The invention further provides a process for the preparation of a pharmaceutical formulation, as hereinbefore defined, which process comprises bringing into association a compound of the invention, as hereinbefore defined, or a pharmaceutically acceptable salt thereof with a pharmaceutically-acceptable adjuvant, diluent or carrier.

Also disclosed herein are methods for the treatment of cancer comprising administering a therapeutically effective amount of a compound of the invention to a subject (e.g. patient) in need of such treatment.

"Patients" include mammalian (including human) patients.

The term "effective amount" refers to an amount of a compound, which confers a therapeutic effect on the treated patient. The effect may be objective (i.e. measurable by some test or marker) or subjective (i.e. the subject gives an indication of or feels an effect).

Compounds of the invention may also be combined with other therapeutic agents that are useful in the treatment of cancer.

According to a further aspect of the invention, there is provided a combination product comprising:
(A) a compound of the invention, as hereinbefore defined; and
(B) another therapeutic agent that is useful in the in the treatment of cancer, wherein each of components (A) and (B) is formulated in admixture with a pharmaceutically-acceptable adjuvant, diluent or carrier.

Such combination products provide for the administration of a compound of the invention in conjunction with the other therapeutic agent, and may thus be presented either as separate formulations, wherein at least one of those formulations comprises a compound of the invention, and at least one comprises the other therapeutic agent, or may be presented (i.e. formulated) as a combined preparation (i.e. presented as a single formulation including a compound of the invention and the other therapeutic agent).

Thus, there is further provided:
(1) a pharmaceutical formulation including a compound of the invention, as hereinbefore defined, another therapeutic agent that is useful in the treatment of cancer, and a pharmaceutically-acceptable adjuvant, diluent or carrier; and
(2) a kit of parts comprising components:
   (a) a pharmaceutical formulation including a compound of the invention, as hereinbefore defined, in admixture with a pharmaceutically-acceptable adjuvant, diluent or carrier; and
   (b) a pharmaceutical formulation including another therapeutic agent that is useful in the treatment of cancer in admixture with a pharmaceutically-acceptable adjuvant, diluent or carrier,
      which components (a) and (b) are each provided in a form that is suitable for administration in conjunction with the other.

The invention further provides a process for the preparation of a combination product as hereinbefore defined, which process comprises bringing into association a compound of the invention, as hereinbefore defined, or a pharmaceutically acceptable salt thereof with the other therapeutic agent that is useful in the treatment of cancer, and at least one pharmaceutically-acceptable adjuvant, diluent or carrier.

By "bringing into association", we mean that the two components are rendered suitable for administration in conjunction with each other.

Thus, in relation to the process for the preparation of a kit of parts as hereinbefore defined, by bringing the two components "into association with" each other, we include that the two components of the kit of parts may be:
(i) provided as separate formulations (i.e. independently of one another), which are subsequently brought together for use in conjunction with each other in combination therapy; or
(ii) packaged and presented together as separate components of a "combination pack" for use in conjunction with each other in combination therapy.

Compounds of the invention may be administered at varying doses. Oral, pulmonary and topical dosages (and subcutaneous dosages, although these dosages may be relatively lower) may range from between about 0.01 mg/kg of body weight per day (mg/kg/day) to about 100 mg/kg/day, preferably about 0.01 to about 10 mg/kg/day, and more preferably about 0.1 to about 5.0 mg/kg/day. For e.g. oral administration, the compositions typically contain between about 0.01 mg to about 2000 mg, for example between about 0.1 mg to about 500 mg, or between 1 mg to about 100 mg, of the active ingredient. Intravenously, the most preferred doses will range from about 0.001 to about 10 mg/kg/hour during constant rate infusion. Advantageously, compounds may be administered in a single daily dose, or the total daily dosage may be administered in divided doses of two, three or four times daily.

In any event, the physician, or the skilled person, will be able to determine the actual dosage which will be most suitable for an individual patient, which is likely to vary with the route of administration, the type and severity of the condition that is to be treated, as well as the species, age, weight, sex, renal function, hepatic function and response of the particular patient to be treated. The above-mentioned dosages are exemplary of the average case; there can, of course, be individual instances where higher or lower dosage ranges are merited, and such are within the scope of this invention.

Compounds of the invention may also have the advantage that they may be more efficacious than, be less toxic than, be longer acting than, be more potent than, produce fewer side effects than, be more easily absorbed than, and/or have a better pharmacokinetic profile (e.g. higher oral bioavailability and/or lower clearance) than, and/or have other useful pharmacological, physical, or chemical properties over, compounds known in the prior art, whether for use in the above-stated indications or otherwise. In particular, compounds of the invention may have the advantage that they are more efficacious and/or exhibit advantageous properties *in vivo.*

It is contemplated that any method or composition described herein can be implemented with respect to any other method or composition described herein.

Unless otherwise defined, all technical and scientific terms used herein have the same meaning as commonly understood by one of ordinary skill in the art to which this invention pertains.

### Examples

The invention is illustrated by way of the following examples, in which the following abbreviations may be employed.
- aq: aqueous
- DMF: dimethylformamide
- DMSO: dimethyl sulfoxide
- EtOAc: ethyl acetate
- EtOH: ethanol
- MeOH: methanol
- MeCN: acetonitrile
- Pd-C: palladium on carbon
- sat.: saturated
- TFA: trifluoroacetic acid
- THF: tetrahydrofuran
- min.: minutes
- h.: hours
- Hunigs base: *N,N*-diisopropylethylamine
- DCM: dichloromethane
- n-BuOH: butan-1-ol
- iPrOH: propan-2-ol
- NEt₃: trethylamine
- Boc: *tert*-butoxycabonyl
- HATU: (1-[Bis(dimethylamino)methylene]-1H-1,2,3-triazolo-[4,5-b]pyridinium 3-oxid hexafluorophosphate
- NMP: *N*-methylpyrrolidine
- LCMS: liquid-chromatography electrospray mass spectroscopy
- NMR: nuclear magnetic resonance
- NCS: N-chlorosuccinimide
- Pd(PPh₃)₄: tetrakis(triphenylphosphine)palladium (0)
- B(OMe)₃: trimethylborate
- n-BuLi: n-butyl lithium
- Mel: iodomethane
- NaOMe: sodium methoxide
- CHCl₃: chloroform
- MgSO₄: anhydrous magnesium sulphate
- K₂CO₃: anhydrous potassium carbonate
- NH₄OH: ammonium hydroxide
- Ac₂O: acetic anhydride
- POCl₃: phosphorus oxychloride

Starting materials and chemical reagents specified in the syntheses described below are commercially available, e.g. from Sigma-Aldrich, Fine Chemicals Combi-Blocks and other vendors.

In the event that there is a discrepancy between nomenclature and any compounds depicted graphically, then it is the latter that presides (unless contradicted by any experimental details that may be given or unless it is clear from the context). Final compounds were named using Marvin software version 6.1.

Purification of compounds may be carried out using silica gel column chromatography or preparative reverse phase HPLC (ACE column, acidic gradients with MeCN-H₂O containing 0.1 % TFA or XBridge column, basic gradients using MeCN-H₂O containing ammonium bicarbonate) to give the products as their free bases or trifluoroacetic acid salts.

### Intermediate 18

### 6-chloro-4-N-(2-phenylethyl)pyrimidine-2,4-diamine.

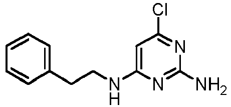

To a solution of 4,6-dichloropyrimidin-2-amine (66 mg, 0.40 mmol, 1 eq.) in n-BuOH (2.5 mL) were added 2-phenylethanamine (75 µL, 0.60 mmol, 1.1 eq.) and Hünig's base (100 µg, 0.60 mmol, 1.1 eq.). The reaction mixture was stirred at 95 °C for 3 h. The solvent was removed in vacuo. The crude product was diluted in EtOAc and washed with H₂O, brine, dried over MgSO₄ and concentrated to afford the desired product as a yellow solid (88 mg, 88 %). LCMS [M+H]⁺ 249.

### Intermediate 19

### 4-chloro-6-(2,3-dimethylphenyl)pyrimidin-2-amine.

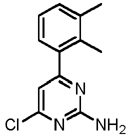

A mixture of 2-amino-4,6-dichloropyrimidine (0.82 g, 5.0 mmol, 1 eq.), 2,3-dimethylphenylboronic acid (0.75 g, 5.0 mmol, 1 eq.), K₂CO₃ (1.38 g, 10.0 mmol, 2 eq.) and palladium tetrakis(triphenylphosphine)palladium (0) (0.12 g, 0.10 mmol, 0.1 eq.) in 1,4-dioxane (20 mL) and water (5 mL) was heated in a sealed tube at 90°C for 2.5 hours. The mixture was run through a plug of silica using EtOAc as eluent, concentrated and purified by column chromatography (1:4 EtOAc/pentane) to give the desired product as a white solid (0.76 g, 65 %). LCMS [M+H]⁺ 234; ¹H NMR (400 MHz, CD₃OD) δ_{H} 7.21 - 7.29 (1H, m), 7.20 - 7.09 (2H, m), 6.70 (1H, s), 2.34 (3H, s), 2.23 (3H, s).

### Intermediate 20

### 4-chloro-6-(2,3-dichlorophenyl)pyrimidin-2-amine.

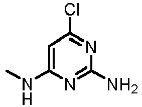

A mixture of 4,6-dichloropyrimidin-2-amine (3.28 g, 20.0 mmol), methanamine (12.0 mL, 24.0 mmol; as a 2 M solution in methanol) and Hünig's base in n-butanol (20 mL) was heated at 95°C overnight. The mixture was concentrated and the crude was taken up in EtOAc (300 mL) and washed with water (3 x 150 mL). The organic layer was dried over MgSO4, filtered and concentrated to give the desired product as a buff solid (2.90 g, 91%). LCMS [M+H]⁺ 159.

### Intermediate 22

### 6-Chloro-4-N-[2-(4-chlorophenyl)ethyl]pyrimidine-2,4-diamine.

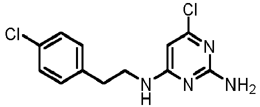

A mixture of 4,6-dichloropyrimidin-2-amine (0.50 g, 3.05 mmol), 2-(4-chlorophenyl)ethan-1-amine (0.56 mL, 3.96 mmol) and Hünig's base (0.80 mL, 4.57 mmol) in n-butanol (5 mL) was heated in a sealed tube at 95°C overnight. The mixture was concentrated and the crude was taken up in EtOAc (50 mL) and washed with water (3 x 40 mL). The organic layer was dried over MgSO4, filtered and concentrated to give the desired product as a buff solid (0.61 g, 71%). LCMS [M+H]⁺ 283.

### Intermediate 24

### 4-chloro-6-(3-chloro-2-methylphenyl)pyrimidin-2-amine.

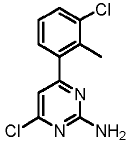

A stirred mixture of 2-amino-4,6-dichloropyrimidine (0.50 g, 3.1 mmol), 3-chloro-2-methylphenylboronic acid (0.57 g, 3.4 mmol), Na₂CO₃ (1.0 g, 9.8 mmol), palladium tetrakis(triphenylphosphine)palladium (0) (88 mg, 0.076 mmol), dioxane (22 mL) and water (8 mL) were heated in a sealed tube at 90°C for 2 hours. The solvents were removed in vacuo and the remaining solid was added EtOAc (20 mL) and washed with water. The organic phase was dried over MgSO₄ and removed in vacuo. The crude material was purified by flash chromatography (1:4 EtOAc/petroleum ether) to give the desired product as a white solid (365 mg, 47%). LCMS [M+H]⁺ 254; ¹H NMR (400 MHz, DMSO-d₆) δ_{H} ppm 7.52 - 7.56 (1 H, dd, J₁ = 6.5 Hz, J₂ = 2.5 Hz) 7.30 - 7.33 (2 H, m) 7.26 (2 H, s) 6.79 (1 H, s) 2.32 (3 H, s).

### Intermediate 35

### 4-N-(2-aminoethyl)-6-(2,3-dimethylphenyl)pyrimidine-2,4-diamine.

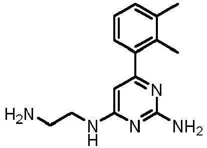

Step 1: To a suspension of 4,6-dichloropyrimidin-2-amine (500 mg, 3.05 mmol) and Hünig's base (0.80 mL) in 2-propanol (3.0 mL) was added tert-butyl N-(2-aminoethyl)carbamate (586 mg, 3.66 mmol) and the mixture was stirred at 150 °C for 15 min. The crude mixture was poured into NaHCO3 (aq) and extracted three times with DCM. The combined organic layers were dried and concentrated. Purification by column chromatography (0→10% MeOH in DCM) afforded tert-butyl N-[2-[(2-amino-6-chloro-pyrimidin-4-yl)amino]ethyl]carbamate (850 mg, 2.95 mmol).

Step 2: tert-Butyl N-[2-[(2-amino-6-chloro-pyrimidin-4-yl)amino]ethyl]carbamate (850 mg, 2.95 mmol), (2,3-dimethylphenyl)boronic acid (532 mg, 3,55 mmol), palladium tetrakis(triphenylphosphine)palladium (0) (34 mg, 0.030 mmol), and K₂CO₃ (1020 mg, 7.39 mmol) were suspended in 1,4-dioxane (10 ml) and H₂O (2.0 ml). The vial was flushed with nitrogen and the resulting mixture was stirred at 90 °C for 16 h. The crude mixture was poured into NaHCO3 (aq) and extracted three times with DCM. The combined organic layers were dried and concentrated. Purification by column chromatography (0→10% MeOH in DCM) afforded tert-butyl N-[2-[[2-amino-6-(2,3-dimethylphenyl)pyrimidin-4-yl]amino]ethyl]carbamate (770 mg, 2.15 mmol).

Step 3: tert-Butyl N-[2-[[2-amino-6-(2,3-dimethylphenyl)pyrimidin-4-yl]amino]ethyl]carbamate (770 mg, 2.15 mmol) was dissolved in TFA (6 mL) and the resulting mixture was stirred for 1 h at rt, after which the TFA was distilled off. Purification by column chromatography (5→30% MeOH [containing 1 v/v% NH₄OH] in DCM) afforded 4-N-(2-aminoethyl)-6-(2,3-dimethylphenyl)pyrimidine-2,4-diamine (500 mg, 1.94 mmol). LCMS [M+H]⁺ 258.

### Intermediate 36

### 4-N-(3-aminopropyl)-6-(2,3-dimethylphenyl)pyrimidine-2,4-diamine.

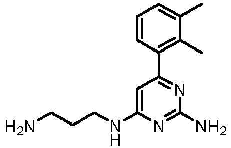

Step 1: A vial was charged with 4,6-dichloropyrimidin-2-amine (500 mg, 3.0 mmol) and tert-butyl N-(2-aminopropyl)carbamate (640 mg, 3.7 mmol). Then 2-propanol (3.0 ml) and Hünig's base (0.80 ml) were added and the resulting mixture was heated at 150 °C using microwave irradiation for 15 min. The mixture was then concentrated and purified by column chromatography (2→10% MeOH in DCM) to afford tert-butyl N-[3-[(2-amino-6-chloro-pyrimidin-4-yl)amino]propyl]-carbamate (788 mg, 2.61 mmol).

Step 2: tert-Butyl N-[3-[(2-amino-6-chloro-pyrimidin-4-yl)amino]propyl]carbamate (790 mg, 2.6 mmol), (2,3-dimethylphenyl)boronic acid (470 mg, 3.1 mmol), palladium tetrakis(triphenylphosphine)palladium (0) (60 mg, 0.050 mmol), and K₂CO₃ (720 mg 5.2 mmol) were suspended in 1,4-dioxane (6.0 ml) and H₂O (1.5 ml). The resulting mixture was heated at 90 °C for 16 h and then poured into H₂O and extracted three times with DCM. The combined organic layers were dried and concentrated. Purification by column chromatography (1→10% MeOH in DCM) afforded tert-butyl N-[3-[[2-amino-6-(2,3-dimethylphenyl)pyrimidin-4-yl]amino]propyl]carbamate (800 mg, 2.1 mmol).

Step 3: tert-Butyl N-[3-[[2-amino-6-(2,3-dimethylphenyl)pyrimidin-4-yl]amino]propyl]carbamate (800 mg, 2.1 mmol) was dissolved in TFA and heated at reflux for 1 h. The TFA was evaporated and the crude residue was purified by column chromatography (2→30% MeOH [containing 1 v/v% NH₄OH] in DCM) to afford 4-N-(3-aminopropyl)-6-(2,3-dimethylphenyl)pyrimidine-2,4-diamine (540 mg, 2.0 mmol). LCMS [M+H]⁺ 272.

### Intermediate 37

### 4-chloro-6-(2-chloro-3-methylphenyl)pyrimidin-2-amine.

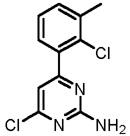

To a suspension of 4,6-dichloropyrimidin-2-amine (250 mg, 1.52 mmol, 1 eq) in dioxane/H₂O (5 mL, 4:1) was added (2-chloro-3-methylphenyl)boronic acid (260 mg, 1.52 mmol, 1 eq) followed by potassium carbonate (421 mg, 3.05 mmol, 2 eq) and Pd(PPh₃)₄ (44 mg, 0.04 mmol, 0.025 eq). The resulting mixture was stirred at 90 °C for 12 hrs. The solvent was removed in vacuo. The residue was taken up in DMF and purified by preparative to afford the desired product as an off-white solid (166 mg, 43%). LCMS [M+H]⁺ 254; ¹H NMR (400 MHz, CDCl₃) δ_{H} 7.32 - 7.28 (2H, m), 7.25 - 7.21 (1H, m), 6.92 (1H, s), 5.31 (2H, br s), 2.42 (3H, s).

### Intermediate 39

### 4-chloro-6-(1H-indol-4-yl)pyrimidin-2-amine.

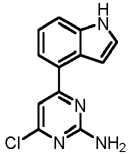

To a suspension of 4,6-dichloropyrimidin-2-amine (150 mg, 0.91 mmol, 1 eq) in dioxane/H₂O (5 mL, 4:1) was added (1H-indol-4-yl)boronic acid (147 mg, 0.91 mmol, 1 eq) followed by potassium carbonate (253 mg, 1.83 mmol, 2 eq) and Pd(PPh₃)₄ (26 mg, 0.02 mmol, 0.025 eq). The resulting mixture was stirred at 90 °C for 12 hrs. The solvent was removed in vacuo and the residue was purified by preparative HPLC to afford the desired product as a yellow solid (111 mg, 50%). LCMS [M+H]⁺ 245; ¹H NMR (400 MHz, CDCl₃) δ_{H} 8.45 (1H, br s), 7.57 (1H, d, J = 7.2 Hz), 7.51 (1H, d, J = 8.0 Hz), 7.35 (1H, t, J = 4.0 Hz), 7.27 (1H, d, J = 7.6 Hz), 7.18 (1H, s), 7.00 - 6.99 (1H, m), 5.90 (2H, br s).

### General procedures

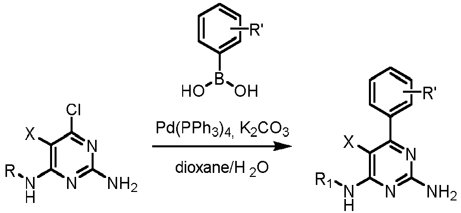

X = H or Me

*General Procedure* 2: To a mixture of a suitable chloropyrimidine derivative (1 equiv.) in 1,4-dioxane/water (4:1) is added the appropriate boronic acid (or boronic ester) derivative (1.3 equiv.), K₂CO₃ (2 equiv.) and Pd(PPh₃)₄ (0.1 equiv.). The mixture is heated at 95 °C overnight or in a microwave reactor until the reaction is complete as shown by LCMS. The crude mixture is purified by preparative HPLC to afford the desired product. 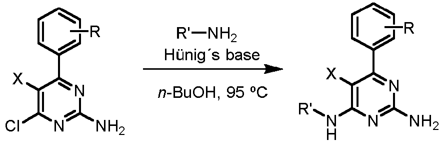 X = H, Me

*General Procedure 3:* To a mixture of a suitable chloropyrimidine derivative (1 equiv.) is added Hünig's base (3.4 equiv.) and an appropriate amine (1.6 equiv.) in n-BuOH (500 µL). The mixture is heated at 95 °C overnight. The crude mixture was purified by preparative HPLC to afford the desired product.

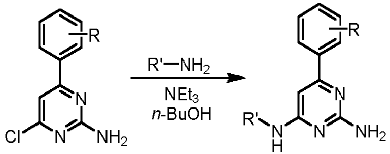

*General procedure* 7: A mixture of a suitable 6-aryl-4-chloropyrimidin-2-amine (1 equiv.), a suitable amine (1.5 equiv.) and triethylamine (2 equiv.) in n-butanol (1.5 mL) is heated in a sealed tube at 95°C overnight. Concentrated and purified by preparative HPLC to give the desired product.

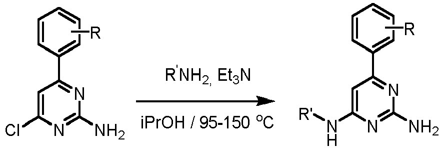

General procedure 9: A mixture of a suitable amine (1 equiv.), and a suitable chloropyrimidine derivative (1.2 equiv.) and triethylamine (1.5 equiv.) in 2-propanol (1.0 mL) is heated in a sealed tube at 95 °C overnight or at 150 °C for 15 min in a microwave reactor. The reaction mixture is then concentrated and purified by preparative HPLC or by silica gel chromatography. 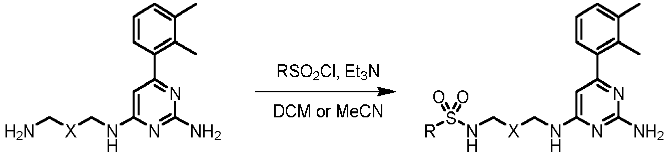 X = CH2, CH2CH2, CH(CH₃)₂ or a bond

General procedure 10: A mixture of an 4-N-(aminoalkyl)-6-(2,3-dimethylphenyl)pyrimidine-2,4-diamine (1.0 equiv.), a suitable sulfonyl chloride (1.2 equiv.), and triethylamine (1.5 equiv.) in DCM or MeCN (1.0 mL) is stirred in a sealed tube at rt or 50 °C. After completion the crude mixture is concentrated and purified by preparative HPLC or by silica gel chromatography.

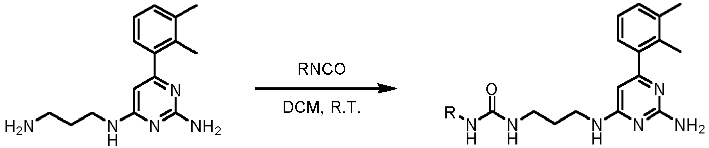

General procedure 11: A mixture of 4-N-(3-aminopropyl)-6-(2,3-dimethylphenyl)pyrimidine-2,4-diamine (1.0 eq.) and the corresponding isocyanate (1.05 eq.) is dissolved in DCM. The resulting reaction mixture is stirred at rt until completion according to LCMS. The mixture is then concentrated and purified by preparative HPLC or by silica gel chromatography.

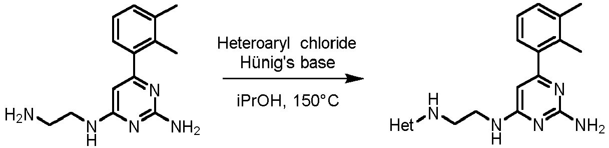

General procedure 12: A mixture of 4-N-(2-aminoethyl)-6-(2,3-dimethylphenyl)pyrimidine-2,4-diamine (1.0 equiv.) and the corresponding heteroaryl chloride (1.5 equiv.), and Hünig's base (1.5 equiv.) in 2-propanol (0.5 mL) is stirred in a sealed tube at 150 °C in a microwave reactor for 30 min. The crude mixture is then concentrated and purified by preparative HPLC or by silica gel chromatography.

### EXAMPLES

The utility of the compounds of the present invention can be illustrated, for example, by their activity in vitro in the cancer cell clonogenic and/or viability assay described below. The link between activity in tumor cell clonogenic assay and anti-tumor activity in the clinical setting has been well established in the art (e.g. see ref Silverstini et al Stem Cells 1993, 11(6), 258-35).

### Example 118

6-(2,3-dimethylphenyl)-4-N-(2-phenylethyl)pyrimidine-2,4-diamine.
Prepared according to general procedure 2 from (2,3-dimethylphenyl)boronic acid and 6-chloro-4-N-(2-phenylethyl)pyrimidine-2,4-diamine. LCMS [M+H]⁺ 319; ¹H NMR (400 MHz, CD₃OD) δ_{H} 7.31 - 7.23 (4H, m), 7.21 - 7.15 (2H, m), 7.10 (1H, t, J = 7.0 Hz), 7.05 (1H, d, J = 7.0 Hz), 5.77 (1H, s), 3.64 - 3.50 (2H, m), 2.90 (2H, t, J = 7.6 Hz), 2.31 (3H, s), 2.19 (3H, s).

### Example 125

6-(2,3-dimethylphenyl)-4-N-[2-(4-methoxyphenyl)ethyl]pyrimidine-2,4-diamine.
Prepared according to general procedure 7 from 2-(4-methoxyphenyl)ethan-1-amine and 4-chloro-6-(2,3-dimethylphenyl)pyrimidin-2-amine. LCMS [M+H]⁺ 349.

### Example 127

6-(2,3-dimethylphenyl)-4-N-[2-(4-methylphenyl)ethyl]pyrimidine-2,4-diamine.
Prepared according to general procedure 7 from 2-(4-methylphenyl)ethan-1-amine and 4-chloro-6-(2,3-dimethylphenyl)pyrimidin-2-amine. LCMS [M+H]⁺ 333.

### Example 128

4-N-[2-(4-chlorophenyl)ethyl]-6-(2,3-dimethylphenyl)pyrimidine-2,4-diamine.
Prepared according to general procedure 7 from 2-(4-chlorophenyl)ethan-1-amine and 4-chloro-6-(2,3-dimethylphenyl)pyrimidin-2-amine. LCMS [M+H]⁺ 353.

### Example 130

6-(2,3-dimethylphenyl)-4-N-(2-phenylpropyl)pyrimidine-2,4-diamine.
Prepared according to general procedure 7 from 2-phenylpropan-1-amine and 4-chloro-6-(2,3-dimethylphenyl)pyrimidin-2-amine. LCMS [M+H]⁺ 333.

### Example 131

6-(2,3-dimethylphenyl)-4-N-(3-phenylpropyl)pyrimidine-2,4-diamine.
Prepared according to general procedure 7 from 3-phenylpropan-1-amine and 4-chloro-6-(2,3-dimethylphenyl)pyrimidin-2-amine. LCMS [M+H]⁺ 333.

### Example 132

6-(2,3-dimethylphenyl)-4-N-(2-phenoxyethyl)pyrimidine-2,4-diamine.
Prepared according to general procedure 7 from (2-aminoethoxy)benzene and 4-chloro-6-(2,3-dimethylphenyl)pyrimidin-2-amine. LCMS [M+H]⁺ 335.

### Example 133

6-(2,3-dimethylphenyl)-4-N-[2-(phenylamino)ethyl]pyrimidine-2,4-diamine.
Prepared according to general procedure 7 from N-(2-aminoethyl)aniline and 4-chloro-6-(2,3-dimethylphenyl)pyrimidin-2-amine. LCMS [M+H]⁺ 334.

### Example 228

6-(2-chloro-3-methylphenyl)-4-N-[2-(4-chlorophenyl)ethyl]pyrimidine-2,4-diamine.
Prepared according to general procedure 3 from 2-(4-chlorophenyl)ethan-1-amine and 4-chloro-6-(2-chloro-3-methylphenyl)pyrimidin-2-amine. LCMS [M+H]⁺ 373; ¹H NMR (400 MHz, CD₃OD) δ_{H} 7.57 - 7.55 (1H, m), 7.41 (1H, t, J = 7.6 Hz), 7.37 (1H, dd, J = 8.0 and 2.0 Hz), 7.34 - 7.28 (4H, m), 6.08 (1H, s), 3.79 (2H, t, J = 7.2 Hz), 2.97 (2H, t, J = 7.2 Hz), 2.49 (3H, s).

### Example 231

4-N-[2-(4-chlorophenyl)ethyl]-6-(1H-indol-4-yl)pyrimidine-2,4-diamine.
Prepared according to general procedure 3 from 2-(4-chlorophenyl)ethan-1-amine and 4-chloro-6-(1H-indol-4-yl)pyrimidin-2-amine. LCMS [M+H]⁺ 364; ¹H NMR (400 MHz, CD₃OD) δ_{H} 7.67 - 7.66 (1H, m), 7.52 - 7.46 (1H, m), 7.35 - 7.29 (6H, m), 6.86 (1H, s), 6.44 (1H, s), 3.80 (2H, t, J = 7.2 Hz), 2.95 (2H, t, J = 6.8 Hz).

### Example 239 (Not according to the invention)

tert-Butyl (3-((2-amino-6-(2,3-dimethylphenyl)pyrimidin-4-yl)amino)-2,2-dimethylpropyl)carbamate.
Prepared according to general procedure 3 from 4-chloro-6-(3-chloro-2-methylphenyl)pyrimidin-2-amine and tert-butyl N-(3-amino-2,2-dimethylpropyl)carbamate. LCMS [M+H]⁺ 400.

### Example 241

6-(3-chloro-2-methylphenyl)-4-N-[(1R)-1-phenylethyl]pyrimidine-2,4-diamine.
4-Chloro-6-(3-chloro-2-methylphenyl)pyrimidin-2-amine (25 mg, 0,098 mmol) and (+)1-phenylethan-1-amine (0,050 mL) were stirred neat at 150°C for 1 hour. The crude material was dissolved in MeOH (2 mL) and purified by preparative HPLC to give the desired product. LCMS [M+H]⁺ 339; ¹H NMR (400 MHz, DMSO-d₆) δ_{H} ppm 9.25 (1 H, d, J=8.08 Hz) 7.62 - 7.71 (1 H, m) 7.24 - 7.47 (7 H, m) 6.23 (0.1 H, s) 6.09 (0.9 H, s) 5.35 (1 H, quin, J=7.26 Hz) 2.31 (2.7 H, s) 2.13 (0.3 H, s) 1.46 - 1.57 (3 H, m).

### Example 242

6-(3-chloro-2-methylphenyl)-4-N-(2-phenylpropan-2-yl)pyrimidine-2,4-diamine.
4-Chloro-6-(3-chloro-2-methylphenyl)pyrimidin-2-amine (25 mg, 0,098 mmol) and 2-phenylpropan-2-amine (0,050 mL) were stirred neat at 150°C for 24 hours. The crude material was dissolved in MeOH (2 mL) and purified by preparative HPLC to give the desired product. LCMS [M+H]⁺ 353; ¹H NMR (400 MHz, DMSO-d₆) δ_{H} ppm 12.45 (1 H, br. s.) 8.96 (1 H, br. s.) 7.12 - 7.79 (8 H, m) 6.22 (1 H, s) 2.31 (3 H, s) 1.79 (6 H, br. s.)

### Example 247

4-N-[2-(2-chlorophenoxy)ethyl]-6-(2,3-dimethylphenyl)pyrimidine-2,4-diamine.
4-Chloro-6-(2,3-dimethylphenyl)pyrimidin-2-amine (13 mg, 0,054 mmol) and 1-(2-aminoethoxy)-2-chlorobenzene (18 mg, 0,11 mmol) were stirred neat at 150°C for 1 h. The crude material was dissolved in MeOH (1 mL) and purified by preparative HPLC to give the desired product. LCMS [M+H]⁺ 369.

### Example 250

6-(2,3-dimethylphenyl)-4-N-[2-(pyridin-3-yloxy)propyl]pyrimidine-2,4-diamine.
4-Chloro-6-(2,3-dimethylphenyl)pyrimidin-2-amine (12 mg, 0,050 mmol) and 3-[(1-aminopropan-2-yl)oxy]pyridine (15 mg, 0,10 mmol) were stirred neat at 150°C for 1 h. The crude material was dissolved in MeOH (1 mL) and purified by preparative HPLC to give the desired product. LCMS [M+H]⁺ 350.

### Example 263

4-N-[2-(4-chlorophenyl)ethyl]-6-(2,3-dichlorophenyl)pyrimidine-2,4-diamine.
A mixture of 4-chloro-6-(2,3-dichlorophenyl)pyrimidin-2-amine (28 mg, 0.10 mmol), 2-(4-chlorophenyl)ethan-1-amine (22 mg, 0.14 mmol) and Hünig's base (36 µL, 0.20 mmol) in n-butanol (2 mL) was heated in a sealed tube at 85°C overnight. The reaction mixture was concentrated and purified by preparative HPLC. LCMS [M+H]⁺ 393. 1H NMR (400 MHz, DMSO-d6) δ_{H} ppm 12.56 (br. s., 1 H), 8.90 (br. s., 1 H), 7.83 - 7.89 (m, 1 H), 7.51 - 7.59 (m, 2 H), 7.35 - 7.41 (m, 2 H), 7.28 - 7.34 (m, 2 H), 6.11 (s, 1 H), 3.63 (q, J=6.4 Hz, 2 H), 2.89 (t, J=7.1 Hz, 2 H).

### Example 266

4-N-[2-(4-chlorophenyl)ethyl]-6-(quinolin-5-yl)pyrimidine-2,4-diamine.
A mixture of 6-chloro-4-N-[2-(4-chlorophenyl)ethyl]pyrimidine-2,4-diamine (34 mg, 0.12 mmol), (quinolin-5-yl)boronic acid (25 mg, 0.14 mmol), potassium carbonate (33 mg, 0.24 mmol) and palladium tetrakis(triphenylphosphine)palladium (0) (7 mg, 0.006 mmol) in 1,4-dioxane/water (4 mL; 4:1) was heated in a sealed tube at 90°C overnight. The reaction mixture was concentrated and purified by preparative HPLC. LCMS [M+H]⁺ 376.

### Example 267

4-N-[2-(4-chlorophenyl)cyclopropyl]-6-(2,3-dimethylphenyl)pyrimidine-2,4-diamine.
A mixture of 4-chloro-6-(2,3-dimethylphenyl)pyrimidin-2-amine (28 mg, 0.12 mmol), 2-(4-chlorophenyl)cyclopropan-1-amine (43 mg, 0.26 mmol) and Hünig's base (90 µL, 0.52 mmol) in n-butanol (2 mL) was heated in a sealed tube at 85°C overnight. The reaction mixture was concentrated and purified by preparative HPLC. LCMS [M+H]⁺ 365.

### Example 269

3-(2-{[2-amino-6-(2,3-dimethylphenyl)pyrimidin-4-yl]amino}ethyl)phenol.
A mixture of 4-chloro-6-(2,3-dimethylphenyl)pyrimidin-2-amine (28 mg, 0.12 mmol), 3-(2-aminoethyl)phenol (23 mg, 0.17 mmol) and Hünig's base (42 µL, 0.24 mmol) in n-butanol (2 mL) was heated in a sealed tube at 85°C overnight. The reaction mixture was concentrated and purified by preparative HPLC. LCMS [M+H]⁺ 335.

### Example 295

4-N-{[1-(4-chlorophenyl)cyclopropyl]methyl}-6-(2,3-dimethylphenyl)pyrimidine-2,4-diamine.
A mixture of 4-chloro-6-(2,3-dimethylphenyl)pyrimidin-2-amine (47 mg, 0.20 mmol), [1-(4-chlorophenyl)cyclopropyl]methanamine hydrochloride (65 mg, 0.30 mmol) and Hünig's base (70 µL, 0.40 mmol) in n-butanol (1.5 mL) was heated in a sealed tube at 130°C overnight. The reaction mixture was concentrated and purified by preparative HPLC. LCMS [M+H]⁺ 379.

### Example 296

4-(2-{[2-amino-6-(2,3-dichlorophenyl)pyrimidin-4-yl]amino}ethyl)benzene-1-sulfonamide.
A mixture of 4-chloro-6-(2,3-dichlorophenyl)pyrimidin-2-amine (41 mg, 0.15 mmol), 4-(2-aminoethyl)benzene-1-sulfonamide (45 mg, 0.23 mmol) and Hünig's base (39 µL, 0.23 mmol) in n-butanol (2 mL) was heated in a sealed tube at 90°C for 48 h. The reaction mixture was concentrated and purified by preparative HPLC. LCMS [M+H]⁺ 438.

### Example 297

4-(2-{[2-amino-6-(3-chloro-2-methylphenyl)pyrimidin-4-yl]amino}ethyl)benzene-1-sulfonamide.
A mixture of 4-chloro-6-(3-chloro-2-methylphenyl)pyrimidin-2-amine (38 mg, 0.15 mmol), 4-(2-aminoethyl)benzene-1-sulfonamide (45 mg, 0.23 mmol) and Hünig's base (39 µL, 0.23 mmol) in n-butanol (2 mL) was heated in a sealed tube at 90°C for 48 h. The reaction mixture was concentrated and purified by preparative HPLC. LCMS [M+H]⁺ 418. ¹H NMR (400 MHz, DMSO-d6) δ_{H} ppm 12.33 (br. s., 1 H), 8.89 (br. s., 1 H), 7.77 (d, J=8.1 Hz, 2 H), 7.64 - 7.68 (m, 1 H), 7.48 (d, J=8.1 Hz, 2 H), 7.37 - 7.41 (m, 2 H), 7.32 (s, 2 H), 6.03 (s, 1 H), 3.67 (q, J=6.7 Hz, 2 H), 2.98 (t, J=7.1 Hz, 2 H), 2.30 (s, 3 H).

### Example 301

4-(2-{[2-Amino-6-(2,3-dichlorophenyl)pyrimidin-4-yl]amino}ethyl)phenol.
A mixture of 4-chloro-6-(2,3-dichlorophenyl)pyrimidin-2-amine (25 mg) and 4-(2-aminoethyl)phenol (50 mg) was heated at 150°C for 1 h. The mixture was cooled, diluted in methanol then purified by preparative HPLC. LCMS [M+H]⁺ 375.

### Example 330

4-(2-{[2-Amino-6-(2,3-dimethylphenyl)pyrimidin-4-yl]amino}ethyl)benzene-1-sulfonamide.
A mixture of 4-chloro-6-(2,3-dimethylphenyl)pyrimidin-2-amine (0.12 mmol), 4-(2-aminoethyl)benzene-1-sulfonamide (0.9 eq.) and triethylamine (1.5 eq.) in acetonitrile/ethanol/methanol 5:3:2 (1 mL) was heated in a sealed tube at 95°C overnight. Methanol was added, and the mixture was filtered and purified by preparative HPLC. LCMS [M+H]⁺ 398; ¹H NMR (400 MHz, DMSO-d₆) δ_{H} ppm 12.28 (1 H, br. s.), 8.87 (1 H, br. s.), 7.77 (2 H, d, J=8.34 Hz), 7.48 (2 H, d, J=8.34 Hz), 7.37 (1 H, d, J=7.33 Hz), 7.32 (2 H, s), 7.23 - 7.28 (1 H, m), 7.18 - 7.23 (1 H, m), 5.99 (1 H, s), 3.67 (2 H, q, J=6.82 Hz), 2.98 (2 H, t, J=7.20 Hz), 2.31 (3 H, s), 2.17 (3 H, s).

### Example 331

6-(2,3-Dimethylphenyl)-4-N-[2-(4-methanesulfonylphenyl)ethyl]pyrimidine-2,4-diamine.
A mixture of 4-chloro-6-(2,3-dimethylphenyl)pyrimidin-2-amine (0.15 mmol), 2-(4-methanesulfonylphenyl)ethan-1-amine (1.2 eq.) and N,N-diisopropylethylamine (2.25 eq.) in n-butanol (0.3 mL) was heated in a sealed tube at 110°C overnight. Methanol was added and the mixture was filtered and purified by preparative HPLC. LCMS [M+H]⁺ 397.

### Example 333

4-(2-{[2-Amino-6-(2,3-dimethylphenyl)pyrimidin-4-yl]amino}ethyl)benzonitrile.
A mixture of 4-chloro-6-(2,3-dimethylphenyl)pyrimidin-2-amine (0.15 mmol), 4-(2-aminoethyl)benzonitrile (1.2 eq.) and N,N-diisopropylethylamine (2.25 eq.) in n-butanol (0.3 mL) was heated in a sealed tube at 110°C overnight. Methanol was added, and the mixture was filtered and purified by preparative HPLC. LCMS [M+H]⁺ 344; ¹H NMR (400 MHz, DMSO-d₆) δ_{H} ppm 12.36 (1 H, br. s.), 8.87 (1 H, t, J=5.31 Hz), 7.80 (2 H, d, J=8.08 Hz), 7.51 (2 H, d, J=8.34 Hz), 7.37 (1 H, d, J=7.33 Hz), 7.23 - 7.28 (1 H, m), 7.16 - 7.22 (1 H, m), 5.98 (1 H, s), 3.67 (2 H, q, J=6.65 Hz), 2.99 (2 H, t, J=7.20 Hz), 2.31 (3 H, s), 2.17 (3 H, s).

### Example 335

4-(2-{[2-Amino-6-(2,3-dimethylphenyl)pyrimidin-4-yl]amino}ethoxy)benzonitrile.
A mixture of 4-chloro-6-(2,3-dimethylphenyl)pyrimidin-2-amine (0.15 mmol), 4-(2-aminoethoxy)benzonitrile (1.2 eq.) and N,N-diisopropylethylamine (1.25 eq.) in n-butanol (0.3 mL) was heated in a sealed tube at 110°C overnight. Methanol was added, and the mixture was filtered and purified by preparative HPLC. LCMS [M+H]⁺ 360; ¹H NMR (400 MHz, DMSO-d₆) δ_{H} ppm 12.37 (1 H, br. s.), 9.02 (1 H, t, J=5.31 Hz), 7.77 - 7.83 (2 H, m), 7.37 (1 H, d, J=7.33 Hz), 7.23 - 7.29 (1 H, m), 7.18 - 7.22 (1 H, m), 7.12 - 7.18 (2 H, m), 6.06 (1 H, s), 4.29 (2 H, t, J=5.43 Hz), 3.82 (2 H, q, J=5.22 Hz), 2.31 (3 H, s), 2.17 (3 H, s).

### Example 341

4-N-[2-(3-Chlorophenyl)ethyl]-6-(2,3-dimethylphenyl)pyrimidine-2,4-diamine.
Prepared according to general procedure 9 from 2-(3-chlorophenyl)ethan-1-amine and 4-chloro-6-(2,3-dimethylphenyl)pyrimidin-2-amine. LCMS [M+H]⁺ 353; ¹H NMR (400 MHz, CDCl₃) δ_{H} ppm 2.24 (s, 3 H) 2.31 (s, 3 H) 2.88 (t, J=7.07 Hz, 2 H) 3.56 (d, J=5.81 Hz, 2 H) 4.86 - 5.12 (m, 3 H) 5.79 (s, 1 H) 7.07 - 7.15 (m, 3 H) 7.17 (q, J=4.04 Hz, 1 H) 7.20 - 7.26 (m, 3 H).

### Example 343

4-N-[2-(4-Chlorophenyl)propyl]-6-(2,3-dimethylphenyl)pyrimidine-2,4-diamine.
Prepared according to general procedure 9 from 2-(4-chlorophenyl)propan-1-amine and 4-chloro-6-(2,3-dimethylphenyl)pyrimidin-2-amine. LCMS [M+H]⁺ 367; ¹H NMR (400 MHz, CDCl₃) δ_{H} ppm 1.31 (d, J=7.07 Hz, 3 H) 2.23 (s, 3 H) 2.31 (s, 3 H) 2.97 - 3.09 (m, 1 H) 3.28 - 3.40 (m, 1 H) 3.46 - 3.66 (m, 1 H) 4.74 (br. s., 1 H) 4.95 (br. s., 2 H) 5.74 (s, 1 H) 7.09 - 7.14 (m, 2 H) 7.14 - 7.19 (m, 3 H) 7.28 - 7.33 (m, 2 H).

### Example 344

6-(2,3-Dimethylphenyl)-4-N-[2-(4-fluorophenyl)ethyl]pyrimidine-2,4-diamine.
Prepared according to general procedure 9 from 2-(4-fluorophenyl)ethan-1-amine and 4-chloro-6-(2,3-dimethylphenyl)pyrimidin-2-amine. LCMS [M+H]⁺ 337; ¹H NMR (400 MHz, CDCl₃) δ_{H} ppm 2.23 (s, 3 H) 2.29 - 2.34 (m, 3 H) 2.89 - 2.96 (m, 3 H) 3.49 - 3.60 (m, 1 H) 3.76 (d, J=6.06 Hz, 3 H) 5.77 (s, 4 H) 7.00 - 7.07 (m, 3 H) 7.08 - 7.12 (m, 2 H) 7.13 - 7.22 (m, 5 H) 7.28 - 7.32 (m, 1 H).

### Example 347

4-N-[2-(Benzenesulfonyl)ethyl]-6-(2,3-dimethylphenyl)pyrimidine-2,4-diamine.
Prepared according to general procedure 9 from 2-(benzenesulfonyl)ethan-1-amine and 4-chloro-6-(2,3-dimethylphenyl)pyrimidin-2-amine. LCMS [M+H]⁺ 383; ¹H NMR (400 MHz, CDCl₃) δ_{H} ppm 2.22 (s, 3 H) 2.31 (s, 3 H) 3.41 - 3.46 (m, 2 H) 3.86 (q, J=6.06 Hz, 2 H) 5.10 (br. s., 2 H) 5.41 - 5.53 (m, 1 H) 5.76 (s, 1 H) 7.07 - 7.16 (m, 2 H) 7.16 - 7.20 (m, 1 H) 7.57 - 7.63 (m, 2 H) 7.66 - 7.71 (m, 1 H) 7.92 - 7.97 (m, 2 H),

### Example 357

N-(3-{[2-Amino-6-(2,3-dimethylphenyl)pyrimidin-4-yl]amino}propyl)-4-chlorobenzene-1-sulfonamide.
Prepared according to general procedure 10 from 4-chlorobenzene-1-sulfonyl chloride and 4-N-(3-aminopropyl)-6-(2,3-dimethylphenyl)pyrimidine-2,4-diamine. LCMS [M+H]⁺ 446; ¹H NMR (400 MHz, CDCl₃) δ_{H} ppm 1.72 (quin, J=6.06 Hz, 2 H) 2.21 (s, 3 H) 2.30 (s, 3 H) 2.90 (d, J=5.05 Hz, 2 H) 3.49 (q, J=6.32 Hz, 2 H) 3.44 - 3.52 (m, 2 H) 5.33 - 5.43 (m, 1 H) 5.57 (br. s., 2 H) 5.78 (s, 1 H) 7.05 - 7.09 (m, 1 H) 7.09 - 7.13 (m, 1 H) 7.16 - 7.20 (m, 1 H) 7.41 - 7.46 (m, 2 H) 7.79 - 7.84 (m, 2 H).

### Example 358

3-(3-{[2-Amino-6-(2,3-dimethylphenyl)pyrimidin-4-yl]amino}propyl)-1-(4-chlorophenyl)urea.
Prepared according to general procedure 11 from 1-chloro-4-isocyanatobenzene and 4-N-(3-aminopropyl)-6-(2,3-dimethylphenyl)pyrimidine-2,4-diamine. LCMS [M+H]⁺ 425; ¹H NMR (400 MHz, CD₃OD) δ_{H} ppm 1.85 (t, J=6.69 Hz, 2 H) 2.23 (s, 3 H) 2.34 (s, 3 H) 3.28 (overlap with methanol) 3.50 - 3.61 (m, 2 H) 5.97 (s, 1 H) 7.15 (s, 1 H) 7.17 - 7.25 (m, 3 H) 7.28 - 7.37 (m, 3 H).

### Example 359

N-(3-{[2-Amino-6-(2,3-dimethylphenyl)pyrimidin-4-yl]amino}propyl)-3,5-dimethy!-1,2-oxazole-4-sulfonamide.
Prepared according to general procedure 10 from dimethyl-1,2-oxazole-4-sulfonyl chloride and 4-N-(3-aminopropyl)-6-(2,3-dimethylphenyl)pyrimidine-2,4-diamine. LCMS [M+H]⁺ 431; ¹H NMR (400 MHz, CDCl₃) δ_{H} ppm 1.79 (br. quin, J=6.0 Hz, 2 H) 2.23 (s, 3 H) 2.30 (s, 3 H) 2.42 (s, 3 H) 2.64 (s, 3 H) 3.01 (br. q, J=5.90 Hz, 2 H) 3.53 (br. q, J=6.00 Hz, 2 H) 5.89 (s, 1 H) 7.05 - 7.13 (m, 2 H) 7.18 - 7.21 (m, 1 H).

### Example 361

3-(3-{[2-Amino-6-(2,3-dimethylphenyl)pyrimidin-4-yl]amino}propyl)-1-(2,6-dichloropyridin-4-yl)urea.
Prepared according to general procedure 11 from 2,6-dichloro-4-isocyanatopyridine and 4-N-(3-aminopropyl)-6-(2,3-dimethylphenyl)pyrimidine-2,4-diamine. LCMS [M+H]⁺ 460; ¹H NMR (400 MHz, CDCl₃) δ_{H} ppm 1.86 (br. quint, J=6.7 Hz, 2 H) 2.24 (s, 3 H) 2.34 (s, 3 H) 3.30 - 3.34 (m [overlap w MeOH], 2H) 3.55 (br. s., 3 H) 5.98 (s, 1 H) 7.11 - 7.24 (m, 2 H) 7.27 - 7.33 (m, 1 H) 7.46 (s, 2 H).

### Example 362

3-(3-{[2-Amino-6-(2,3-dimethylphenyl)pyrimidin-4-yl]amino}propyl)-1-(3,4-difluorophenyl)urea.
Prepared according to general procedure 11 from 1,2-difluoro-4-isocyanatobenzene and 4-N-(3-aminopropyl)-6-(2,3-dimethylphenyl)pyrimidine-2,4-diamine. LCMS [M+H]⁺ 427; ¹H NMR (400 MHz, CDCl₃) δ_{H} ppm 1.81 - 1.89 (m, 2 H) 2.24 (s, 3 H) 2.34 (s, 3 H) 3.25 - 3.30 (m [overlap w MeOH], 2H) 3.55 (br. s., 2 H) 5.98 (s, 1 H) 6.94 - 7.01 (m, 1 H) 7.06 - 7.17 (m, 2 H) 7.17 - 7.23 (m, 1 H) 7.27 - 7.33 (m, 1 H) 7.45 - 7.54 (m, 1 H).

### Example 363

N-[3-(3-{[2-Amino-6-(2,3-dimethylphenyl)pyrimidin-4-yl]amino}propoxy)phenyl]acetamide.
Prepared according to general procedure 9 from N-[3-(3-aminopropoxy)phenyl]acetamide and 4-chloro-6-(2,3-dimethylphenyl)pyrimidin-2-amine. LCMS [M+H]⁺ 406; ¹H NMR (400 MHz, DMSO-d₆) δ_{H} ppm 1.96 (t, J=6.32 Hz, 2 H) 2.02 (s, 3 H) 2.15 (s, 3 H) 2.25 (s, 3 H) 4.00 (t, J=6.32 Hz, 2 H) 5.70 (s, 1 H) 5.92 (br. s., 2 H) 6.61 (dd, J=8.46, 1.64 Hz, 1 H) 6.85 - 6.99 (m, 1 H) 6.99 - 7.10 (m, 3 H) 7.11 - 7.19 (m, 2 H) 7.31 (s, 1 H) 9.88 (s, 1 H). A signal from one of the CH₂-groups is overlapping with solvent peaks and is not observed by NMR.

### Example 367

N-(3-{[2-Amino-6-(2,3-dimethylphenyl)pyrimidin-4-yl]amino}propyl)-3-bromobenzamide.
4-N-(3-aminopropyl)-6-(2,3-dimethylphenyl)pyrimidine-2,4-diamine (1.0 eq) was dissolved in THF (0.50 mL) and NMP (0.050 mL), then Et₃N (1.5 eq) and 3-bromobenzoyl chloride (1.2 eq) were added. The resulting reaction mixture was stirred at rt overnight. The mixture was then concentrated and purified by column chromatography. LCMS [M+H]⁺ 454; ¹H NMR (400 MHz, CDCl₃) δ_{H} ppm 1.80 - 1.89 (m, 2 H) 2.23 (s, 3 H) 2.30 (s, 3 H) 2.37 - 2.59 (m, 1 H) 3.47 - 3.58 (m, 4 H) 5.14 (br. s., 2 H) 5.55 - 5.69 (m, 1 H) 5.85 (s, 1 H) 7.07 - 7.13 (m, 2 H) 7.17 (dd, J=6.19, 2.91 Hz, 1 H) 7.31 (t, J=7.83 Hz, 1 H) 7.63 (ddd, J=7.89, 1.96, 1.01 Hz, 1 H) 7.75 (dd, J=7.83, 1.01 Hz, 1 H) 7.98 (t, J=1.77 Hz, 1 H).

### Example 368

N-(3-{[2-Amino-6-(2,3-dimethylphenyl)pyrimidin-4-yl]amino}propyl)benzamide.
In a vial 4-N-(3-aminopropyl)-6-(2,3-dimethylphenyl)pyrimidine-2,4-diamine (1.0 eq) was dissolved in NMP (0.050 mL) and THF (0.50 mL), then Et₃N (1.5 eq) and benzoyl chloride (1.0 eq) were added. The resulting reaction mixture was stirred at rt for 1 h. The mixture was then concentrated and purified by column chromatography. LCMS [M+H]⁺ 376; ¹H NMR (400 MHz, CDCl₃) δ ppm 1.86 (t, J=6.06 Hz, 2 H) 2.22 (s, 3 H) 2.29 (s, 3 H) 3.54 (dt, J=12.32, 6.09 Hz, 4 H) 5.36 - 5.54 (m, 1 H) 5.89 (s, 1 H) 6.14 - 6.31 (m, 1 H) 7.06 - 7.13 (m, 2 H) 7.16 - 7.20 (m, 1 H) 7.40 - 7.46 (m, 2 H) 7.47 - 7.53 (m, 1 H) 7.80 - 7.85 (m, 2 H).

### Example 374

N-(3-{[2-Amino-6-(2,3-dimethylphenyl)pyrimidin-4-yl]amino}propyl)-3-cyanobenzene-1-sulfonamide.
Prepared according to general procedure 10 from 3-cyanobenzene-1-sulfonyl chloride and 4-N-(3-aminopropyl)-6-(2,3-dimethylphenyl)pyrimidine-2,4-diamine. LCMS [M+H]⁺ 437; ¹H NMR (400 MHz, CDCl₃) δ_{H} ppm 1.70 - 1.82 (m, 4 H) 2.23 (s, 3 H) 2.31 (s, 3 H) 2.98 (d, J=5.31 Hz, 2 H) 3.54 (d, J=5.81 Hz, 2 H) 4.71 - 4.80 (m, 1 H) 5.29 (br. s., 2 H) 5.76 (s, 1 H) 7.07 - 7.21 (m, 3 H) 7.59 - 7.65 (m, 1 H) 7.82 (dt, J=7.64, 1.36 Hz, 1 H) 8.14 (dq, J=7.96, 0.97 Hz, 1 H) 8.18 - 8.21 (m, 1 H).

### Example 375

N-(3-{[2-Amino-6-(2,3-dimethylphenyl)pyrimidin-4-yl]amino}propyl)-4-methanesulfonylbenzene-1-sulfonamide.
Prepared according to general procedure 10 from 4-methanesulfonylbenzene-1-sulfonyl chloride and 4-N-(3-aminopropyl)-6-(2,3-dimethylphenyl)pyrimidine-2,4-diamine. LCMS [M+H]⁺ 490; ¹H NMR (400 MHz, CDCl₃) δ_{H} ppm 1.73 - 1.80 (m, 2 H) 2.23 (s, 3 H) 2.32 (s, 3 H) 2.95 - 3.01 (m, 2 H) 3.08 (s, 3 H) 3.51 - 3.58 (m, 2 H) 4.69 - 4.77 (m, 1 H) 5.31 (s, 2 H) 5.77 (s, 1 H) 7.08 - 7.21 (m, 3 H) 8.09 (q, J=8.76 Hz, 4 H).

### Example 376

N-(3-{[2-Amino-6-(2,3-dimethylphenyl)pyrimidin-4-yl]amino}propyl)benzenesulfonamide.
Prepared according to general procedure 10 from benzenesulfonyl chloride and 4-N-(3-aminopropyl)-6-(2,3-dimethylphenyl)pyrimidine-2,4-diamine. LCMS [M+H]⁺ 412; ¹H NMR (400 MHz, CDCl₃) δ ppm 1.72 (dt, J=12.06, 5.97 Hz, 2 H) 1.76 - 1.89 (m, 1 H) 2.22 (s, 3 H) 2.31 (s, 3 H) 2.93 (q, J=6.15 Hz, 2 H) 3.49 (q, J=6.40 Hz, 2 H) 4.70 - 4.82 (m, 1 H) 5.28 (br. s., 2 H) 5.72 - 5.74 (m, 1 H) 7.08 - 7.15 (m, 2 H) 7.16 - 7.20 (m, 1 H) 7.45 - 7.51 (m, 2 H) 7.52 - 7.57 (m, 1 H) 7.87 - 7.92 (m, 2 H).

### Example 377

N-(3-{[2-Amino-6-(2,3-dimethylphenyl)pyrimidin-4-yl]amino}-2,2-dimethylpropyl)-3-fluorobenzene-1-sulfonamide.
Step 1: tert-butyl N-(3-{[2-amino-6-(2,3-dimethylphenyl)pyrimidin-4-yl]amino}-2,2-dimethylpropyl)carbamate (prepared in example 239) (180 mg, 0.45 mmol) was dissolved in trifluoroacetic acid (2 ml) and stirred at reflux for 1 h. The TFA was evaporated and the crude residue was purified by silica gel chromatography using a gradient of 2-30% MeOH [containing 1 v/v% NH₄OH] in DCM which afforded 4-N-(3-amino-2,2-dimethyl-propyl)-6-(2,3-dimethylphenyl)pyrimidine-2,4-diamine (110 mg, 0.37 mmol). LCMS [M+H]⁺ 300.

Step 2: N-(3-{[2-Amino-6-(2,3-dimethylphenyl)pyrimidin-4-yl]amino}-2,2-dimethylpropyl)-3-fluorobenzene-1-sulfonamide was prepared according to general procedure 10 from 3-fluorobenzene-1-sulfonyl chloride and 4-N-(3-amino-2,2-dimethyl-propyl)-6-(2,3-dimethylphenyl)pyrimidine-2,4-diamine (prepared in step 1). LCMS [M+H]⁺ 458; ¹H NMR (400 MHz, CDCl₃) δ_{H} ppm 0.92 (s, 6 H) 2.16 - 2.20 (m, 3 H) 2.29 (s, 3 H) 2.69 (d, J=7.07 Hz, 2 H) 3.38 (d, J=6.82 Hz, 2 H) 5.91 (s, 1 H) 6.53 (s, 1 H) 7.04 - 7.08 (m, 1 H) 7.09 - 7.15 (m, 1 H) 7.23 - 7.30 (m, 1 H) 7.38 - 7.45 (m, 1 H) 7.46 - 7.56 (m, 2 H) 7.64 (d, J=7.83 Hz, 1 H).

### Example 378

N-(3-{[2-Amino-6-(2,3-dimethylphenyl)pyrimidin-4-yl]amino}-2,2-dimethylpropyl)-4-(morpholine-4-sulfonyl)benzene-1-sulfonamide.
Step 1: tert-butyl N-(3-{[2-amino-6-(2,3-dimethylphenyl)pyrimidin-4-yl]amino}-2,2-dimethylpropyl)carbamate (prepared in example 239) (180 mg, 0.45 mmol) was dissolved in trifluoroacetic acid (2 ml) and stirred at reflux for 1 h. The TFA was evaporated and the crude residue was purified by silica gel chromatography using a gradient of 2-30% MeOH [containing 1 v/v% NH₄OH] in DCM which afforded 4-N-(3-amino-2,2-dimethyl-propyl)-6-(2,3-dimethylphenyl)pyrimidine-2,4-diamine (110 mg, 0.37 mmol). LCMS [M+H]⁺ 300.

Step 2: N-(3-{[2-Amino-6-(2,3-dimethylphenyl)pyrimidin-4-yl]amino}-2,2-dimethylpropyl)-4-(morpholine-4-sulfonyl)benzene-1-sulfonamide was prepared according to general procedure 10 from 4-(morpholine-4-sulfonyl)benzene-1-sulfonyl chloride and 4-N-(3-amino-2,2-dimethyl-propyl)-6-(2,3-dimethylphenyl)pyrimidine-2,4-diamine (prepared in step 1 above). LCMS [M+H]⁺ 589; ¹H NMR (400 MHz, CDCl₃) δ_{H} ppm 0.92 (s, 6 H) 2.18 (d, J=0.51 Hz, 3 H) 2.28 (s, 3 H) 2.71 (d, J=6.82 Hz, 2 H) 2.99 - 3.06 (m, 4 H) 3.38 (d, J=6.82 Hz, 2 H) 3.70 - 3.78 (m, 4 H) 5.93 (s, 1 H) 6.94 (s, 1 H) 7.03 - 7.08 (m, 1 H) 7.09 - 7.15 (m, 1 H) 7.25 (d, J=7.58 Hz, 1 H) 7.42 (s, 1 H) 7.87 (d, J=8.59 Hz, 2 H) 8.02 (d, J=8.59 Hz, 2 H).

### Example 381

N-(3-{[2-Amino-6-(2,3-dimethylphenyl)pyrimidin-4-yl]amino}propyl)-3-fluorobenzene-1-sulfonamide.
Prepared according to general procedure 9 from 3-fluorobenzene-1-sulfonyl chloride and 4-N-(3-aminopropyl)-6-(2,3-dimethylphenyl)pyrimidine-2,4-diamine. LCMS [M+H]⁺ 430.

### Example 382

N-{4-[(3-{[2-Amino-6-(2,3-dimethylphenyl)pyrimidin-4-yl]amino}propyl)sulfamoyl]-phenyl}acetamide.
Prepared according to general procedure 10 from 4-acetamidobenzene-1-sulfonyl chloride and 4-N-(3-aminopropyl)-6-(2,3-dimethylphenyl)pyrimidine-2,4-diamine. LCMS [M+H]⁺ 469.

### Example 384

N-(2-{[2-Amino-6-(2,3-dimethylphenyl)pyrimidin-4-yl]amino}ethyl)-3-fluorobenzene-1-sulfonamide.
Prepared according to general procedure 10 from 3-fluorobenzene-1-sulfonyl chloride and 4-N-(2-aminoethyl)-6-(2,3-dimethylphenyl)pyrimidine-2,4-diamine. LCMS [M+H]⁺ 416.

### Example 385

N-(2-{[2-Amino-6-(2,3-dimethylphenyl)pyrimidin-4-yl]amino}ethyl)-4-methoxybenzene-1-sulfonamide.
Prepared according to general procedure 10 from 4-methoxybenzene-1-sulfonyl chloride and 4-N-(2-aminoethyl)-6-(2,3-dimethylphenyl)pyrimidine-2,4-diamine. LCMS [M+H]⁺ 428.

### Example 388

N-(3-{[2-Amino-6-(2,3-dimethylphenyl)pyrimidin-4-yl]amino}propyl)-4,5-dichlorothiophene-2-sulfonamide.
Prepared according to general procedure 10 from 4,5-dichlorothiophene-2-sulfonyl chloride and 4-N-(3-aminopropyl)-6-(2,3-dimethylphenyl)pyrimidine-2,4-diamine. LCMS [M+H]⁺ 486.

### Example 389

N-(3-{[2-Amino-6-(2,3-dimethylphenyl)pyrimidin-4-yl]amino}propyl)-5-chloro-1,3-dimethyl-1H-pyrazole-4-sulfonamide.
Prepared according to general procedure 10 from 5-chloro-1,3-dimethyl-1H-pyrazole-4-sulfonyl chloride and 4-N-(3-aminopropyl)-6-(2,3-dimethylphenyl)-pyrimidine-2,4-diamine. LCMS [M+H]⁺ 464.

### Example 391

N-(3-{[2-Amino-6-(2,3-dimethylphenyl)pyrimidin-4-yl]amino}propyl)-1,2-dimethyl-1H-imidazole-4-sulfonamide.
Prepared according to general procedure 10 from 1,2-dimethyl-1H-imidazole-4-sulfonyl chloride and 4-N-(3-aminopropyl)-6-(2,3-dimethylphenyl)pyrimidine-2,4-diamine. LCMS [M+H]⁺ 430; ¹H NMR (400 MHz, CD₃OD) δ_{H} ppm 7.69 (s, 2 H), 7.33 - 7.38 (m, 1 H), 7.21 - 7.27 (m, 1 H), 7.16 - 7.20 (m, 1 H), 6.00 (s, 1 H), 3.71 (s, 3 H), 3.58 (s, 2 H), 3.06 (s, 2 H), 2.45 (s, 3 H), 2.36 (s, 3 H), 2.25 (s, 3 H), 1.80 - 1.90 (m, 2 H).

### Example 392

N-(2-{[2-Amino-6-(2,3-dimethylphenyl)pyrimidin-4-yl]amino}ethyl)-5-chloro-1,3-dimethyl-1H-pyrazole-4-sulfonamide.
Prepared according to general procedure 10 from 5-chloro-1,3-dimethyl-1H-pyrazole-4-sulfonyl chloride and 4-N-(2-aminoethyl)-6-(2,3-dimethylphenyl)-pyrimidine-2,4-diamine. LCMS [M+H]⁺ 450.

### Example 393

4-N-{2-[(1,3-Benzoxazol-2-yl)amino]ethyl}-6-(2,3-dimethylphenyl)pyrimidine-2,4-diamine.
Prepared according to general procedure 12 from 2-chloro-1,3-benzoxazole and 4-N-(2-aminoethyl)-6-(2,3-dimethylphenyl)pyrimidine-2,4-diamine. LCMS [M+H]⁺ 375.

### Example 394

6-(2,3-Dimethylphenyl)-4-N-(4-phenylbutan-2-yl)pyrimidine-2,4-diamine.
Prepared according to general procedure 9 from 4-phenylbutan-2-amine and 4-chloro-6-(2,3-dimethylphenyl)pyrimidin-2-amine. LCMS [M+H]⁺ 347.

### Example 397

6-[(2-{[2-Amino-6-(2,3-dimethylphenyl)pyrimidin-4-yl]amino}ethyl)amino]pyridine-3-carbonitrile.
Prepared according to general procedure 12 from 6-chloropyridine-3-carbonitrile and 4-N-(2-aminoethyl)-6-(2,3-dimethylphenyl)pyrimidine-2,4-diamine. LCMS [M+H]⁺ 360; ¹H NMR (400 MHz, CD₃OD) δ_{H} ppm 8.32 - 8.34 (m, 1 H), 7.59 - 7.64 (m, 1 H), 7.34 - 7.38 (m, 1 H), 7.22 - 7.27 (m, 2 H), 7.15 - 7.18 (m, 1 H), 6.55 - 6.59 (m, 1 H), 5.97 (s, 1 H), 3.72 - 3.77 (m, 3 H), 3.66 - 3.71 (m, 3 H), 2.36 (s, 4 H), 2.24 (s, 3 H).

### Example 398

4-N-{2-[(3-Bromo-1,2,4-thiadiazol-5-yl)amino]ethyl}-6-(2,3-dimethylphenyl)pyrimidine-2,4-diamine.
Prepared according to general procedure 12 from 3-bromo-5-chloro-1,2,4-thiadiazole and 4-N-(2-aminoethyl)-6-(2,3-dimethylphenyl)pyrimidine-2,4-diamine. LCMS [M+H]⁺ 420.

### Example 401

N-(2-{[2-Amino-6-(2,3-dimethylphenyl)pyrimidin-4-yl]amino}ethyl)-4-methanesulfonylbenzene-1-sulfonamide.
Prepared according to general procedure 10 from 4-methanesulfonylbenzene-1-sulfonyl chloride and 4-N-(2-aminoethyl)-6-(2,3-dimethylphenyl)pyrimidine-2,4-diamine. LCMS [M+H]⁺ 476; ¹H NMR (400 MHz, CD₃OD) δ_{H} ppm 8.10 - 8.14 (m, 3 H), 8.05 - 8.09 (m, 3 H), 7.16 - 7.20 (m, 1 H), 7.09 - 7.14 (m, 1 H), 7.05 - 7.08 (m, 1H), 5.73 - 5.76 (m, 1 H), 3.42 - 3.49 (m, 2 H), 3.14 (s, 6 H), 2.31 (s, 4 H), 2.18 (s, 3 H).

### Example 402

N-(3-{[2-Amino-6-(2,3-dimethylphenyl)pyrimidin-4-yl]amino}propyl)-3-methanesulfonylbenzene-1-sulfonamide.
Prepared according to general procedure 10 from 3-methanesulfonylbenzene-1-sulfonyl chloride and 4-N-(3-aminopropyl)-6-(2,3-dimethylphenyl)pyrimidine-2,4-diamine. LCMS [M+H]⁺ 490; ¹H NMR (400 MHz, CDCl₃) δ_{H} ppm 8.43 - 8.45 (m, 1 H), 8.18 (ddd, J=7.8, 1.8, 1.0 Hz, 3 H), 8.11 (ddd, J=7.8, 1.8, 1.0 Hz, 3 H), 7.67 - 7.73 (m, 1 H), 7.15 - 7.19 (m, 1 H), 7.09 - 7.13 (m, 1 H), 7.08 (d, J=2.0 Hz, 1 H), 5.74 (s, 1 H), 5.31 (br. s., 2 H), 4.82 - 5.00 (m, 1 H), 3.50 (d, J=5.8 Hz, 2 H), 3.09 (s, 3 H), 2.94 (d, J=4.0 Hz, 2 H), 2.29 (s, 3 H), 2.20 (s, 3 H), 1.71 (t, J=5.8 Hz, 2 H).

### Example 403

N-(3-{[2-Amino-6-(2,3-dimethylphenyl)pyrimidin-4-yl]amino}propyl)-2-methanesulfonylbenzene-1-sulfonamide.
Prepared according to general procedure 10 from 2-methanesulfonylbenzene-1-sulfonyl chloride and 4-N-(3-aminopropyl)-6-(2,3-dimethylphenyl)pyrimidine-2,4-diamine. LCMS [M+H]⁺ 490; ¹H NMR (400 MHz, CD₃OD) δ_{H} ppm 8.20 - 8.33 (m, 3 H), 8.10 - 8.17 (m, 1 H), 7.84 - 7.90 (m, 5 H), 7.68 - 7.80 (m, 3 H), 7.16 - 7.21 (m, 3H), 7.12 (s, 3 H), 7.08 (d, J=1.5 Hz, 3 H), 5.74 (s, 3 H), 3.46 (s, 3 H), 3.43 (s, 8 H), 3.32 - 3.36 (m, 3 H), 3.07 (t, J=6.7 Hz, 5 H), 2.31 (s, 8 H), 2.19 (s, 3 H), 1.66 (t, J=6.7 Hz, 5 H).

### Example 414

4-(2-{[2-amino-6-(3-chloro-2-methylphenyl)pyrimidin-4-yl]amino}ethyl)-N,N-dimethylbenzene-1-sulfonamide.
A mixture of 4-chloro-6-(3-chloro-2-methyl-phenyl)pyrimidin-2-amine (0.14 mmol), 4-(2-aminoethyl)-N,N-dimethyl-benzenesulfonamide (1.0 eq.) and N,N-diisopropylethylamine (2.25 eq.) in n-butanol (0.3 mL) was heated in a sealed tube at 110°C overnight. Methanol was added and the mixture was filtered and purified by preparative HPLC. LCMS [M+H]⁺ 446.

### Example 415

4-(2-{[2-amino-6-(2,3-dichlorophenyl)pyrimidin-4-yl]amino}ethyl)-N,N-dimethylbenzene-1-sulfonamide.
A mixture of 4-chloro-6-(2,3-dichlorophenyl)pyrimidin-2-amine (0.13 mmol), 4-(2-aminoethyl)-N,N-dimethyl-benzenesulfonamide (1.0 eq.) and N,N-diisopropylethylamine (2.25 eq.) in n-butanol (0.3 mL) was heated in a sealed tube at 110°C overnight. Methanol was added and the mixture was filtered and purified by preparative HPLC. LCMS [M+H]⁺ 466.

### Example 416

4-(2-{[2-amino-6-(2,3-dimethylphenyl)pyrimidin-4-yl]amino}ethyl)-N,N-dimethylbenzene-1-sulfonamide.
A mixture of 4-chloro-6-(2,3-dimethylphenyl)pyrimidin-2-amine (0.15 mmol), 4-(2-aminoethyl)-N,N-dimethyl-benzenesulfonamide (1.0 eq.) and N,N-diisopropylethylamine (2.25 eq.) in n-butanol (0.3 mL) was heated in a sealed tube at 110°C overnight. Methanol was added and the mixture was filtered and purified by preparative HPLC. LCMS [M+H]⁺ 426.

### Example 417

4-N-{1-[(4-Chlorophenyl)methyl]cyclopropyl}-6-(2,3-dimethylphenyl)pyrimidine-2,4-diamine.
A mixture of 4-chloro-6-(2,3-dimethylphenyl)pyrimidin-2-amine (47 mg, 0.20 mmol), 1-[(4-chlorophenyl)methyl]cyclopropan-1-amine hydrochloride (52 mg, 0.24 mmol) and triethylamine (50 µL, 0.36 mmol) in n-butanol (3 mL) was heated in a sealed tube at 130°C overnight. The reaction mixture was concentrated and purified by preparative HPLC. LCMS [M+H]⁺ 379.

### Example 423

4-N-[2-(4-Chlorophenyl)ethyl]-6-(1H-pyrrol-2-yl)pyrimidine-2,4-diamine.
Prepared according to general procedure 10 from 6-chloro-4-N-[2-(4-chlorophenyl)ethyl]pyrimidine-2,4-diamine and (1-tert-butoxycarbonylpyrrol-2-yl)boronic acid. The t-butoxycarbonyl group was removed during work-up. LCMS [M+H]⁺ 314; ¹H NMR (400 MHz, CD₃OD) δ_{H} ppm 7.29 - 7.33 (m, 5 H), 7.27 (s, 2 H), 7.10 - 7.13 (m, 1 H), 6.88 - 6.92 (m, 1 H), 6.33 - 6.37 (m, 1 H), 6.12 (s, 1 H), 3.70 - 3.76 (m, 2 H), 2.91 - 2.96 (m, 2 H).

### Example 424

N-(4-{[2-amino-6-(2,3-dimethylphenyl)pyrimidin-4-yl]amino}butyl)-4-methanesulfonylbenzene-1-sulfonamide.
Prepared according to general procedure 10 from 4-(methylsulfonyl)-benzenesulfonyl chloride and 4-N-(4-aminobutyl)-6-(2,3-dimethylphenyl)-pyrimidine-2,4-diamine. LCMS [M+H]⁺ 504; ¹H NMR (400 MHz, CD₃OD) δ_{H} ppm 8.12 - 8.17 (m, 2 H), 8.06 - 8.10 (m, 2 H), 7.16 - 7.20 (m, 1 H), 7.09 - 7.14 (m, 1 H), 7.05 - 7.09 (m, 1 H), 5.76 - 5.79 (m, 1 H), 3.18 (s, 3 H), 2.94 - 2.99 (m, 2 H), 2.31 (s, 3 H), 2.19 (s, 3 H), 1.50 - 1.66 (m, 4 H).

### Example 426

4-(2-{[2-amino-6-(3-cyano-2-methylphenyl)pyrimidin-4-yl]amino}ethyl)benzene-1-sulfonamide.
Prepared according to general procedure 2 from 4-[2-[(2-amino-6-chloro-pyrimidin-4-yl)amino]ethyl]benzenesulfonamide and 3-cyano-2-methyl-benzeneboronic acid. LCMS [M+H]⁺ 409; ¹H NMR (400 MHz, CD₃OD) δ_{H} ppm 7.80 - 7.86 (m, 2 H), 7.70 (dd, J=7.8, 1.3 Hz, 1 H), 7.53 (d, J=6.8 Hz, 1 H), 7.37 - 7.47 (m, 3 H), 5.80 (s, 1 H), 3.65 (br. s., 2 H), 3.00 (t, J=7.2 Hz, 2 H), 2.49 (s, 3 H).

### Example 441

4-N-{2-[(6-chloropyridazin-3-yl)amino]ethyl}-6-(2,3-dimethylphenyl)pyrimidine-2,4-diamine.
Prepared according to general procedure 12 from 4-N-(2-aminoethyl)-6-(2,3-dimethylphenyl)pyrimidine-2,4-diamine and 3,6-dichloropyridazine. [M+H]+ 370.

### Example 442

2-[(2-{[2-amino-6-(2,3-dimethylphenyl)pyrimidin-4-yl]amino}ethyl)amino]pyridine-4-carbonitrile.
Prepared according to general procedure 12 from 4-N-(2-aminoethyl)-6-(2,3-dimethylphenyl)pyrimidine-2,4-diamine and 2-chloropyridine-4-carbonitrile. LCMS [M+H]⁺ 360.

### Example 443

6-[(2-{[2-amino-6-(2,3-dimethylphenyl)pyrimidin-4-yl]amino}ethyl)amino]pyridine-3-sulfonamide.
Prepared according to general procedure 12 from 4-N-(2-aminoethyl)-6-(2,3-dimethylphenyl)pyrimidine-2,4-diamine and 6-chloropyridine-3-sulfonamide. LCMS [M+H]⁺ 414.

### Example 444

1-N-(3-{[2-amino-6-(2,3-dimethylphenyl)pyrimidin-4-yl]amino}propyl)benzene-1,4-disulfonamide.
Prepared according to general procedure 10 from 4-N-(4-aminopropyl)-6-(2,3-dimethylphenyl)pyrimidine-2,4-diamine and 4-(aminosulfonyl)benzenesulfonyl chloride. LCMS [M+H]⁺ 491; ¹H NMR (400 MHz, CD₃OD) δ_{H} ppm 8.04 - 8.08 (m, 2 H), 7.98 - 8.02 (m, 2 H), 7.28 (d, J=7.1 Hz, 1 H), 7.16 - 7.22 (m, 1 H), 7.15 (d, J=1.5 Hz, 1 H), 5.91 (s, 1 H), 3.48 (d, J=1.5 Hz, 2 H), 2.98 (t, J=6.8 Hz, 2 H), 2.34 (s, 3 H), 2.22 (s, 3 H), 1.78 (t, J=6.8 Hz, 2 H).

### Example 445

6-(2,3-dichlorophenyl)-4-N-[2-(4-methanesulfonylphenyl)ethyl]pyrimidine-2,4-diamine.
Prepared according to general procedure 2 from 6-chloro-4-N-[2-(4-methylsulfonylphenyl)ethyl]pyrimidine-2,4-diamine and 2,3-dichlorobenzene-boronic acid. LCMS [M+H]⁺ 437; ¹H NMR (400 MHz, CD₃OD) δ_{H} ppm 7.90 (d, J=8.6 Hz, 2 H), 7.78 (t, J=4.9 Hz, 1 H), 7.56 (d, J=8.6 Hz, 2 H), 7.49 (dd, J=4.9, 0.6 Hz, 2 H), 6.08 (s, 1 H), 3.84 (s, 2 H), 3.06 - 3.11 (m, 5 H).

### Example 447

6-(2,3-dimethylphenyl)-4-N-{2-[(3-methoxyphenyl)amino]ethyl}pyrimidine-2,4-diamine.
Prepared according to general procedure 9 from 4-chloro-6-(2,3-dimethylphenyl)pyrimidin-2-amine and N'-(3-methoxyphenyl)ethane-1,2-diamine. LCMS [M+H]⁺ 364; ¹H NMR (400 MHz, CDCl₃) δ_{H} ppm 7.18 - 7.26 (m, 1 H), 7.01 - 7.07 (m, 1 H), 6.96 - 7.00 (m, 1 H), 6.95 (t, J=2.0 Hz, 1 H), 6.84 (d, J=7.8 Hz, 1 H), 6.76 (dd, J=8.3, 2.0 Hz, 1 H), 5.91 (s, 1 H), 3.82 (d, J=4.8 Hz, 2 H), 3.75 (s, 3 H), 3.48 (br. s., 2 H), 2.24 (s, 3 H), 2.10 (s, 3 H).

### Example 448

6-(2,3-dimethylphenyl)-4-N-{2-[(3-fluoro-4-methylphenyl)amino]ethyl}pyrimidine-2,4-diamine.
Step 1: A vial was charged with 2-fluoro-4-iodo-1-methyl-benzene (240 mg, 1.0 mmol), ethane-1,2-diamine (0.20 mL, 3.0 mmol), CuCI (9.9 mg, 0.10 mmol), and KOH (110 mg, 2.0 mmol). The vial was then flushed with nitrogen and sealed. The mixture was stirred at r.t. for 16 h, thereafter the mixture was extracted with EtOAc. The combined organic phases were dried, concentrated and purified by column chromatography to afford N'-(3-fluoro-4-methyl-phenyl)ethane-1,2-diamine.

Step 2: A mixture of 6-(2,3-dimethylphenyl)-4-chloropyrimidin-2-amine (30 mg, 0.13 mmol), N'-(3-fluoro-4-methyl-phenyl)ethane-1,2-diamine (20 mg, 0.12 mmol), and diisopropylethylamine (0.040 mL, 0.23 mmol) in 2-propanol (0.50 mL) was heated in a sealed tube at 150 °C for 30 min in a microwave reactor. The reaction mixture was then concentrated and purified by preparative HPLC. LCMS [M+H]⁺ 366; ¹H NMR (400 MHz, CD₃OD) δ_{H} ppm 7.33 - 7.37 (m, 1 H), 7.21 - 7.27 (m, 1 H), 7.15 - 7.18 (m, 1 H), 6.93 - 6.99 (m, 1 H), 6.38 - 6.45 (m, 2 H), 5.99 (s, 1 H), 3.72 (s, 2 H), 3.39 (s, 2 H), 2.36 (s, 3 H), 2.24 (s, 3 H), 2.11 (d, J=1.5 Hz, 3 H).

### Example 449

4-N-{2-[(3,4-dichlorophenyl)amino]ethyl}-6-(2,3-dimethylphenyl)pyrimidine-2,4-diamine.
Prepared according to general procedure 9 from 4-chloro-6-(2,3-dimethylphenyl)pyrimidin-2-amine and N'-(3,4-dichlorophenyl)ethane-1,2-diamine. LCMS [M+H]⁺ 402; ¹H NMR (400 MHz, CD₃OD) δ_{H} ppm 7.33 - 7.37 (m, 1 H), 7.24 (s, 1 H), 7.18 (m, 2 H), 6.76 (d, J=2.8 Hz, 1 H), 6.57 (dd, J=8.8, 2.8 Hz, 1 H), 5.97 (s, 3 H), 3.69 - 3.73 (m, 2 H), 3.37 - 3.42 (m, 2 H), 2.35 (s, 3 H), 2.23 (s, 3 H).

### Example 450

4-N-{2-[(5-chloropyridin-2-yl)amino]ethyl}-6-(2,3-dimethylphenyl)pyrimidine-2,4-diamine.
Prepared according to general procedure 9 from 4-chloro-6-(2,3-dimethylphenyl)pyrimidin-2-amine and N'-(5-chloro-2-pyridyl)ethane-1,2-diamine. LCMS [M+H]⁺ 369; ¹H NMR (400 MHz, CDCl₃) δ_{H} ppm 7.61 - 7.69 (m, 2 H), 7.30 (d, J=7.6 Hz, 1 H), 7.10 - 7.22 (m, 3 H), 6.17 (br. s., 1 H), 3.71 (br. s., 4 H), 2.33 (s, 3 H), 2.24 (s, 3 H).

### Example 451

4-N-{2-[(5-bromopyridin-2-yl)amino]ethyl}-6-(2,3-dimethylphenyl)pyrimidine-2,4-diamine.
Prepared according to general procedure 9 from 4-chloro-6-(2,3-dimethylphenyl)pyrimidin-2-amine and N'-(5-bromo-2-pyridyl)ethane-1,2-diamine. LCMS [M+H]⁺ 413; ¹H NMR (400 MHz, CD₃OD) δ_{H} ppm 8.07 (dd, J=2.3, 0.8 Hz, 1 H), 7.86 (dd, J=9.3, 2.3 Hz, 2 H), 7.33 - 7.37 (m, 2 H), 7.24 (t, J=7.6 Hz, 1 H), 7.15 - 7.19 (m, 1 H), 6.90 (dd, J=9.5, 0.6 Hz, 1 H), 6.03 (s, 1 H), 3.77 - 3.82 (m, 2 H), 3.64 - 3.69 (m, 2 H), 2.35 (s, 3 H), 2.24 (s, 3 H).

### Example 452

4-[(2-{[2-amino-6-(2,3-dimethylphenyl)pyrimidin-4-yl]amino}ethyl)amino]benzene-1-sulfonamide.
Prepared according to general procedure 9 from 4-chloro-6-(2,3-dimethylphenyl)pyrimidin-2-amine and 4-(2-aminoethylamino)benzenesulfonamide. LCMS [M+H]⁺ 413; ¹H NMR (400 MHz, CD₃OD) δ_{H} ppm 7.64 (d, J=9.1 Hz, 2 H), 7.33 - 7.37 (m, 1 H), 7.24 (s, 1 H), 7.15 - 7.19 (m, 1 H), 6.70 (d, J=9.1 Hz, 2 H), 6.00 (s, 1 H), 3.72 - 3.77 (m, 2 H), 3.45 - 3.50 (m, 2 H), 2.35 (s, 3 H), 2.24 (s, 3 H).

### Example 454

6-(2,3-dimethylphenyl)-4-N-{2-[(pyridin-3-yl)amino]ethyl}pyrimidine-2,4-diamine.
Prepared according to general procedure 9 from 4-chloro-6-(2,3-dimethylphenyl)pyrimidin-2-amine and N'-(3-pyridyl)ethane-1,2-diamine. LCMS [M+H]⁺ 335; ¹H NMR (400 MHz, CD₃OD) δ_{H} ppm 8.10 - 8.12 (m, 1 H), 7.97 - 8.00 (m, 1 H), 7.72 - 7.80 (m, 2 H), 7.34 - 7.38 (m, 1 H), 7.22 - 7.27 (m, 1 H), 7.19 (dd, J=1.0, 0.5 Hz, 1 H), 6.04 (s, 1 H), 3.75 - 3.80 (m, 2 H), 3.54 (t, J=6.2 Hz, 2 H), 2.36 (s, 3 H), 2.25 (s, 3 H).

| | |
|---|---|
| 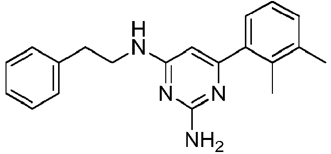 | 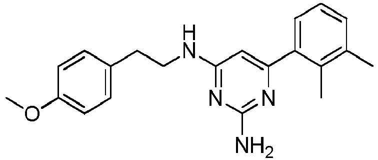 |
| Example 118 | Example 125 |
| 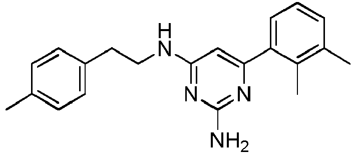 | 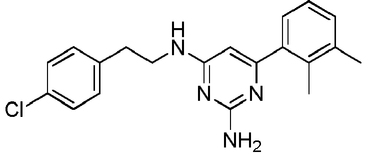 |
| Example 127 | Example 128 |
| 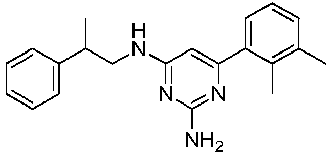 | 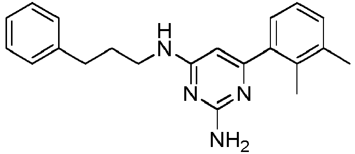 |
| Example 130 | Example 131 |
| 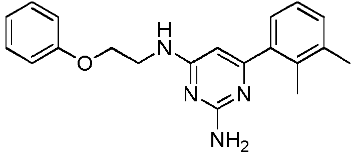 | 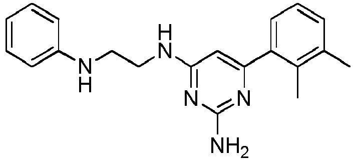 |
| Example 132 | Example 133 |
| 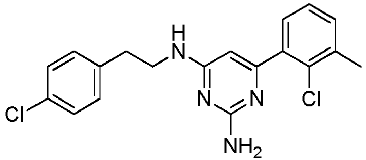 | 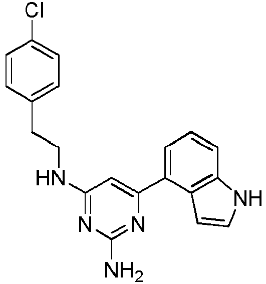 |
| Example 228 | Example 231 |
| 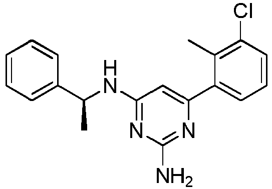 | 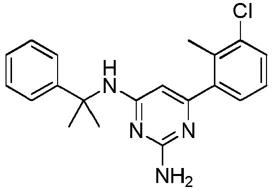 |
| Example 241 | Example 242 |
| 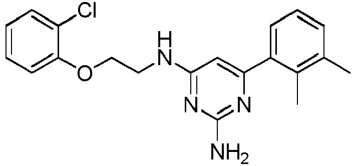 | 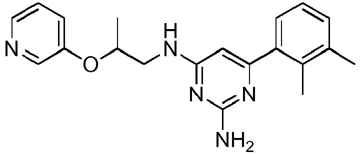 |
| Example 247 | Example 250 |
| 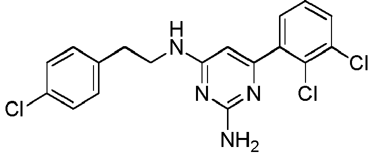 | 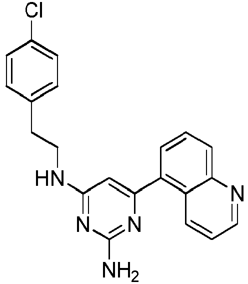 |
| Example 263 | Example 266 |
| 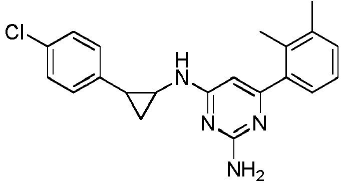 | 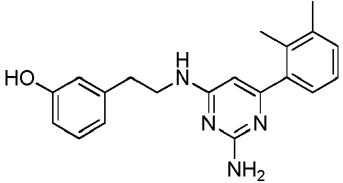 |
| Example 267 | Example 269 |
| 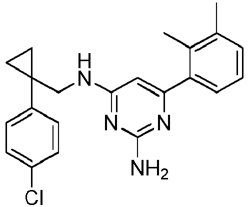 | 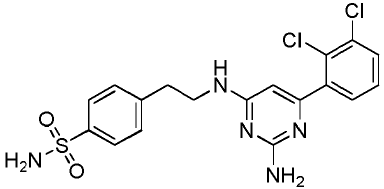 |
| Example 295 | Example 296 |
| 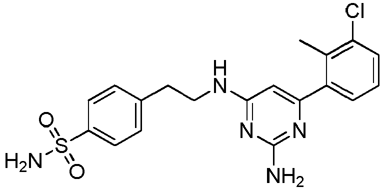 | 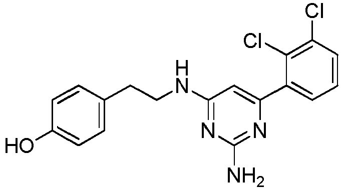 |
| Example 297 | Example 301 |
| 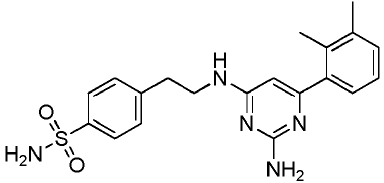 | 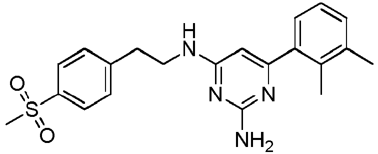 |
| Example 330 | Example 331 |
| 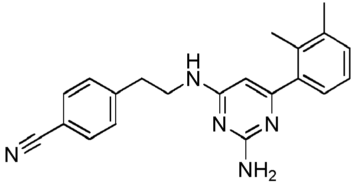 | 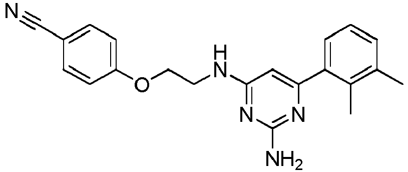 |
| Example 333 | Example 335 |
| 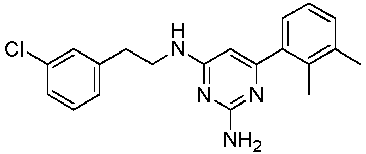 | 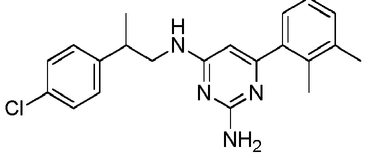 |
| Example 341 | Example 343 |
| 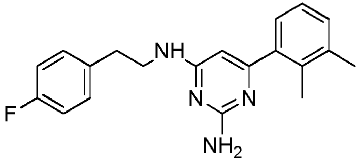 | 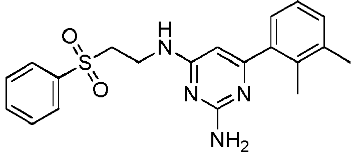 |
| Example 344 | Example 347 |
| 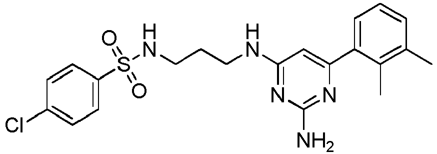 | 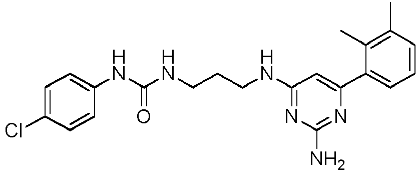 |
| Example 357 | Example 358 |
| 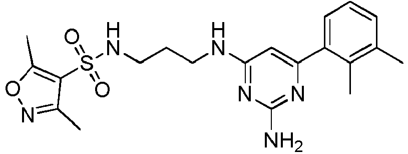 | 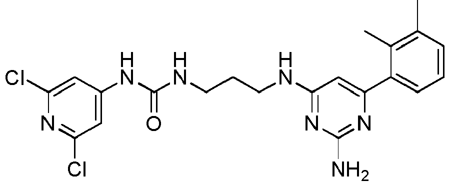 |
| Example 359 | Example 361 |
| 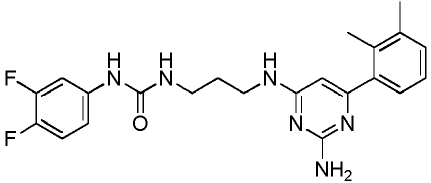 | 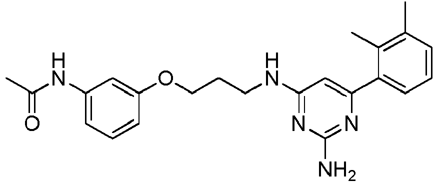 |
| Example 362 | Example 363 |
| 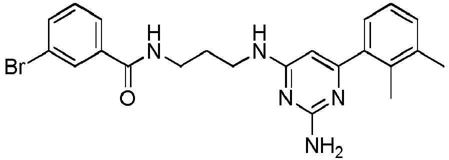 | 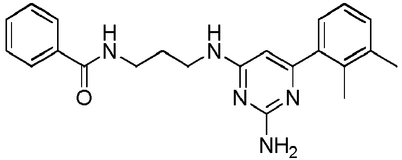 |
| Example 367 | Example 368 |
| 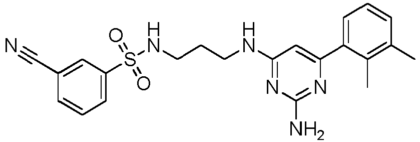 | 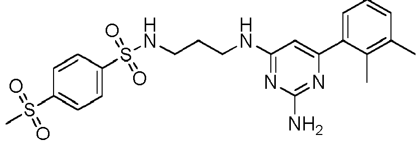 |
| Example 374 | Example 375 |
| 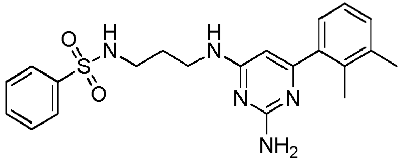 | 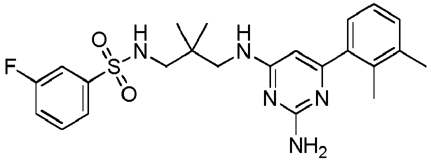 |
| Example 376 | Example 377 |
| 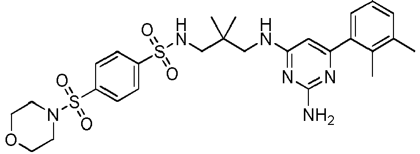 | 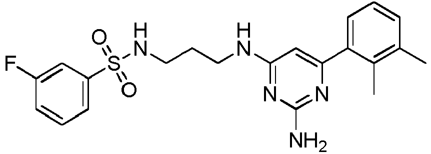 |
| Example 378 | Example 381 |
| 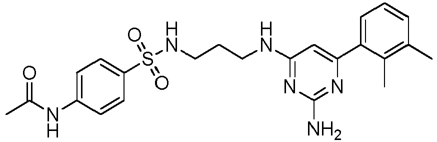 | 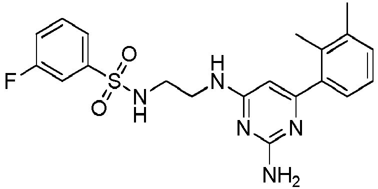 |
| Example 382 | Example 384 |
| 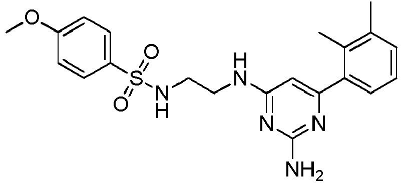 | 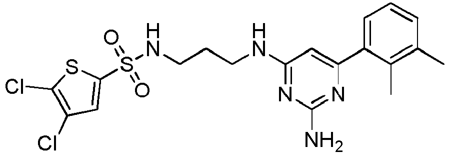 |
| Example 385 | Example 388 |
| 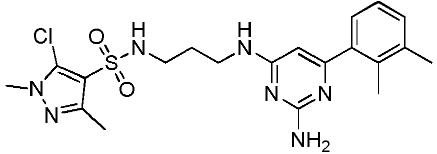 | 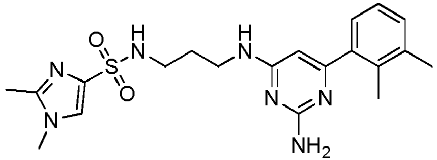 |
| Example 389 | Example 391 |
| 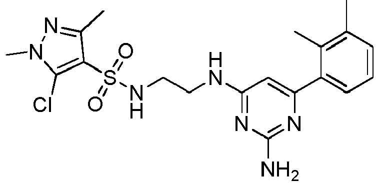 | 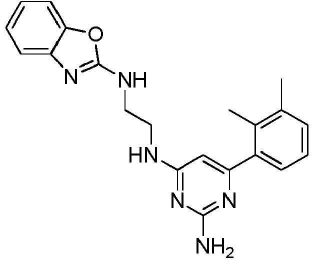 |
| Example 392 | Example 393 |
| 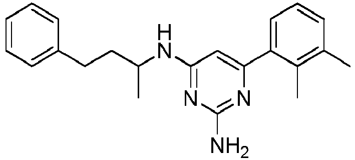 | 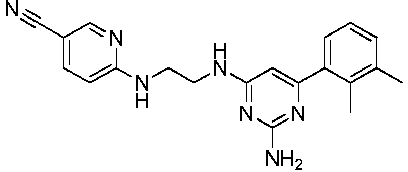 |
| Example 394 | Example 397 |
| 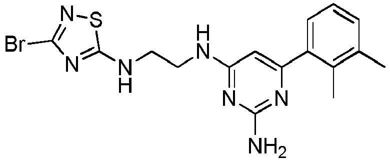 | 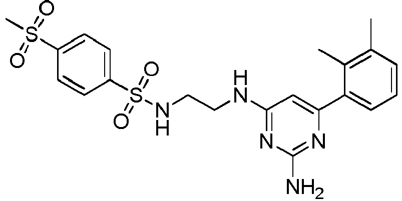 |
| Example 398 | Example 401 |
| 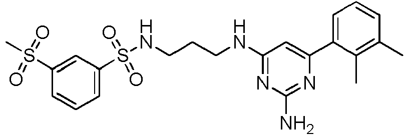 | 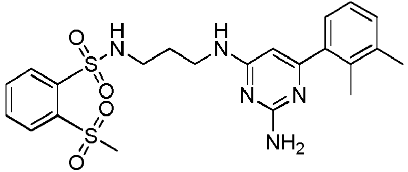 |
| Example 402 | Example 403 |
| 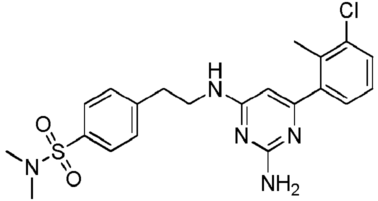 | 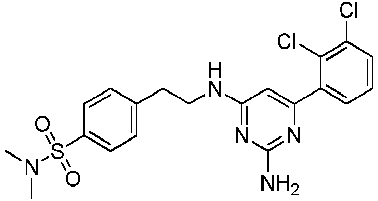 |
| Example 414 | Example 415 |
| 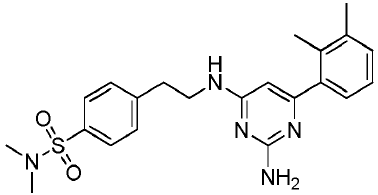 | 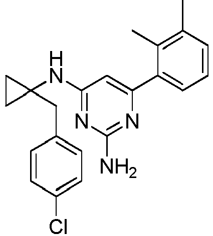 |
| Example 416 | Example 417 |
| 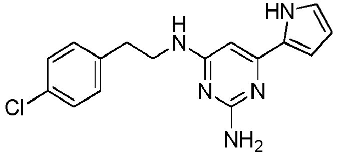 | 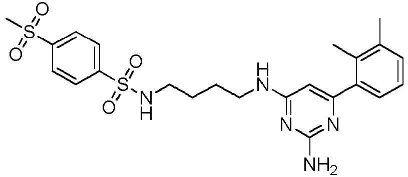 |
| Example 423 | Example 424 |
| 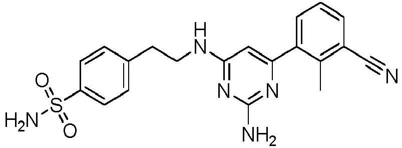 | 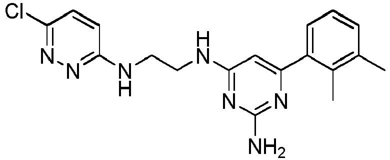 |
| Example 426 | Example 441 |
| 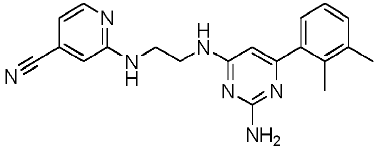 | 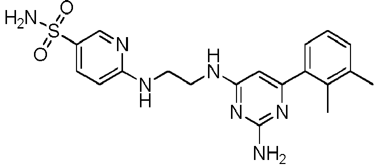 |
| Example 442 | Example 443 |
| 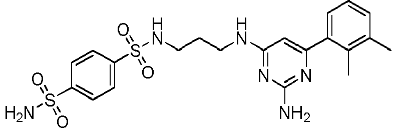 | 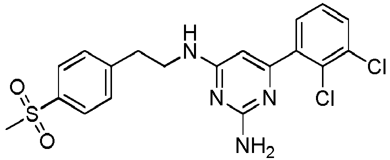 |
| Example 444 | Example 445 |
| 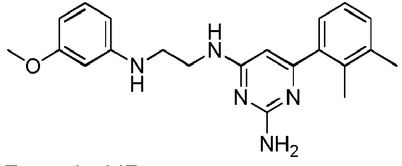 | 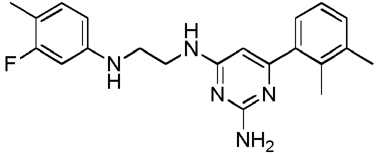 |
| Example 447 | Example 448 |
| 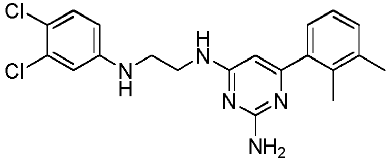 | 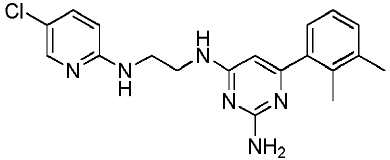 |
| Example 449 | Example 450 |
| 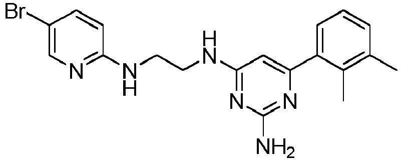 | 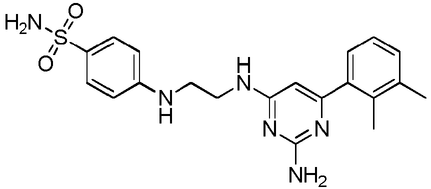 |
| Example 451 | Example 452 |
| 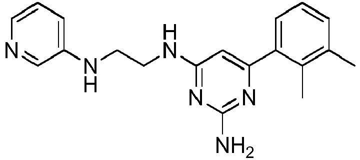 | |
| Example 454 | |

### Biological Examples

### Biological Example 1

### MTH1 enzymatic assay and IC50 value determination

MTH1 catalyzes the hydrolysis of dGTP to dGMP and PPi. By coupling the reaction to pyrophosphatase added in excess PPi is converted to Pi that can be detected by using the malachite green assay reagent. Briefly, for IC₅₀ value determination the compound to be analyzed is diluted in assay buffer in a 1:3 dilution series generating 12 different compound concentrations giving a final DMSO concentration of 1% in the assay well. MTH1 diluted in assay buffer (100 mM Tris-acetate, 40 mM NaCl, 10 mM magnesium acetate, 1 mM DTT and 0.005% Tween 20) fortified with *E.coli* pyrophosphatase (0.8 U/ml) is added to a final concentration of 4.8 nM. dGTP diluted in assay buffer is added to a final concentration of 100 µM. The reaction mixture is incubated with shaking for 15 minutes at 22°C. To 100 µl reaction mixture is 25 µl Malachite green assay regent (0.095% Malachite green in 17% H₂SO₄, 1.5% Ammonium molybdate, 0.17% Tween 20) added followed by incubation with shaking for 15 minutes at 22°C. The absorbance of the assay plate is read at 630 nm using an EnVision Multilabel plate reader. The IC₅₀ value is determined by fitting a dose response curve to the data points using nonlinear regression analysis and the equation Y=Bottom + (Top-Bottom)/(1+10^((LₒgIC₅₀-X)^{∗}HillSlope)), where Y is the read absorbance at 630 nm and X is log[compound].

Using this approach the following representative IC₅₀ values were derived. Examples - 127, 131, 132, 228, 231, 266, 267, 269, 295, 296, 301, 330, 331, 333, 335, 343, 359, 361, 363, 367, 374, 376, 381, 389, 391, 397, 402, 403, 415, 416, 424, 426, 443, 444, 445, 447, 451, 454 had IC₅₀'s of less than 200 nM

Examples - 241, 242, 347, 378, 384, 423, had IC₅₀'s of between 200 nM and 2 µM

### Biological example 2: MTH1 siRNA knock down and cell survival

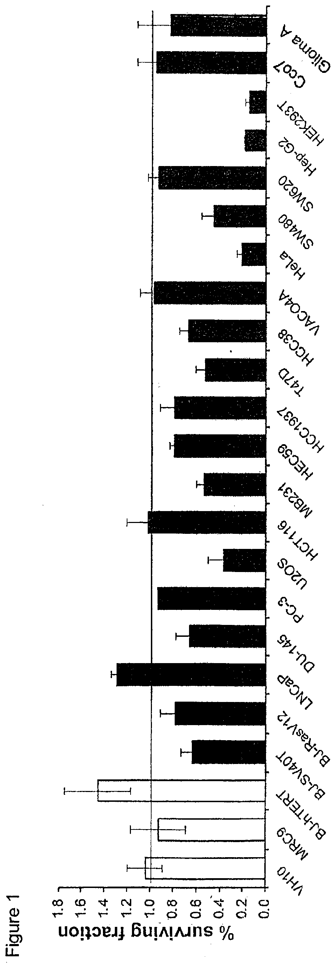

Cells were cultivated in suitable medium supplemented with 10% FBS and 10 U/ml PeSt. On day 1, cells were seeded at approximately 30% confluency in 6-well plates in complete medium. The day after, the medium was aspired and fresh medium without antibiotics was added. The cells were transfected with All-stars Non-targeting RNA (NT RNA, Qiagen) and MTH1 siRNA (5'-CGACGACAGCUACUGGUUU-3') and Interferin (Polyplus) according to the manufacturer's protocol. On day 5, cells were either trypsinized, counted and re-seeded for clonogenic outgrowth on 10-cm plates at 500 cells/plate or washed with PBS and scraped in lysis buffer (10 mM Hepes pH 7.1, 50 mM NaCl, 0.3 M sucrose, 0.1 mM EDTA, 0.5% Triton X100, 1 mM DTT and 1x protease inhib, cocktail (Pierce)) and processed for Western blot. Blots were stained with anti-MTH1 antibody (rabbit polyclonal, HPA, KTH, Sweden) and anti-tubulin (mouse monoclonal, Sigma Aldrich). After additional 7-10 days, survival plates were fixed/stained with 4% methylene blue in MeOH and colonies were counted manually. Figure 1 shows the effect on cell survival following MTH1 siRNA depletion in various human cancer and normal cell lines.

### Biological example 3: Cell survival assay

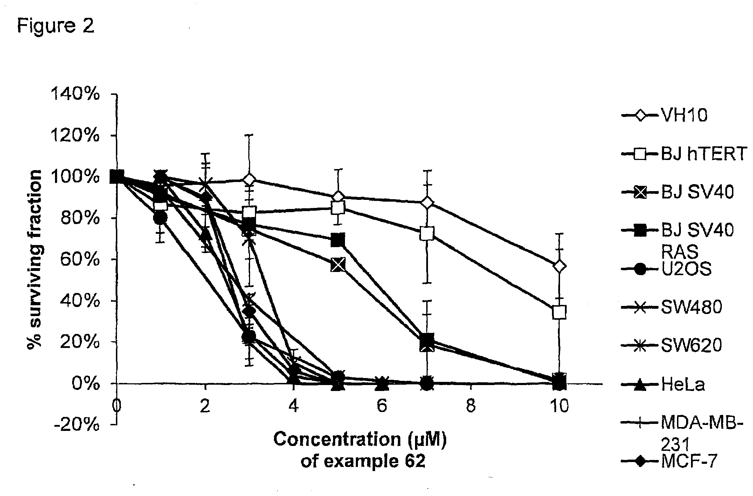

Cells are cultured in DMEM GlutaMAX™ or other suitable cell medium and plated in cell culture dishes (100 mm x 20 mm) (500 cells/dish) and incubated in 5% CO₂ at 37 °C. After 5 hours of incubation cells are treated with compound at various concentrations and left in the incubator for 7-14 days dependent on cell line. Clonogenic survival is measured by fixating and staining the cells with methylene blue (4 g/L in methanol) and number of colonies are counted and compared to vehicle treated dishes. Figure 2 shows how an MTH1 inhibitor (not according to the invention) reduces cell survival in various cancer cell lines, with less effect on normal immortalised cells (VH10 and BJ hTERT).

### Biological example 4: Cell viability assay

Cells are seeded into 96 well plates (1500 cells/well) in DMEM GlutaMAX™ or other suitable media dependent on cell line and incubated overnight in 5% CO₂, 37 °C. Thereafter cells are treated with compound or vehicle and left in incubator for 3 days until staining with resazurin. Cells are incubated in resazurin (diluted in suitable media) at 37 °C for 2 hours before measuring the fluorescence.

## Claims

1. A compound of formula I, 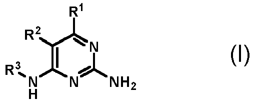 or a pharmaceutically acceptable salt thereof, wherein:
R¹ represents heteroaryl selected from benzofuran-3-yl, benzothiophen-3-yl, dihydrobenzofuran-7-yl, indol-3-yl, indol-4-yl, indol-5-yl, isoquinolin-4-yl, pyridin-3-yl, pyridin-4-yl, pyrrol-2-yl and quinolin-5-yl, which heteroaryl is connected to the pyrimidine of formula I via a carbon atom of the heteroaryl ring, and which heteroaryl is optionally substituted by one or more substituents selected from Y¹, or -C₁₋₆alkyl optionally substituted by one or more Y²; or aryl represented by 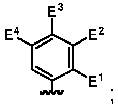
E³ and E⁴ represent hydrogen; and
E¹ represents -F and E² represent -F, -CI, -CH₃ or -CF₃; or
E¹ represents -Cl and E² represents -F, -CI, -CH₃ or -CF₃; or
E¹ represents -CH₃ and E² represents -F, -CI, -CH₃, -CF₃, or -CN;
R² represents hydrogen;
R³ represents -C₂₋₆alkyl substituted by Z¹;
each Y¹ independently represents halogen, -CN, -C(O)R^{a}, -C(O)N(R^{b})R^{c}, -C(O)OR^{d}, -N(R^{e})R^{f}, -N(R^{g})C(O)R^{h}, -N(Rⁱ)C(O)OR^{j}, -N(R^{k})C(O)N(R^{l})R^{m}, -NO₂, -N(Rⁿ)S(O)₂R^{o}, -OR^{p}, -OC(O)R^{q}, -OS(O)₂R^{r}, -S(O)ₘR^{s}, -S(O)₂N(R^{t})R^{u}, 3- to 12-membered heterocycloalkyl optionally substituted by one or more substituents selected from W², 6- to 14-membered aryl optionally substituted by one or more substituents selected from W³, or 5- to 10-membered heteroaryl containing one or more heteroatoms selected from oxygen, nitrogen and/or sulfur optionally substituted by one or more substituents selected from W³;
each R^{a}, R^{b}, R^{c}, R^{d}, R^{e}, R^{g}, R^{h}, Rⁱ, R^{k}, R^{l}, R^{m}, Rⁿ, R^{p}, R^{q}, R^{s}, R^{t} and R^{u} independently represents hydrogen, -C₁₋₆ alkyl optionally substituted by one or more substituents selected from W¹, 3- to 12-membered heterocycloalkyl optionally substituted by one or more substituents selected from W², 6- to 14-membered aryl optionally substituted by one or more substituents selected from W³, or 5- to 10-membered heteroaryl containing one or more heteroatoms selected from oxygen, nitrogen and/or sulfur optionally substituted by one or more substituents selected from W³; or
any two R^{b} and R^{c}, R^{e} and R^{f} , R^{l} and R^{m} and/or R^{t} and R^{u} are linked together to form, along with the nitrogen atom to which they are attached, a 3- to 8-membered monocyclic or bicyclic ring, which ring optionally contains one or two further heteroatoms and which ring optionally is substituted by one or more substituents selected from W², C₁₋₃alkyl optionally substituted by one or more substituents selected from W¹, and =O;
each R^{f}, R^{j}, R^{o} and R^{r} independently represent -C₁₋₆ alkyl optionally substituted by one or more substituents selected from W¹, 3- to 12-membered heterocycloalkyl optionally substituted by one or more substituents selected from W², 6- to 14-membered aryl optionally substituted by one or more substituents selected from W³ or 5- to 10-membered heteroaryl containing one or more heteroatoms selected from oxygen, nitrogen and/or sulfur optionally substituted by one or more substituents selected from W³;
each Y² independently represents halogen, -CN, -C(O)R^{b1}, -C(O)N(R^{c1})R^{d1}, -C(O)OR^{e1}, -N(R^{f1})R^{g1}, -N(R^{h1})C(O)Rⁱ¹, -N(R^{j1})C(O)OR^{k1}, -N(R^{l1})C(O)N(R^{m1})Rⁿ¹, -N(R^{o1})S(O)₂R^{p1}, -OR^{q1}, -OC(O)R^{r1}, -OS(O)₂R^{s1}, -S(O)ₘR^{t1}, -S(O)₂N(R^{u1})R^{v1}, 3- to 12-membered heterocycloalkyl optionally substituted by one or more substituents selected from W¹, 6- to 14-membered aryl optionally substituted by one or more substituents selected from W², 5- to 10-membered heteroaryl containing one or more heteroatoms selected from oxygen, nitrogen and/or sulfur optionally substituted by one or more substituents selected from W²;
each R^{b1}, R^{c1}, R^{d1}, R^{e1}, R^{f1}, R^{h1}, Rⁱ¹, R^{j1}, R^{l1}, R^{m1}, Rⁿ¹, R^{o1}, R^{q1}, R^{r1}, R^{t1}, R^{u1} and R^{v1} independently represents hydrogen, C₁₋₆ alkyl optionally substituted by one or more substituents selected from W¹, 3- to 12-membered heterocycloalkyl optionally substituted by one or more substituents selected from W², 6- to 14-membered aryl optionally substituted by one or more substituents selected from W³, or 5- to 10-membered heteroaryl containing one or more heteroatoms selected from oxygen, nitrogen and/or sulfur optionally substituted by one or more substituents selected from W³; or
any two R^{c1} and R^{d1}, R^{f1} and R^{g1}, R^{m1} and Rⁿ¹ and/or R^{u1} and R^{v1} are linked together to form, along with the nitrogen atom to which they are attached, a 3- to 8-membered monocyclic or bicyclic ring, which ring optionally contains one or two further heteroatoms and which ring optionally is substituted by one or more substituents selected from W², C₁₋₃alkyl optionally substituted by one or more substituents selected from W¹, and =O;
each R^{g1}, R^{k1}, R^{p1} and R^{s1} independently represent -C₁₋₆ alkyl optionally substituted by one or more substituents selected from W¹, 3- to 12-membered heterocycloalkyl optionally substituted by one or more substituents selected from W², 6- to 14-membered aryl optionally substituted by one or more substituents selected from W³, or 5- to 10-membered heteroaryl containing one or more heteroatoms selected from oxygen, nitrogen and/or sulfur optionally substituted by one or more substituents selected from W³;
Z¹ represents -C(O)N(R^{c2})R^{d2}, -N(R^{f2})R^{g2}, -N(R^{h2})C(O)Rⁱ², -N(R^{l2})C(O)N(R^{m2})Rⁿ²,-N(R^{o2})S(O)₂R^{p2}, -OR^{q2}, -S(O)ₘR^{t2}, -S(O)₂N(R^{u2})R^{v2}, or aryl represented by 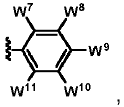
R^{c2}, R^{f2}, R^{h2}, R^{l2}, R^{m2}, R^{o2}, and R^{u2} represents hydrogen; and
R^{d2}, R^{g2}, Rⁱ², Rⁿ² R^{p2}, R^{q2}, R^{t2} and R^{v2} represents 6- to 14-membered aryl optionally substituted by one or more substituents selected from W⁶, or 5- to 10-membered heteroaryl containing one or more heteroatoms selected from oxygen, nitrogen and/or sulfur optionally substituted by one or more substituents selected from W⁶;
each W¹ independently represents halogen, -CN, -N(R^{f3})R^{g3}, -N(R^{h3})C(O)Rⁱ³,-N(R^{j3})C(O)OR^{k3}, -N(R^{l3})C(O)N(R^{m3})Rⁿ³, -N(R^{o3})S(O)₂R^{p3}, -OR^{q3}, -OC(O)R^{r3},-OS(O)₂R^{s3}, -S(O)ₘR^{t3}, -S(O)₂N(R^{u3})R^{v3}, 3- to 12-membered heterocycloalkyl optionally substituted by one or more substituents selected from G¹, 6- to 14-membered aryl optionally substituted by one or more substituents selected from G², 5- to 10-membered heteroaryl containing one or more heteroatoms selected from oxygen, nitrogen and/or sulfur optionally substituted by one or more substituents selected from G², or =O;
each W², W³, and W⁶ independently represents halogen, -R^{a3}, -CN, -C(O)R^{b3} -C(O)N(R^{c3})R^{d3}, -C(O)OR^{e3}, -N(R^{f3})R^{g3}, -N(R^{h3})C(O)Rⁱ³, -N(R^{j3})C(O)OR^{k3}, -N(R^{l3})C(O)N(R^{m3})Rⁿ³, -N(R^{o3})S(O)₂R^{p3}, -OR^{q3}, -OC(O)R^{r3}, -OS(O)₂R^{s3}, -S(O)ₘR^{t3}, -S(O)₂N(R^{u3})R^{v3}, 3- to 12-membered heterocycloalkyl optionally substituted by one or more substituents selected from G¹, 6- to 14-membered aryl optionally substituted by one or more substituents selected from G², 5- to 10-membered heteroaryl containing one or more heteroatoms selected from oxygen, nitrogen and/or sulfur optionally substituted by one or more substituents selected from G², or =O;
W⁷ and W¹¹ represent hydrogen;
each W⁸ and W¹⁰ independently represents halogen, -R^{a3}, -CN, -C(O)R^{b3}, -C(O)N(R^{c3})R^{d3}, -C(O)OR^{e3}, -N(R^{f3})R^{g3}, -N(R^{h3})C(O)Rⁱ³, -N(R^{j3})C(O)OR^{k3}, -N(R^{l3})C(O)N(R^{m3})Rⁿ³, -N(R^{o3})S(O)₂R^{p3}, -OR^{q3}, -OC(O)R^{r3}, -OS(O)₂R^{s3}, -S(O)ₘR^{t3}, -S(O)₂N(R^{u3})R^{v3}, 3- to 12-membered heterocycloalkyl optionally substituted by one or more substituents selected from G¹, 6- to 14-membered aryl optionally substituted by one or more substituents selected from G², or 5- to 10-membered heteroaryl containing one or more heteroatoms selected from oxygen, nitrogen and/or sulfur optionally substituted by one or more substituents selected from G²;
W⁹ represents halogen, -R^{a3}, -CN, -C(O)R^{b3}, -C(O)N(R^{c3})R^{d3}, -C(O)OR^{e3},-N(R^{f3})R^{g3}, -N(R^{h3})C(O)Rⁱ³, -N(R^{j3})C(O)OR^{k3}, -N(R¹³)C(O)N(R^{m3})Rⁿ³,-N(R^{o3})S(O)₂R^{p3}, -OR^{q3x}, -OC(O)R^{r3}, -OS(O)₂R^{s3}, -S(O)ₘR^{t3}, -S(O)₂N(R^{u3})R^{v3}, 3- to 12-membered heterocycloalkyl optionally substituted by one or more substituents selected from G¹, 6- to 14-membered aryl optionally substituted by one or more substituents selected from G², or 5- to 10-membered heteroaryl containing one or more heteroatoms selected from oxygen, nitrogen and/or sulfur optionally substituted by one or more substituents selected from G²;
each R^{a3}, R^{b3}, R^{c3}, R^{d3}, R^{e3}, R^{f3}, R^{h3}, Rⁱ³, R^{j3}, R^{l3}, R^{m3}, Rⁿ³, R^{o3}, R^{q3}, R^{r3}, R^{t3}, R^{u3} and R^{v3} independently represents hydrogen or -C₁₋₆ alkyl optionally substituted by one or more G³; or
any two R^{c3} and R^{d3}, R^{f3} and R^{g3}, R^{m3} and Rⁿ³ and/or R^{u3} and R^{v3} are linked together to form, along with the nitrogen atom to which they are attached, a 4- to 6-membered ring, which ring optionally contains one further heteroatom and which ring optionally is substituted by one or more G²;
each R^{g3}, R^{k3}, R^{p3}, and R^{s3} independently represent C₁₋₆ alkyl optionally substituted by one or more G³;
R^{q3x} represents C₂₋₆ alkyl optionally substituted by one or more G³;
each G¹ and G² independently represents halogen, -R^{a4}, -CN, -C(O)R^{b4}, -C(O)N(R^{c4})R^{d4}, -C(O)OR^{e4}, -N(R^{f4})R^{g4}, -N(R^{h4})C(O)Rⁱ⁴, -N(R^{j4})C(O)OR^{k4}, -N(R^{l4})C(O)N(R^{m4})Rⁿ⁴, -N(R^{o4})S(O)₂R^{p4}, -OR^{q4}, -OC(O)R^{r4}, -OS(O)₂R^{s4}, -S(O)ₘR^{t4}, -S(O)₂N(R^{u4})R^{v4}, or =O;
G³ represents halogen, -CN, -C(O)R^{b4}, -C(O)N(R^{c4})R^{d4}, -C(O)OR^{e4}, -N(R^{f4})R^{g4}, -N(R^{h4})C(O)Rⁱ⁴, -N(R^{j4})C(O)OR^{k4}, -N(R^{l4})C(O)N(R^{m4})Rⁿ⁴, -N(R^{o4})S(O)₂R^{p4}, -OR^{q4}, -OC(O)R^{r4}, -OS(O)₂R^{s4}, -S(O)ₘR^{t4}, -S(O)₂N(R^{u4})R^{v4}, or =O;
each R^{a4}, R^{b4}, R^{c4}, R^{d4}, R^{e4}, R^{f4}, R^{h4}, Rⁱ⁴, R^{j4}, R^{l4}, R^{m4}, Rⁿ⁴, R^{o4}, R^{q4}, R^{r4}, R^{t4}, R^{u4} and R^{v4} independently represents hydrogen or -C₁₋₆ alkyl optionally substituted by one or more -F; or
any two R^{c4} and R^{d4}, R^{f4} and R^{g4}, R^{m4} and Rⁿ⁴ and/or R^{u4} and R^{v4} are linked together to form, along with the nitrogen atom to which they are attached, a 3- to 6-membered ring, which ring optionally is substituted by one or more -F, -CH₃, -CH₂CH₃, -CHF₂, -CF₃, -CH₂CF₃, or =O;
each R^{g4}, R^{k4}, R^{p4} and R^{s4} independently represents C₁₋₆ alkyl optionally substituted by one or more -F; and
each m independently represents 0, 1 or 2.

2. A compound as claimed in claim 1, wherein R¹ represents heteroaryl.

3. A compound as claimed in Claim 1, wherein R¹ represents 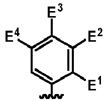 where:
E³ and E⁴ represent hydrogen; and
E¹ represents -F and E² represent -F, -Cl, -CH₃ or -CF₃; or
E¹ represents -Cl and E² represents -F, -Cl, -CH₃ or -CF₃; or
E¹ represents -CH₃ and E² represents -F, -Cl, -CH₃, -CF₃, or -CN.

4. A compound as claimed in any one of the Claims 1 to 3, wherein:
Z¹ represents 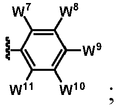 and
(a) W⁸ and W¹⁰ are independently selected from hydrogen, -F, -CI, -CH₃, -CN, and -CF₃; and
W⁹ is selected from -R^{a3}, -CN, -C(O)R^{b3}, -C(O)N(R^{c3})R^{d3}, -C(O)OR^{e3}, -N(R^{f3})R^{g3}, -N(R^{h3})C(O)Rⁱ³, -N(R^{j3})C(O)OR^{k3}, -N(R^{l3})C(O)N(R^{m3})Rⁿ³, -N(R^{o3})S(O)₂R^{p3}, -OR^{q3x}, -OC(O)R^{r3}, -OS(O)₂R^{s3}, -S(O)ₘR^{t3}, -S(O)₂N(R^{u3})R^{v3}, 3- to 12-membered heterocycloalkyl optionally substituted by one or more substituents selected from G¹, 6- to 14-membered aryl optionally substituted by one or more substituents selected from G², or 5- to 10-membered heteroaryl containing one or more heteroatoms selected from oxygen, nitrogen and/or sulfur optionally substituted by one or more substituents selected from G²; or
(b) W⁹ and W¹⁰ are independently selected from hydrogen, -F, -CI, -CH₃, -CN, and -CF₃; and W⁸ is selected from -R^{a3}, -CN, -C(O)R^{b3}, -C(O)N(R^{c3})R^{d3}, -C(O)OR^{e3}, -N(R^{f3})R^{g3}, -N(R^{h3})C(O)Rⁱ³, -N(R^{j3})C(O)OR^{k3}, -N(R^{l3})C(O)N(R^{m3})Rⁿ³, -N(R^{o3})S(O)₂R^{p3}, -OR^{q3x}, -OC(O)R^{r3}, -OS(O)₂R^{s3}, -S(O)ₘR^{t3}, -S(O)₂N(R^{u3})R^{v3}, 3- to 12-membered heterocycloalkyl optionally substituted by one or more substituents selected from G¹, 6- to 14-membered aryl optionally substituted by one or more substituents selected from G², or 5- to 10-membered heteroaryl containing one or more heteroatoms selected from oxygen, nitrogen and/or sulfur optionally substituted by one or more substituents selected from G².

5. A compound as claimed in any one of the Claims 1 to 4, wherein:
W⁸ and W¹⁰ represent hydrogen and W⁹ represents -F, -CI, -CH₃, cyclopropyl,-CF₃, -CN, -NH₂, -N(CH₃)₂, -N(H)C(O)CH₃, -N(H)C(O)OC(CH₃)₃, -SO₂CH₃,-SO₂NH₂, -S(O)₂N(CH₃)₂, -S(O)₂-4-morpholinyl, 4-methylpiperazin-1-yl, 4-methyl-piperidin-1-ylmethyl or 1,2,3-thiadiazol-4-yl.

6. A compound as claimed in any one of the Claims 1 to 3, wherein:
Z¹ represents -N(R^{f2})R^{g2}, R^{f2} represents hydrogen and R^{g2} represents 6- to 14-membered aryl optionally substituted by one or more substituents selected from W⁶, or 5- to 10-membered heteroaryl containing one or more heteroatoms selected from oxygen, nitrogen and/or sulfur optionally substituted by one or more substituents selected from W⁶.

7. A compound as claimed in any one of the Claims 1 to 3, wherein:
Z¹ represents -OR^{q2} and R^{q2} represents 6- to 14-membered aryl optionally substituted by one or more substituents selected from W⁶, or 5- to 10-membered heteroaryl containing one or more heteroatoms selected from oxygen, nitrogen and/or sulfur optionally substituted by one or more substituents selected from W⁶.

8. A compound as claimed in any one of the Claims 1, 2, 3, 6, or 7, wherein:
W⁶ represents -F, -Cl, -Br, -CH₃, cyclopropyl, -CF₃, -CN, -N(CH₃)₂, -SO₂CH₃, -SO₂NH₂ or -SO₂N(CH₃)₂.

9. A compound as claimed in Claim 1, selected from:
6-(2,3-dimethylphenyl)-4-N-(2-phenylethyl)pyrimidine-2,4-diamine;
6-(2,3-dimethylphenyl)-4-N-[2-(4-methoxyphenyl)ethyl]pyrimidine-2,4-diamine;
6-(2,3-dimethylphenyl)-4-N-[2-(4-methylphenyl)ethyl]pyrimidine-2,4-diamine;
4-N-[2-(4-chlorophenyl)ethyl]-6-(2,3-dimethylphenyl)pyrimidine-2,4-diamine;
6-(2,3-dimethylphenyl)-4-N-(2-phenylpropyl)pyrimidine-2,4-diamine;
6-(2,3-dimethylphenyl)-4-N-(3-phenylpropyl)pyrimidine-2,4-diamine;
6-(2,3-dimethylphenyl)-4-N-(2-phenoxyethyl)pyrimidine-2,4-diamine;
6-(2,3-dimethylphenyl)-4-N-[2-(phenylamino)ethyl]pyrimidine-2,4-diamine;
6-(2-chloro-3-methylphenyl)-4-N-[2-(4-chlorophenyl)ethyl]pyrimidine-2,4-diamine;
4-N-[2-(4-chlorophenyl)ethyl]-6-(1H-indol-4-yl)pyrimidine-2,4-diamine;
6-(3-chloro-2-methylphenyl)-4-N-[(1R)-1-phenylethyl]pyrimidine-2,4-diamine;
6-(3-chloro-2-methylphenyl)-4-N-(2-phenylpropan-2-yl)pyrimidine-2,4-diamine;
4-N-[2-(2-chlorophenoxy)ethyl]-6-(2,3-dimethylphenyl)pyrimidine-2,4-diamine;
6-(2,3-dimethylphenyl)-4-N-[2-(pyridin-3-yloxy)propyl]pyrimidine-2,4-diamine;
4-N-[2-(4-chlorophenyl)ethyl]-6-(2,3-dichlorophenyl)pyrimidine-2,4-diamine;
4-N-[2-(4-chlorophenyl)ethyl]-6-(quinolin-5-yl)pyrimidine-2,4-diamine;
4-N-[2-(4-chlorophenyl)cyclopropyl]-6-(2,3-dimethylphenyl)pyrimidine-2,4-diamine;
3-(2-{[2-amino-6-(2,3-dimethylphenyl)pyrimidin-4-yl]amino}ethyl)phenol;
4-N-{[1-(4-chlorophenyl)cyclopropyl]methyl}-6-(2,3-dimethylphenyl)pyrimidine-2,4-diamine;
4-(2-{[2-amino-6-(2,3-dichlorophenyl)pyrimidin-4-yl]amino}ethyl)benzene-1-sulfonamide;
4-(2-{[2-amino-6-(3-chloro-2-methylphenyl)pyrimidin-4-yl]amino}ethyl)benzene-1-sulfonamide;
4-(2-{[2-amino-6-(2,3-dichlorophenyl)pyrimidin-4-yl]amino}ethyl)phenol;
4-(2-{[2-amino-6-(2,3-dimethylphenyl)pyrimidin-4-yl]amino}ethyl)benzene-1-sulfonamide;
6-(2,3-dimethylphenyl)-4-N-[2-(4-methanesulfonylphenyl)ethyl]pyrimidine-2,4-diamine;
4-(2-{[2-amino-6-(2,3-dimethylphenyl)pyrimidin-4-yl]amino}ethyl)benzonitrile,
4-(2-{[2-amino-6-(2,3-dimethylphenyl)pyrimidin-4-yl]amino}ethoxy)benzonitrile,
4-N-[2-(3-chlorophenyl)ethyl]-6-(2,3-dimethylphenyl)pyrimidine-2,4-diamine;
4-N-[2-(4-chlorophenyl)propyl]-6-(2,3-dimethylphenyl)pyrimidine-2,4-diamine;
6-(2,3-dimethylphenyl)-4-N-[2-(4-fluorophenyl)ethyl]pyrimidine-2,4-diamine;
4-N-[2-(benzenesulfonyl)ethyl]-6-(2,3-dimethylphenyl)pyrimidine-2,4-diamine;
N-(3-{[2-amino-6-(2,3-dimethylphenyl)pyrimidin-4-yl]amino}propyl)-4-chlorobenzene-1-sulfonamide;
3-(3-{[2-amino-6-(2,3-dimethylphenyl)pyrimidin-4-yl]amino}propyl)-1-(4-chlorophenyl)urea;
N-(3-{[2-amino-6-(2,3-dimethylphenyl)pyrimidin-4-yl]amino}propyl)-3,5-dimethyl-1,2-oxazole-4-sulfonamide;
3-(3-{[2-amino-6-(2,3-dimethylphenyl)pyrimidin-4-yl]amino}propyl)-1-(2,6-dichloropyridin-4-yl)urea;
3-(3-{[2-amino-6-(2,3-dimethylphenyl)pyrimidin-4-yl]amino}propyl)-1-(3,4-difluorophenyl)urea;
N-[3-(3-{[2-amino-6-(2,3-dimethylphenyl)pyrimidin-4-yl]amino}propoxy)phenyl]acetamide;
N-(3-{[2-amino-6-(2,3-dimethylphenyl)pyrimidin-4-yl]amino}propyl)-3-bromobenzamide;
N-(3-{[2-amino-6-(2,3-dimethylphenyl)pyrimidin-4-yl]amino}propyl)benzamide;
N-(3-{[2-amino-6-(2,3-dimethylphenyl)pyrimidin-4-yl]amino}propyl)-3-cyanobenzene-1-sulfonamide;
N-(3-{[2-amino-6-(2,3-dimethylphenyl)pyrimidin-4-yl]amino}propyl)-4-methanesulfonylbenzene-1-sulfonamide;
N-(3-{[2-amino-6-(2,3-dimethylphenyl)pyrimidin-4-yl]amino}propyl)benzenesulfonamide;
N-(3-{[2-amino-6-(2,3-dimethylphenyl)pyrimidin-4-yl]amino}-2,2-dimethylpropyl)-3-fluorobenzene-1-sulfonamide;
N-(3-{[2-amino-6-(2,3-dimethylphenyl)pyrimidin-4-yl]amino}-2,2-dimethylpropyl)-4-(morpholine-4-sulfonyl)benzene-1-sulfonamide;
N-(3-{[2-amino-6-(2,3-dimethylphenyl)pyrimidin-4-yl]amino}propyl)-3-fluorobenzene-1-sulfonamide;
N-{4-[(3-{[2-amino-6-(2,3-dimethylphenyl)pyrimidin-4-yl]amino}propyl)sulfamoyl]phenyl}acetamide;
N-(2-{[2-amino-6-(2,3-dimethylphenyl)pyrimidin-4-yl]amino}ethyl)-3-fluorobenzene-1-sulfonamide;
N-(2-{[2-amino-6-(2,3-dimethylphenyl)pyrimidin-4-yl]amino}ethyl)-4-methoxybenzene-1-sulfonamide;
N-(3-{[2-amino-6-(2,3-dimethylphenyl)pyrimidin-4-yl]amino}propyl)-4,5-dichlorothiophene-2-sulfonamide;
N-(3-{[2-amino-6-(2,3-dimethylphenyl)pyrimidin-4-yl]amino}propyl)-5-chloro-1,3-dimethyl-1H-pyrazole-4-sulfonamide;
N-(3-{[2-amino-6-(2,3-dimethylphenyl)pyrimidin-4-yl]amino}propyl)-1,2-dimethyl-1H-imidazole-4-sulfonamide;
N-(2-{[2-amino-6-(2,3-dimethylphenyl)pyrimidin-4-yl]amino}ethyl)-5-chloro-1,3-dimethyl-1H-pyrazole-4-sulfonamide;
4-N-{2-[(1,3-benzoxazol-2-yl)amino]ethyl}-6-(2,3-dimethylphenyl)pyrimidine-2,4-diamine;
6-(2,3-dimethylphenyl)-4-N-(4-phenylbutan-2-yl)pyrimidine-2,4-diamine;
6-[(2-{[2-amino-6-(2,3-dimethylphenyl)pyrimidin-4-yl]amino}ethyl)amino]pyridine-3-carbonitrile,
4-N-{2-[(3-bromo-1,2,4-thiadiazol-5-yl)amino]ethyl}-6-(2,3-dimethylphenyl)pyrimidine-2,4-diamine;
N-(2-{[2-amino-6-(2,3-dimethylphenyl)pyrimidin-4-yl]amino}ethyl)-4-methanesulfonylbenzene-1-sulfonamide;
N-(3-{[2-amino-6-(2,3-dimethylphenyl)pyrimidin-4-yl]amino}propyl)-3-methanesulfonylbenzene-1-sulfonamide;
N-(3-{[2-amino-6-(2,3-dimethylphenyl)pyrimidin-4-yl]amino}propyl)-2-methanesulfonylbenzene-1-sulfonamide;
4-(2-{[2-amino-6-(3-chloro-2-methylphenyl)pyrimidin-4-yl]amino}ethyl)-N,N-dimethylbenzene-1-sulfonamide;
4-(2-{[2-amino-6-(2,3-dichlorophenyl)pyrimidin-4-yl]amino}ethyl)-N,N-dimethylbenzene-1-sulfonamide;
4-(2-{[2-amino-6-(2,3-dimethylphenyl)pyrimidin-4-yl]amino}ethyl)-N,N-dimethylbenzene-1-sulfonamide;
4-N-{1-[(4-chlorophenyl)methyl]cyclopropyl}-6-(2,3-dimethylphenyl)pyrimidine-2,4-diamine;
4-N-[2-(4-chlorophenyl)ethyl]-6-(1H-pyrrol-2-yl)pyrimidine-2,4-diamine;
N-(4-{[2-amino-6-(2,3-dimethylphenyl)pyrimidin-4-yl]amino}butyl)-4-methanesulfonylbenzene-1-sulfonamide;
4-(2-{[2-amino-6-(3-cyano-2-methylphenyl)pyrimidin-4-yl]amino}ethyl)benzene-1-sulfonamide;
4-N-{2-[(6-chloropyridazin-3-yl)amino]ethyl}-6-(2,3-dimethylphenyl)pyrimidine-2,4-diamine;
2-[(2-{[2-amino-6-(2,3-dimethylphenyl)pyrimidin-4-yl]amino}ethyl)amino]pyridine-4-carbonitrile,
6-[(2-{[2-amino-6-(2,3-dimethylphenyl)pyrimidin-4-yl]amino}ethyl)amino]pyridine-3-sulfonamide;
1-N-(3-{[2-amino-6-(2,3-dimethylphenyl)pyrimidin-4-yl]amino}propyl)benzene-1,4-disulfonamide;
6-(2,3-dichlorophenyl)-4-N-[2-(4-methanesulfonylphenyl)ethyl]pyrimidine-2,4-diamine;
6-(2,3-dimethylphenyl)-4-N-{2-[(3-methoxyphenyl)amino]ethyl}pyrimidine-2,4-diamine;
6-(2,3-dimethylphenyl)-4-N-{2-[(3-fluoro-4-methylphenyl)amino]ethyl}pyrimidine-2,4-diamine;
4-N-{2-[(3,4-dichlorophenyl)amino]ethyl}-6-(2,3-dimethylphenyl)pyrimidine-2,4-diamine;
4-N-{2-[(5-chloropyridin-2-yl)amino]ethyl}-6-(2,3-dimethylphenyl)pyrimidine-2,4-diamine;
4-N-{2-[(5-bromopyridin-2-yl)amino]ethyl}-6-(2,3-dimethylphenyl)pyrimidine-2,4-diamine;
4-[(2-{[2-amino-6-(2,3-dimethylphenyl)pyrimidin-4-yl]amino}ethyl)amino]benzene-1-sulfonamide; and
6-(2,3-dimethylphenyl)-4-N-{2-[(pyridin-3-yl)amino]ethyl}pyrimidine-2,4-diamine;
or a pharmaceutically acceptable salt thereof.

10. A compound as claimed in Claim 1, selected from:
4-N-[2-(4-chlorophenyl)ethyl]-6-(2,3-dichlorophenyl)pyrimidine-2,4-diamine;
4-(2-{[2-amino-6-(2,3-dichlorophenyl)pyrimidin-4-yl]amino}ethyl)benzene-1-sulfonamide;
4-(2-{[2-amino-6-(3-chloro-2-methylphenyl)pyrimidin-4-yl]amino}ethyl)benzene-1-sulfonamide;
4-(2-{[2-amino-6-(2,3-dimethylphenyl)pyrimidin-4-yl]amino}ethyl)benzene-1-sulfonamide;
6-(2,3-dimethylphenyl)-4-N-[2-(4-methanesulfonylphenyl)ethyl]pyrimidine-2,4-diamine;
4-(2-{[2-amino-6-(3-chloro-2-methylphenyl)pyrimidin-4-yl]amino}ethyl)-N,N-dimethylbenzene-1-sulfonamide;
4-(2-{[2-amino-6-(2,3-dichlorophenyl)pyrimidin-4-yl]amino}ethyl)-N,N-dimethylbenzene-1-sulfonamide;
4-(2-{[2-amino-6-(2,3-dimethylphenyl)pyrimidin-4-yl]amino}ethyl)-N,N-dimethylbenzene-1-sulfonamide;
6-[(2-{[2-amino-6-(2,3-dimethylphenyl)pyrimidin-4-yl]amino}ethyl)amino]pyridine-3-sulfonamide;
6-(2,3-dichlorophenyl)-4-N-[2-(4-methanesulfonylphenyl)ethyl]pyrimidine-2,4-diamine; and
4-[(2-{[2-amino-6-(2,3-dimethylphenyl)pyrimidin-4-yl]amino}ethyl)amino]benzene-1-sulfonamide,
or a pharmaceutically acceptable salt thereof.

11. A pharmaceutical formulation comprising a compound as claimed in any one of Claims 1 to 10, or a pharmaceutically acceptable salt thereof, in admixture with a pharmaceutically acceptable adjuvant, diluent or carrier.

12. A compound as claimed in any one of the Claims 1 to 10, or a pharmaceutically acceptable salt thereof, for use in the treatment of cancer.

13. A compound for use as claimed in Claim 12, wherein the cancer is selected from Soft Tissue Cancers: sarcoma (angiosarcoma, fibrosarcoma, rhabdomyosarcoma, liposarcoma), myxoma, rhabdomyoma, fibroma, lipoma and teratoma; Lung cancers/diseases: bronchogenic carcinoma (squamous cell, undifferentiated small cell, undifferentiated large cell, adenocarcinoma), alveolar (bronchiolar) carcinoma, bronchial adenoma, sarcoma, lymphoma, chondromatous hamartoma, mesothelioma; Gastrointestinal cancers/diseases: esophagus (squamous cell carcinoma, adenocarcinoma, leiomyosarcoma, lymphoma), stomach (carcinoma, lymphoma, leiomyosarcoma), pancreas (ductal adenocarcinoma, insulinoma, glucagonoma, gastrinoma, carcinoid tumors, vipoma), small bowel (adenocarcinoma, lymphoma, carcinoid tumors, Kaposi's sarcoma, leiomyoma, hemangioma, lipoma, neurofibroma, fibroma), large bowel (adenocarcinoma, tubular adenoma, villous adenoma, hamartoma, leiomyoma); Genitourinary tract cancers/diseases: kidney (adenocarcinoma, Wilm's tumor [nephroblastoma], lymphoma, leukemia), bladder and urethra (squamous cell carcinoma, transitional cell carcinoma, adenocarcinoma), prostate (adenocarcinoma, sarcoma), testis (seminoma, teratoma, embryonal carcinoma, teratocarcinoma, choriocarcinoma, sarcoma, interstitial cell carcinoma, fibroma, fibroadenoma, adenomatoid tumors, lipoma); Liver cancers/diseases: hepatoma (hepatocellular carcinoma), cholangiocarcinoma, hepatoblastoma, angiosarcoma, hepatocellular adenoma, hemangioma; Bone cancers/diseases: osteogenic sarcoma (osteosarcoma), fibrosarcoma, malignant fibrous histiocytoma, chondrosarcoma, Ewing's sarcoma, malignant lymphoma (reticulum cell sarcoma), multiple myeloma, malignant giant cell tumor chordoma, osteochronfroma (osteocartilaginous exostoses), benign chondroma, chondroblastoma, chondromyxofibroma, osteoid osteoma and giant cell tumors; Nervous system cancers/diseases: skull (osteoma, hemangioma, granuloma, xanthoma, osteitis deformans), meninges (meningioma, meningiosarcoma, gliomatosis), brain (astrocytoma, medulloblastoma, glioma, ependymoma, germinoma [pinealoma], glioblastoma multiform, oligodendroglioma, schwannoma, retinoblastoma, congenital tumors), spinal cord neurofibroma, meningioma, glioma, sarcoma); Gynecological cancers/diseases: uterus (endometrial carcinoma), cervix (cervical carcinoma, pre-tumor cervical dysplasia), ovaries (ovarian carcinoma [serous cystadenocarcinoma, mucinous cystadenocarcinoma, unclassified carcinoma], granulosa-thecal cell tumors, Sertoli- Leydig cell tumors, dysgerminoma, malignant teratoma), vulva (squamous cell carcinoma, intraepithelial carcinoma, adenocarcinoma, fibrosarcoma, melanoma), vagina (clear cell carcinoma, squamous cell carcinoma, botryoid sarcoma (embryonal rhabdomyosarcoma], fallopian tubes (carcinoma); Hematologic cancers/diseases: blood and bone marrow (myeloid leukemia [acute and chronic], acute lymphoblastic leukemia, chronic lymphocytic leukemia, myeloproliferative diseases, multiple myeloma, myelodysplastic syndrome), Hodgkin's disease, non-Hodgkin's lymphoma [malignant lymphoma]; Skin cancers/diseases: malignant melanoma, basal cell carcinoma, squamous cell carcinoma, Kaposi's sarcoma, moles dysplastic nevi, lipoma, angioma, dermatofibroma, keloids; Adrenal glands cancers/diseases, neuroblastoma, neurofibromatosis and head and neck cancers.

14. A compound for use as claimed in Claim 12, wherein the cancer is selected from the group consisting of breast cancers, prostate cancers, lung cancers, oesophageal cancers, colon cancers, brain cancers, skin cancers, ovarian cancers, testicular cancers, neurofibromatosis and leukemias.

15. A compound as claimed in any one of the Claims 1 to 10, or a pharmaceutically acceptable salt thereof, for use as a pharmaceutical.

16. A combination product comprising:
(A) a compound as claimed in any one of Claims 1 to 10, or a pharmaceutically acceptable salt thereof; and
(B) one or more other therapeutic agent(s) that is/are useful in the treatment of a disease as defined in Claim 13 or 14,
wherein each one of components (A) and (B) is formulated in admixture with a pharmaceutically-acceptable adjuvant, diluent or carrier.

17. A combination product as claimed in Claim 16, wherein component (B) is selected from the group consisting of anti-microtubule agents, platinum coordination complexes, alkylating agents, antibiotic agents, topoisomerase II inhibitors, antimetabolites, topoisomerase I inhibitors, hormones and hormonal analogues, signal transduction pathway inhibitors; kinase inhibitors; angiogenesis inhibitors; immunotherapeutic agents; pro-apoptotic agents; and cell cycle signaling inhibitors.

## Patentansprüche

1. Verbindung der Formel I, 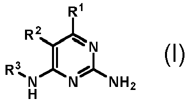 oder ein pharmazeutisch annehmbares Salz davon, wobei:
R¹ Heteroaryl, ausgewählt aus Benzofuran-3-yl, Benzothiophen-3-yl, Dihydrobenzofuran-7-yl, Indol-3-yl, Indol-4-yl, Indol-5-yl, Isochinolin-4-yl, Pyridin-3-yl, Pyridin-4-yl, Pyrrol-2-yl und Chinolin-5-yl, wobei das Heteroaryl über ein Kohlenstoffatom des Heteroarylrings mit dem Pyrimidin von Formel I verbunden ist, und wobei das Heteroaryl wahlweise mit einem oder mehreren Substituenten, ausgewählt aus Y¹ oder -C₁₋₆-Alkyl, wahlweise substituiert mit einem oder mehreren Y², substituiert ist, oder Aryl, dargestellt durch 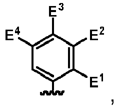 darstellt;
E³ und E⁴ Wasserstoff darstellen und
E¹ -F darstellt und E² -F, -Cl, -CH₃ oder -CF₃ darstellt oder
E¹ -Cl darstellt und E² -F, -Cl, -CH₃ oder -CF₃ darstellt oder
E¹ -CH₃ darstellt und E² -F, -Cl, -CH₃, -CF₃ oder -CN darstellt;
R² Wasserstoff darstellt;
R³ durch Z¹ substituiertes -C₂₋₆-Alkyl darstellt;
jedes Y¹ unabhängig Halogen, -CN, -C(O)R^{a}, -C(O)N(R^{b})R^{c}, -C(O)OR^{d}, -N(R^{e})R^{f}, -N(R^{g})C(O)R^{h}, -N(Rⁱ)C(O)OR^{j}, -N(R^{k})C(O)N(R^{l})R^{m}, -NO₂, -N(Rⁿ)S(O)₂R^{o}, -OR^{p}, -OC(O)R^{q}, -OS(O)₂R^{r}, -S(O)ₘR^{s}, -S(O)₂N(R^{t})R^{u}, 3- bis 12-gliedriges Heterocycloalkyl, wahlweise substituiert mit einem oder mehreren aus W² ausgewählten Substituenten, 6- bis 14-gliedriges Aryl, wahlweise substituiert mit einem oder mehreren aus W³ ausgewählten Substituenten, oder 5- bis 10-gliedriges Heteroaryl, das ein oder mehrere Heteroatome enthält, die aus Sauerstoff, Stickstoff und/oder Schwefel ausgewählt sind, wahlweise substituiert mit einem oder mehreren aus W³ ausgewählten Substituenten, darstellt;
jedes R^{a}, R^{b}, R^{c}, R^{d}, R^{e}, R^{g}, R^{h}, Rⁱ, R^{k}, R^{l}, R^{m}, Rⁿ, R^{p}, R^{q}, R^{s}, R^{t} und R^{u} unabhängig Wasserstoff, -C₁₋₆-Alkyl, wahlweise substituiert mit einem oder mehreren aus W¹ ausgewählten Substituenten, 3- bis 12-gliedriges Heterocycloalkyl, wahlweise substituiert mit einem oder mehreren aus W² ausgewählten Substituenten, 6- bis 14-gliedriges Aryl, wahlweise substituiert mit einem oder mehreren aus W³ ausgewählten Substituenten, oder 5- bis 10-gliedriges Heteroaryl, das ein oder mehrere Heteroatome enthält, die aus Sauerstoff, Stickstoff und/oder Schwefel ausgewählt sind, wahlweise substituiert mit einem oder mehreren aus W³ ausgewählten Substituenten, darstellt oder
beliebige zwei R^{b} und R^{c}, R^{e} und R^{f}, R^{l} und R^{m} und/oder R^{t} und R^{u} miteinander verbunden sind, um zusammen mit dem Stickstoffatom, an das sie gebunden sind, einen 3- bis 8-gliedrigen monocyclischen oder bicyclischen Ring zu bilden, wobei der Ring wahlweise ein oder zwei weitere Heteroatome enthält und wobei der Ring wahlweise mit einem oder mehreren Substituenten, ausgewählt aus W², C₁₋₃-Alkyl, wahlweise substituiert mit einem oder mehreren aus W¹ ausgewählten Substituenten, und =O, substituiert ist;
jedes R^{f}, R^{j}, R^{o} und R^{r} unabhängig -C₁₋₆-Alkyl, wahlweise substituiert mit einem oder mehreren aus W¹ ausgewählten Substituenten, 3- bis 12-gliedriges Heterocycloalkyl, wahlweise substituiert mit einem oder mehreren aus W² ausgewählten Substituenten, 6- bis 14-gliedriges Aryl, wahlweise substituiert mit einem oder mehreren aus W³ ausgewählten Substituenten, oder 5- bis 10-gliedriges Heteroaryl, das ein oder mehrere Heteroatome enthält, die aus Sauerstoff, Stickstoff und/oder Schwefel ausgewählt sind, wahlweise substituiert mit einem oder mehreren aus W³ ausgewählten Substituenten, darstellt;
jedes Y² unabhängig Halogen, -CN, -C(O)R^{b1}, -C(O)N(R^{c1})R^{d1}, -C(O)OR^{e1}, -N(R^{f1})R^{g1}, -N(R^{h1})C(O)Rⁱ¹, -N(R^{j1})C(O)OR^{k1}, -N(R^{l1})C(O)N(R^{m1})Rⁿ¹, -N(R^{o1})S(O)₂R^{p1}, -OR^{q1}, -OC(O)R^{r1}, -OS(O)₂R^{s1}, -S(O)ₘR^{t1}, -S(O)₂N(R^{u1})R^{v1}, 3- bis 12-gliedriges Heterocycloalkyl, wahlweise substituiert mit einem oder mehreren aus W¹ ausgewählten Substituenten, 6- bis 14-gliedriges Aryl, wahlweise substituiert mit einem oder mehreren aus W² ausgewählten Substituenten, 5- bis 10-gliedriges Heteroaryl, das ein oder mehrere Heteroatome enthält, die aus Sauerstoff, Stickstoff und/oder Schwefel ausgewählt sind, wahlweise substituiert mit einem oder mehreren aus W² ausgewählten Substituenten, darstellt;
jedes R^{b1}, R^{c1}, R^{d1}, R^{e1}, R^{f1}, R^{h1}, Rⁱ¹, R^{j1}, R^{l1}, R^{m1}, Rⁿ¹, R^{o1}, R^{q1}, R^{r1}, R^{t1}, R^{u1} und R^{v1} unabhängig Wasserstoff, -C₁₋₆-Alkyl, wahlweise substituiert mit einem oder mehreren aus W¹ ausgewählten Substituenten, 3- bis 12-gliedriges Heterocycloalkyl, wahlweise substituiert mit einem oder mehreren aus W² ausgewählten Substituenten, 6- bis 14-gliedriges Aryl, wahlweise substituiert mit einem oder mehreren aus W³ ausgewählten Substituenten, oder 5- bis 10-gliedriges Heteroaryl, das ein oder mehrere Heteroatome enthält, die aus Sauerstoff, Stickstoff und/oder Schwefel ausgewählt sind, wahlweise substituiert mit einem oder mehreren aus W³ ausgewählten Substituenten, darstellt oder
beliebige zwei R^{c1} und R^{d1}, R^{f1} und R^{g1}, R^{m1} und Rⁿ¹ und/oder R^{u1} und R^{v1} miteinander verbunden sind, um zusammen mit dem Stickstoffatom, an das sie gebunden sind, einen 3- bis 8-gliedrigen monocyclischen oder bicyclischen Ring zu bilden, wobei der Ring wahlweise ein oder zwei weitere Heteroatome enthält und wobei der Ring wahlweise mit einem oder mehreren Substituenten, ausgewählt aus W², C₁₋₃-Alkyl, wahlweise substituiert mit einem oder mehreren aus W¹ ausgewählten Substituenten, und =O, substituiert ist;
jedes R^{g1}, R^{k1}, R^{p1} und R^{s1} unabhängig -C₁₋₆-Alkyl, wahlweise substituiert mit einem oder mehreren aus W¹ a ausgewählten Substituenten, 3- bis 12-gliedriges Heterocycloalkyl, wahlweise substituiert mit einem oder mehreren aus W² ausgewählten Substituenten, 6- bis 14-gliedriges Aryl, wahlweise substituiert mit einem oder mehreren aus W³ ausgewählten Substituenten, oder 5- bis 10-gliedriges Heteroaryl, das ein oder mehrere Heteroatome enthält, die aus Sauerstoff, Stickstoff und/oder Schwefel ausgewählt sind, wahlweise substituiert mit einem oder mehreren aus W³ ausgewählten Substituenten, darstellt;
Z¹ -C(O)N(R^{c2})R^{d2}, -N(R^{f2})R^{g2}, -N(R^{h2})C(O)Rⁱ², -N(R¹²)C(O)N(R^{m2})Rⁿ², -N(R^{o2})S(O)₂R^{p2}, -OR^{q2}, -S(O)ₘR^{t2}, -S(O)₂N(R^{u2})R^{v2} oder Aryl, dargestellt durch 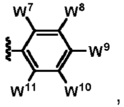 darstellt,
R^{c2}, R^{f2}, R^{h2}, R^{l2}, R^{m2}, R^{o2} und R^{u2} Wasserstoff darstellen und R^{d2}, R^{g2}, Rⁱ², Rⁿ², R^{p2}, R^{q2}, R^{t2} und R^{v2} 6- bis 14-gliedriges Aryl, wahlweise substituiert mit einem oder mehreren aus W⁶ ausgewählten Substituenten, oder 5- bis 10-gliedriges Heteroaryl, das ein oder mehrere Heteroatome enthält, die aus Sauerstoff, Stickstoff und/oder Schwefel ausgewählt sind, wahlweise substituiert mit einem oder mehreren aus W⁶ ausgewählten Substituenten, darstellen;
jedes W¹ unabhängig Halogen, -CN, -N(R^{f3})R^{g3}, -N(R^{h3})C(O)Rⁱ³, -N(Rⁱ³)C(O)OR^{k3}, -N(R^{l3})C(O)N(R^{m3})Rⁿ³, -N(R^{o3})S(O)₂R^{p3}, -OR^{q3}, -OC(O)R^{r3}, -OS(O)₂R^{s3}, -S(O)ₘR^{t3}, -S(O)₂N(R^{u3})R^{v3}, 3- bis 12-gliedriges Heterocycloalkyl, wahlweise substituiert mit einem oder mehreren aus G¹ ausgewählten Substituenten, 6- bis 14-gliedriges Aryl, wahlweise substituiert mit einem oder mehreren aus G² ausgewählten Substituenten, 5- bis 10-gliedriges Heteroaryl, das ein oder mehrere Heteroatome enthält, die aus Sauerstoff, Stickstoff und/oder Schwefel ausgewählt sind, wahlweise substituiert mit einem oder mehreren aus G² ausgewählten Substituenten, oder =O darstellt;
jedes W², W³ und W⁶ unabhängig Halogen, -R^{a3}, -CN, -C(O)R^{b3}, -C(O)N(R^{c3})R^{d3}, -C(O)OR^{e3}, -N(R^{f3})R^{g3}, -N(R^{h3})C(O)Rⁱ³, -N(R^{j3})C(O)OR^{k3}, -N(R^{l3})C(O)N(R^{m3})Rⁿ³, -N(R^{o3})S(O)₂R^{p3}, -OR^{q3}, -OC(O)R^{r3}, -OS(O)₂R^{s3}, -S(O)ₘR^{t3}, -S(O)₂N(R^{u3})R^{v3}, 3- bis 12-gliedriges Heterocycloalkyl, wahlweise substituiert mit einem oder mehreren aus G¹ ausgewählten Substituenten, 6- bis 14-gliedriges Aryl, wahlweise substituiert mit einem oder mehreren aus G² ausgewählten Substituenten, 5- bis 10-gliedriges Heteroaryl, das ein oder mehrere Heteroatome enthält, die aus Sauerstoff, Stickstoff und/oder Schwefel ausgewählt sind, wahlweise substituiert mit einem oder mehreren aus G² ausgewählten Substituenten, oder =O darstellt;
W⁷ und W¹ Wasserstoff darstellen;
jedes W⁸ und W¹⁰ unabhängig Halogen, -R^{a3}, -CN, -C(O)R^{b3}, -C(O)N(R^{c3})R^{d3}, -C(O)OR^{e3}, -N(R^{f3})R^{g3}, -N(R^{h3})C(O)Rⁱ³, -N(R^{j3})C(O)OR^{k3}, -N(R^{l3})C(O)N(R^{m3})Rⁿ³, -N(R^{o3})S(O)₂R^{p3}, -OR^{q3}, -OC(O)R^{r3}, -OS(O)₂R^{s3}, -S(O)ₘR^{t3}, -S(O)₂N(R^{u3})R^{v3}, 3- bis 12-gliedriges Heterocycloalkyl, wahlweise substituiert mit einem oder mehreren aus G¹ ausgewählten Substituenten, 6- bis 14-gliedriges Aryl, wahlweise substituiert mit einem oder mehreren aus G² ausgewählten Substituenten, oder 5- bis 10-gliedriges Heteroaryl, das ein oder mehrere Heteroatome enthält, die aus Sauerstoff, Stickstoff und/oder Schwefel ausgewählt sind, wahlweise substituiert mit einem oder mehreren aus G² ausgewählten Substituenten, darstellt;
W⁹ Halogen, -R^{a3}, -CN, -C(O)R^{b3}, -C(O)N(R^{c3})R^{d3}, -C(O)OR^{e3}, -N(R^{f3})R^{g3}, -N(R^{h3})C(O)Rⁱ³, -N(Rⁱ³)C(O)OR^{k3}, -N(R^{l3})C(O)N(R^{m3})Rⁿ³, -N(R^{o3})S(O)₂R^{p3}, -OR^{q3x}, -OC(O)R^{r3}, -OS(O)₂R^{s3}, -S(O)ₘR^{t3}, -S(O)₂N(R^{u3})R^{v3}, 3- bis 12-gliedriges Heterocycloalkyl, wahlweise substituiert mit einem oder mehreren aus G¹ ausgewählten Substituenten, 6- bis 14-gliedriges Aryl, wahlweise substituiert mit einem oder mehreren aus G² ausgewählten Substituenten, oder 5- bis 10-gliedriges Heteroaryl, das ein oder mehrere Heteroatome enthält, die aus Sauerstoff, Stickstoff und/oder Schwefel ausgewählt sind, wahlweise substituiert mit einem oder mehreren aus G² ausgewählten Substituenten, darstellt;
jedes R^{a3}, R^{b3}, R^{c3}, R^{d3}, R^{e3}, R^{f3}, R^{h3}, Rⁱ³, R^{j3}, R^{l3}, R^{m3}, Rⁿ³, R^{o3}, R^{q3}, R^{r3}, R^{t3}, R^{u3} und R^{v3} unabhängig Wasserstoff oder -C₁₋₆-Alkyl, wahlweise substituiert durch ein oder mehrere G³, darstellt oder
beliebige zwei R^{c3} und R^{d3}, R^{f3} und R^{g3}, R^{m3} und Rⁿ³ und/oder R^{u3} und R^{v3} miteinander verbunden sind, um zusammen mit dem Stickstoffatom, an das sie gebunden sind, einen 4- bis 6-gliedrigen Ring zu bilden, wobei der Ring wahlweise ein weiteres Heteroatom enthält und wobei der Ring wahlweise durch ein oder mehrere G² substituiert ist;
jedes R^{g3}, R^{k3}, R^{p3} und R^{s3} unabhängig C₁₋₆-Alkyl, wahlweise substituiert durch ein oder mehrere G³, darstellt;
R^{q3x} C₂₋₆-Alkyl, wahlweise substituiert durch ein oder mehrere G³, darstellt;
jedes G¹ und G² unabhängig Halogen, -R^{a4}, -CN, -C(O)R^{b4}, -C(O)N(R^{C4})R^{D4}, -C(O)OR^{e4}, -N(R⁴)R^{g4}, -N(R^{h4})C(O)Rⁱ⁴, -N(R^{j4})C(O)OR^{k4}, -N(R¹⁴)C(O)N(R^{m4})Rⁿ⁴, -N(R^{o4})S(O)₂R^{p4}, -OR^{q4}, -OC(O)R⁴, -OS(O)₂R^{s4}, -S(O)ₘR^{t4}, -S(O)₂N(R^{u4})R^{v4}, oder =O darstellt;
G³ Halogen, -CN, -C(O)R^{b4}, -C(O)N(R^{c4})R^{d}4, -C(O)OR^{e4}, -N(R^{f4})R⁹⁴, -N(R^{h4})C(O)Rⁱ⁴, -N(R^{j4})C(O)OR^{k4}, -N(R^{l4})C(O)N(R^{m4})Rⁿ⁴, -N(R^{O4})S(O)₂R^{P4}, -OR^{q4}, -OC(O)R^{R4}, -OS(O)₂R^{s4} -S(O)ₘR^{t4} , -S(O)₂N(R^{u4})R^{v4}, oder =O darstellt;
jedes R^{a4}, R^{b4}, R^{c4}, R^{d4}, R^{e4}, R^{f4}, R^{h4}, R¹⁴, R^{j4}, R^{l4}, R^{m4}, Rⁿ⁴, R^{o4}, R^{q4}, R^{r4}, R^{t4}, R^{u4} und R^{v4} unabhängig Wasserstoff oder C₁₋₆-Alkyl, wahlweise substituiert durch ein oder mehrere -F, darstellt oder
beliebige zwei R^{c4} und R^{d4}, R^{f4} und R^{g4}, R^{m4} und Rⁿ⁴ und/oder R^{u4} und R^{v4} miteinander verbunden sind, um zusammen mit dem Stickstoffatom, an das sie gebunden sind, einen 3- bis 6-gliedrigen Ring zu bilden, wobei der Ring wahlweise durch ein oder mehrere -F, -CH₃, -CH₂CH₃, -CHF₂, -CF₃, -CH₂CF₃ oder =O; substituiert ist;
jedes R^{g4}, R^{k4}, R^{p4} and R^{s4} unabhängig C₁₋₆-Alkyl, wahlweise substituiert durch ein oder mehrere -F, darstellt und
jedes unabhängig 0, 1 oder 2 darstellt.

2. Verbindung nach Anspruch 1, wobei R¹ Heteroaryl darstellt.

3. Verbindung nach Anspruch 1, wobei R¹ 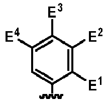 darstellt, wobei
E³ und E⁴ Wasserstoff darstellen und
E¹ -F darstellt und E² -F, -Cl, -CH₃ oder -CF₃ darstellt oder
E¹ -Cl darstellt und E² -F, -Cl, -CH₃ oder -CF₃ darstellt oder
E¹ -CH₃ darstellt und E²-F, -Cl, -CH₃, -CF₃ oder -CN darstellt;

4. Verbindung nach einem der Ansprüche 1 bis 3, wobei:
Z¹ 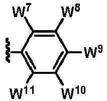 darstellt und
(a) W⁶ und W¹⁰ unabhängig aus Wasserstoff, -F, -Cl, -CH₃, -CN, und -CF₃ ausgewählt sind und
W⁹ aus -R^{a3}, -CN, -C(O)R^{b3}, -C(O)N(R^{c3})R^{d3}, -C(O)OR^{e3}, -N(R^{f3})R^{g3}, -N(R^{h3})C(O)Rⁱ³, -N(R^{j3})C(O)OR^{k3}, -N(R³)C(O)N(R^{m3})Rⁿ³, -N(R^{o3})S(O)₂R^{p3}, -OR^{q3x}, -OC(O)R³, -OS(O)₂R^{s3}, -S(O)ₘR^{t3}, -S(O)₂N(R^{u3})R^{v3}, 3- bis 12-gliedrigem Heterocycloalkyl, wahlweise substituiert mit einem oder mehreren aus G¹ ausgewählten Substituenten, 6- bis 14-gliedrigem Aryl, wahlweise substituiert mit einem oder mehreren aus G² ausgewählten Substituenten, oder 5- bis 10-gliedrigem Heteroaryl, das ein oder mehrere Heteroatome enthält, die aus Sauerstoff, Stickstoff und/oder Schwefel ausgewählt sind, wahlweise substituiert mit einem oder mehreren aus G² ausgewählten Substituenten, ausgewählt ist oder
(b) W⁹ und W¹⁰ unabhängig aus Wasserstoff, -F, -Cl, -CH₃, -CN, und -CF₃ ausgewählt sind und W⁸ aus -R^{a3}, -CN, -C(O)R^{b3}, -C(O)N(R^{e3})R^{d3}, -C(O)OR^{e3}, -N(R^{f3})R^{g3}, -N(R^{h3})C(O)Rⁱ³, -N(R^{j3})C(O)OR^{k3}, -N(R³)C(O)N(R^{m3})Rⁿ³, -N(R^{o3})S(O)₂R^{p3}, -OR^{q3x}, -OC(O)R³, -OS(O)₂R^{s3}, -S(O)ₘR^{t3}, -S(O)₂N(R^{u3})R^{v3}, wahlweise substituiert mit einem oder mehreren aus G¹ ausgewählten Substituenten, 6- bis 14-gliedrigem Aryl, wahlweise substituiert mit einem oder mehreren aus G² ausgewählten Substituenten, oder 5- bis 10-gliedrigem Heteroaryl, das ein oder mehrere Heteroatome enthält, die aus Sauerstoff, Stickstoff und/oder Schwefel ausgewählt sind, wahlweise substituiert mit einem oder mehreren aus G² ausgewählten Substituenten, ausgewählt ist.

5. Verbindung nach einem der Ansprüche 1 bis 4, wobei:
W⁶ und W¹⁰ Wasserstoff darstellen und W⁹ -F, -Cl, -CH₃, Cyclopropyl, -CF₃, -CN, -NH₂, -N(CH₃)₂, -N(H)C(O)CH₃, -N(H)C(O)OC(CH₃)₃,-SO₂CH₃, -SO₂NH₂, -S(O)₂N(CH₃)₂, -S(O)₂-4-Morpholinyl, 4-Methylpiperazin-1-yl, 4-Methylpiperidin-1-ylmethyl oder 1,2,3-Thiadiazol-4-yl darstellt.

6. Verbindung nach einem der Ansprüche 1 bis 3, wobei:
Z¹-N(R^{f2})R^{g2} darstellt, R^{f2} Wasserstoff darstellt und R^{g2} 6- bis 14-gliedriges Aryl, wahlweise substituiert mit einem oder mehreren aus W⁶ ausgewählten Substituenten, oder 5- bis 10-gliedriges Heteroaryl, das ein oder mehrere Heteroatome enthält, die aus Sauerstoff, Stickstoff und/oder Schwefel ausgewählt sind, wahlweise substituiert mit einem oder mehreren aus W⁶ ausgewählten Substituenten, darstellt.

7. Verbindung nach einem der Ansprüche 1 bis 3, wobei:
Z¹-OR^{q2} darstellt und R^{q2} 6- bis 14-gliedriges Aryl, wahlweise substituiert mit einem oder mehreren aus W⁶ ausgewählten Substituenten, oder 5- bis 10-gliedriges Heteroaryl, das ein oder mehrere Heteroatome enthält, die aus Sauerstoff, Stickstoff und/oder Schwefel ausgewählt sind, wahlweise substituiert mit einem oder mehreren aus W⁶ ausgewählten Substituenten, darstellt.

8. Verbindung nach einem der Ansprüche 1, 2, 3, 6, oder 7, wobei:
W⁶ represents -F, -Cl, -Br, -CH₃, cyclopropyl, -CF₃, -CN, -N(CH₃)₂, -SO₂CH₃, -SO₂NH₂ oder -SO₂N(CH₃)₂ darstellt.

9. Verbindung nach Anspruch 1, ausgewählt aus:
6-(2,3-Dimethylphenyl)-4-N-(2-phenylethyl)pyrimidin-2,4-diamin,
6-(2,3-Dimethylphenyl)-4-N-[2-(4-methoxyphenyl)ethyl]pyrimidin-2,4-diamin,
6-(2,3-Dimethylphenyl)-4-N-[2-(4-methylphenyl)ethyl]pyrimidin-2,4-diamin,
4-N-[2-(4-Chlorphenyl)ethyl]-6-(2,3-dimethylphenyl)pyrimidin-2,4-diamin,
6-(2,3-Dimethylphenyl)-4-N-(2-phenylpropyl)pyrimidin-2,4-diamin,
6-(2,3-Dimethylphenyl)-4-N-(3-phenylpropyl)pyrimidin-2,4-diamin,
6-(2,3-Dimethylphenyl)-4-N-(2-phenoxyethyl)pyrimidin-2,4-diamin,
6-(2,3-Dimethylphenyl)-4-N-[2-(phenylamino)ethyl]pyrimidin-2,4-diamin,
6-(2-Chlor-3-methylphenyl)-4-N-[2-(4-chlorphenyl)ethyl]pyrimidin-2,4-diamin,
4-N-[2-(4-Chlorphenyl)ethyl]-6-(1H-indol-4-yl)pyrimidin-2,4-diamin,
6-(3-Chlor-2-methylphenyl)-4-N-[(1R)-1-phenylethyl]pyrimidin-2,4-diamin,
6-(3-Chlor-2-methylphenyl)-4-N-(2-phenylpropan-2-yl)pyrimidin-2,4-diamin,
4-N-[2-(2-Chlorphenoxy)ethyl]-6-(2,3-dimethylphenyl)pyrimidin-2,4-diamin,
6-(2,3-Dimethylphenyl)-4-N-[2-(pyridin-3-yloxy)propyl]pyrimidin-2,4-diamin,
4-N-[2-(4-Chlorphenyl)ethyl]-6-(2,3-dichlorphenyl)pyrimidin-2,4-diamin,
4-N-[2-(4-Chlorphenyl)ethyl]-6-(chinolin-5-yl)pyrimidin-2,4-diamin,
4-N-[2-(4-Chlorphenyl)cyclopropyl]-6-(2,3-dimethylphenyl)pyrimidin-2,4-diamin,
3-(2-{[2-(2-Amino-6-(2,3-dimethylphenyl)pyrimidin-4-yl]amino}ethyl)phenol,
4-N-{[1-(4-Chlorphenyl)cyclopropyl]methyl}-6-(2,3-dimethylphenyl)pyrimidin-2,4-diamin,
4-(2-{[2-Amino-6-(2,3-dichlorphenyl)pyrimidin-4-yl]amino}ethyl)benzol-1-sulfonamid,
4-(2-{[2-Amino-6-(3-chlor-2-methylphenyl)pyrimidin-4-yl]amino}ethyl)benzol-1-sulfonamid,
4-(2-{[2-Amino-6-(2,3-dichlorphenyl)pyrimidin-4-yl]amino}ethyl)phenol,
4-(2-{[2-Amino-6-(2,3-dimethylphenyl)pyrimidin-4-yl]amino}ethyl)benzol-1-sulfonamid,
6-(2,3-Dimethylphenyl)-4-N-[2-(4-methansulfonylphenyl)ethyl]pyrimidin-2,4-diamin,
4-(2-{[2-Amino-6-(2,3-dimethylphenyl)pyrimidin-4-yl]amino}ethyl)benzonitril,
4-(2-{[2-Amino-6-(2,3-dimethylphenyl)pyrimidin-4-yl]amino}ethoxy)benzonitril,
4-N-[2-(3-Chlorphenyl)ethyl]-6-(2,3-dimethylphenyl)pyrimidin-2,4-diamin,
4-N-[2-(4-Chlorphenyl)propyl]-6-(2,3-dimethylphenyl)pyrimidin-2,4-diamin,
6-(2,3-Dimethylphenyl)-4-N-[2-(4-fluorphenyl)ethyl]pyrimidin-2,4-diamin,
4-N-[2-(Benzolsulfonyl)ethyl]-6-(2,3-dimethylphenyl)pyrimidin-2,4-diamin
N-(3-{[2-Amino-6-(2,3-dimethylphenyl)pyrimidin-4-yl]amino}propyl)-4-chlorbenzol-1-sulfonamid
3-(3-{[2-Amino-6-(2,3-dimethylphenyl)pyrimidin-4-yl]amino}propyl)-1-(4-chlorphenyl)harnstoff,
N-(3-{[2-Amino-6-(2,3-dimethylphenyl)pyrimidin-4-yl]amino}propyl)-3,5-dimethyl-1,2-oxazol-4-sulfonamid,
3-(3-{[2-Amino-6-(2,3-dimethylphenyl)pyrimidin-4-yl]amino}propyl)-1-(2,6-dichlorpyridin-4-yl)harnstoff,
3-(3-{[2-Amino-6-(2,3-dimethylphenyl)pyrimidin-4-yl]amino}propyl)-1-(3,4-difluorphenyl)harnstoff,
N-[3-(3-{[2-Amino-6-(2,3-dimethylphenyl)pyrimidin-4-yl]amino}propoxy)phenyl]acetamid,
N-(3-{[2-Amino-6-(2,3-dimethylphenyl)pyrimidin-4-yl]amino}propyl)-3-brombenzamid,
N-(3-{[2-Amino-6-(2,3-dimethylphenyl)pyrimidin-4-yl]amino}propyl)benzamid,
N-(3-{[2-Amino-6-(2,3-dimethylphenyl)pyrimidin-4-yl]amino}propyl)-3-cyanobenzol-1-sulfonamid,
N-(3-{[2-Amino-6-(2,3-dimethylphenyl)pyrimidin-4-yl]amino}propyl)-4-methansulfonylbenzol-1-sulfonamid,
N-(3-{[2-Amino-6-(2,3-dimethylphenyl)pyrimidin-4-yl]amino}propyl)benzolsulfonamid,
N-(3-{[2-Amino-6-(2,3-dimethylphenyl)pyrimidin-4-yl]amino}-2,2-dimethylpropyl)-3-fluorbenzol-1-sulfonamid,
N-(3-{[2-Amino-6-(2,3-dimethylphenyl)pyrimidin-4-yl]amino}-2,2-dimethylpropyl)-4-(morpholin-4-sulfonyl)benzol-1-sulfonamid,
N-(3-{[2-Amino-6-(2,3-dimethylphenyl)pyrimidin-4-yl]amino}propyl)-3-fluorbenzol-1-sulfonamid,
N-{4-[(3-{[2-Amino-6-(2,3-dimethylphenyl)pyrimidin-4-yl]amino}propyl)sulfamoyl]phenyl}acetamid,
N-(2-{[2-Amino-6-(2,3-dimethylphenyl)pyrimidin-4-yl]amino}ethyl)-3-fluorbenzol-1-sulfonamid,
N-(2-{[2-Amino-6-(2,3-dimethylphenyl)pyrimidin-4-yl]amino}ethyl)-4-methoxybenzol-1-sulfonamid,
N-(3-{[2-Amino-6-(2,3-dimethylphenyl)pyrimidin-4-yl]amino}propyl)-4,5-dichlorthiophen-2-sulfonamid,
N-(3-{[2-Amino-6-(2,3-dimethylphenyl)pyrimidin-4-yl]amino}propyl)-5-chlor-1,3-dimethyl-1H-pyrazol-4-sulfonamid,
N-(3-{[2-Amino-6-(2,3-dimethylphenyl)pyrimidin-4-yl]amino}propyl)-1,2-dimethyl-1H-imidazol-4-sulfonamid,
N-(2-{[2-amino-6-(2,3-dimethylphenyl)pyrimidin-4-yl]amino}ethyl)-5-chlor-1,3-dimethyl-1H-pyrazol-4-sulfonamid,
4-N-{2-[(1,3-Benzoxazol-2-yl)amino]ethyl}-6-(2,3-dimethylphenyl)pyrimidin-2,4-diamin,
6-(2,3-Dimethylphenyl)-4-N-(4-phenylbutan-2-yl)pyrimidin-2,4-diamin,
6-[(2-{[2-Amino-6-(2,3-dimethylphenyl)pyrimidin-4-yl]amino}ethyl)amino]pyridin-3-carbonitril,
4-N-{2-[(3-Brom-1,2,4-thiadiazol-5-yl)amino]ethyl}-6-(2,3-dimethylphenyl)pyrimidin-2,4-diamin,
N-(2-{[2-Amino-6-(2,3-dimethylphenyl)pyrimidin-4-yl]amino}ethyl)-4-methansulfonylbenzol-1-sulfonamid,
N-(3-{[2-Amino-6-(2,3-dimethylphenyl)pyrimidin-4-yl]amino}propyl)-3-methansulfonylbenzol-1-sulfonamid,
N-(3-{[2-Amino-6-(2,3-dimethylphenyl)pyrimidin-4-yl]amino}propyl)-2-methansulfonylbenzol-1-sulfonamid,
4-(2-{[2-Amino-6-(3-chlor-2-methylphenyl)pyrimidin-4-yl]amino}ethyl)-N,N-dimethylbenzol-1-sulfonamid,
4-(2-{[2-Amino-6-(2,3-dichlorphenyl)pyrimidin-4-yl]amino}ethyl)-N,N-dimethylbenzol-1-sulfonamid,
4-(2-{[2-Amino-6-(2,3-dimethylphenyl)pyrimidin-4-yl]amino}ethyl)-N,N-dimethylbenzol-1-sulfonamid,
4-N-{1-[(4-Chlorphenyl)methyl]cyclopropyl}-6-(2,3-dimethylphenyl)pyrimidin-2,4-diamin,
4-N-[2-(4-Chlorphenyl)ethyl]-6-(1H-Pyrrol-2-yl)pyrimidin-2,4-diamin,
N-(4-{[2-Amino-6-(2,3-dimethylphenyl)pyrimidin-4-yl]amino}butyl)-4-methansulfonylbenzol-1-sulfonamid,
4-(2-{[2-Amino-6-(3-cyano-2-methylphenyl)pyrimidin-4-yl]amino}ethyl)benzol-1-sulfonamid,
4-N-{2-[(6-Chlorpyridazin-3-yl)amino]ethyl}-6-(2,3-dimethylphenyl)pyrimidin-2,4-diamin,
2-[(2-{[2-Amino-6-(2,3-dimethylphenyl)pyrimidin-4-yl]amino}ethyl)amino]pyridin-4-carbonitril,
6-[(2-{[2-Amino-6-(2,3-dimethylphenyl)pyrimidin-4-yl]amino}ethyl)amino]pyridin-3-sulfonamid,
1-N-(3-{[2-Amino-6-(2,3-dimethylphenyl)pyrimidin-4-yl]amino}propyl)benzol-1,4-disulfonamid,
6-(2,3-Dichlorphenyl)-4-N-[2-(4-methansulfonylphenyl)ethyl]pyrimidin-2,4-diamin,
6-(2,3-Dimethylphenyl)-4-N-{2-[(3-methoxyphenyl)amino]ethyl}pyrimidin-2,4-diamin,
6-(2,3-Dimethylphenyl)-4-N-{2-[(3-fluor-4-methylphenyl)amino]ethyl}pyrimidin-2,4-diamin,
4-N-{2-[(3,4-Dichlorphenyl)amino]ethyl}-6-(2,3-dimethylphenyl)pyrimidin-2,4-diamin,
4-N-{2-[(5-Chlorpyridin-2-yl)amino]ethyl}-6-(2,3-dimethylphenyl)pyrimidin-2,4-diamin,
4-N-{2-[(5-Brompyridin-2-yl)amino]ethyl}-6-(2,3-dimethylphenyl)pyrimidin-2,4-diamin,
4-[(2-{[2-Amino-6-(2,3-dimethylphenyl)pyrimidin-4-yl]amino}ethyl)amino]benzol-1-sulfonamid und
6-(2,3-Dimethylphenyl)-4-N-{2-[(pyridin-3-yl)amino]ethyl}pyrimidin-2,4-diamin oder einem pharmazeutisch annehmbaren Salz davon.

10. Verbindung nach Anspruch 1, ausgewählt aus:
4-N-[2-(4-Chlorphenyl)ethyl]-6-(2,3-dichlorphenyl)pyrimidin-2,4-diamin,
4-(2-{[2-Amino-6-(2,3-dichlorphenyl)pyrimidin-4-yl]amino}ethyl)benzol-1-sulfonamid,
4-(2-{[2-Amino-6-(3-chlor-2-methylphenyl)pyrimidin-4-yl]amino}ethyl)benzol-1-sulfonamid,
4-(2-{[2-Amino-6-(2,3-dimethylphenyl)pyrimidin-4-yl]amino}ethyl)benzol-1-sulfonamid,
6-(2,3-Dimethylphenyl)-4-N-[2-(4-methansulfonylphenyl)ethyl]pyrimidin-2,4-diamin,
4-(2-{[2-Amino-6-(3-chlor-2-methylphenyl)pyrimidin-4-yl]amino}ethyl)-N,N-dimethylbenzol-1-sulfonamid,
4-(2-{[2-Amino-6-(2,3-dichlorphenyl)pyrimidin-4-yl]amino}ethyl)-N,N-dimethylbenzol-1-sulfonamid,
4-(2-{[2-Amino-6-(2,3-dimethylphenyl)pyrimidin-4-yl]amino}ethyl)-N,N-dimethylbenzol-1-sulfonamid,
6-[(2-{[2-Amino-6-(2,3-dimethylphenyl)pyrimidin-4-yl]amino}ethyl)amino]pyridin-3-sulfonamid,
6-(2,3-Dichlorphenyl)-4-N-[2-(4-methansulfonylphenyl)ethyl]pyrimidin-2,4-diamin und
4-[(2-{[2-Amino-6-(2,3-dimethylphenyl)pyrimidin-4-yl]amino}ethyl)amino]benzol-1-sulfonamid
oder einem pharmazeutisch annehmbaren Salz davon.

11. Pharmazeutische Formulierung, die eine Verbindung nach einem der Ansprüche 1 bis 10 oder ein pharmazeutisch annehmbares Salz davon im Gemisch mit einem pharmazeutisch annehmbaren Adjuvans, Verdünnungsmittel oder Träger umfasst.

12. Verbindung nach einem der Ansprüche 1 bis 10 oder ein pharmazeutisch annehmbares Salz davon zur Verwendung bei der Behandlung von Krebs.

13. Verbindung zur Verwendung nach Anspruch 12, wobei der Krebs ausgewählt ist aus Weichteilkrebs: Sarkom (Angiosarkom, Fibrosarkom, Rhabdomyosarkom, Liposarkom), Myxom, Rhabdomyom, Fibrom, Lipom und Teratom; Lungenkrebs/-krankheiten: bronchogenes Karzinom (Plattenepithel-, undifferenziert kleinzelliges, undifferenziert großzelliges, Adenokarzinom), Alveolarkarzinom (Bronchiolarkarzinom), Bronchialadenom, Sarkom, Lymphom, chondromatöses Hamartom, Mesotheliom; Magen-Darm-Karzinome/-Erkrankungen: Speiseröhre (Plattenepithelkarzinom, Adenokarzinom, Leiomyosarkom, Lymphom), Magen (Karzinom, Lymphom, Leiomyosarkom), Bauchspeicheldrüse (duktales Adenokarzinom, Insulinom, Glucagonom, Gastrinom, Karzinoidtumoren, VIPom), Dünndarm (Adenokarzinom, Lymphom, Karzinoidtumoren, Kaposi-Sarkom, Leiomyom, Hämangiom, Lipom, Neurofibrom, Fibrom), Dickdarm (Adenokarzinom, tubuläres Adenom, villöses Adenom, Hamartom, Leiomyom); Krebs/Erkrankungen des Urogenitaltraktes: Niere (Adenokarzinom, Wilms-Tumor [Nephroblastom], Lymphom, Leukämie), Blase und Harnröhre (Plattenepithelkarzinom, Übergangszellkarzinom, Adenokarzinom), Prostata (Adenokarzinom, Sarkom), Hoden (Seminom, Teratom, embryonales Karzinom, Teratokarzinom, Choriokarzinom, Sarkom, interstitielles Zellkarzinom, Fibrom, Fibroadenom, adenomatoide Tumore, Lipom); Leberkrebs/-erkrankungen: Hepatom (hepatozelluläres Karzinom), Cholangiokarzinom, Hepatoblastom, Angiosarkom, hepatozelluläres Adenom, Hämangiom; Knochenkrebs/-erkrankungen: osteogenes Sarkom (Osteosarkom), Fibrosarkom, malignes fibröses Histiozytom, Chondrosarkom, Ewing-Sarkom, malignes Lymphom (Retikulumzell-Sarkom), multiples Myelom, maligner Riesenzelltumor, Chordom, Osteochrondrom (osteokartilaginöse Exostosen), benignes Chondrom, Chondroblastom, Chondromyxofibrom, Osteoidosteom und Riesenzelltumore; Krebs/Erkrankungen des Nervensystems: Schädel (Osteom, Hämangiom, Granulom, Xanthom, Osteitis deformans), Hirnhäute (Meningiom, Meningiosarkom, Gliomatose), Gehirn (Astrozytom, Medulloblastom, Gliom, Ependymom, Germinom [Pinealom], Glioblastom multiform, Oligodendrogliom, Schwannom, Retinoblastom, angeborene Tumore), Rückenmarksneurofibrom, Meningiom, Gliom, Sarkom); gynäkologische Krebserkrankungen/Krankheiten: Gebärmutter (Endometriumkarzinom), Gebärmutterhals (Zervixkarzinom, Prä-Tumor-Zervixdysplasie), Eierstöcke (Ovarialkarzinom [seröses Zystadenokarzinom, mukinöses Zystadenokarzinom, nicht klassifiziertes Karzinom], Granulosa-Thekalzelltumore, Sertoli-Leydig-Zelltumore, Dysgerminom, malignes Teratom), Vulva (Plattenepithelkarzinom, intraepitheliales Karzinom, Adenokarzinom, Fibrosarkom, Melanom), Vagina (klarzelliges Karzinom, Plattenepithelkarzinom, Botryoidsarkom (embryonales Rhabdomyosarkom], Eileiter (Karzinom); hämatologische Krebserkrankungen/Krankheiten: Blut und Knochenmark (myeloische Leukämie [akut und chronisch], akute lymphoblastische Leukämie, chronische lymphatische Leukämie, myeloproliferative Erkrankungen, multiples Myelom, myelodysplastisches Syndrom), Morbus Hodgkin, Non-Hodgkin-Lymphom [malignes Lymphom]; Hautkrebs/-erkrankungen: malignes Melanom, Basalzellkarzinom, Plattenepithelkarzinom, Kaposi-Sarkom, Muttermale, dysplastische Nävi, Lipom, Angiom, Dermatofibrom, Keloide; Nebennierenkrebs/-erkrankungen, Neuroblastom, Neurofibromatose und Kopf- und Halskrebs.

14. Verbindung zur Verwendung nach Anspruch 12, wobei der Krebs aus der Gruppe bestehend aus Brustkrebs, Prostatakrebs, Lungenkrebs, Speiseröhrenkrebs, Dickdarmkrebs, Hirnkrebs, Hautkrebs, Eierstockkrebs, Hodenkrebs, Neurofibromatose und Leukämie ausgewählt ist.

15. Verbindung nach einem der Ansprüche 1 bis 10 oder ein pharmazeutisch annehmbares Salz davon zur Verwendung als Arzneimittel.

16. Kombinationsprodukt umfassend:
(A) eine Verbindung nach einem der Ansprüche 1 bis 10 oder ein pharmazeutisch annehmbares Salz davon und
(B) ein oder mehrere andere(s) therapeutische(s) Mittel, das/die bei der Behandlung einer Erkrankung, wie in Anspruch 13 oder 14 definiert, nützlich ist/sind,
wobei jede der Komponenten (A) und (B) im Gemisch mit einem pharmazeutisch annehmbaren Adjuvans, Verdünnungsmittel oder Träger formuliert ist.

17. Kombinationsprodukt nach Anspruch 16, wobei Komponente (B) ausgewählt ist aus der Gruppe bestehend aus Anti-Mikrotubuli-Agenzien, Platin-Koordinationskomplexen, Alkylierungsmitteln, Antibiotika, Topoisomerase-II-Inhibitoren, Antimetaboliten, Topoisomerase-I-Inhibitoren, Hormonen und hormonellen Analoga, Signaltransduktionsweg-Inhibitoren, Kinase-Inhibitoren, Angiogenese-Inhibitoren, immuntherapeutischen Mitteln, proapoptotischen Mitteln und Zellzyklus-Signalweg-Inhibitoren.

## Revendications

1. Composé de formule I 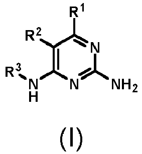 ou un sel pharmaceutiquement acceptable de celui-ci, dans lequel :
R¹ représente un hétéroaryle choisi parmi benzofuran-3-yle, benzothiophén-3-yle, dihydrobenzofuran-7-yle, indol-3-yle, indol-4-yle, indol-5-yle, isoquinolin-4-yle, pyridin-3-yle, pyridin-4-yle, pyrrol-2-yle et quinolin-5-yle, lequel hétéroaryle est relié à la pyrimidine de formule I via un atome de carbone du cycle hétéroaryle, et lequel hétéroaryle est éventuellement substitué par un ou plusieurs substituants choisis parmi Y¹ ou un alkyle en C₁ à C₆ éventuellement substitué par un ou plusieurs Y² ; ou un aryle représenté par 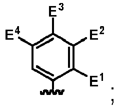
E³ et E⁴ représentent un hydrogène ; et
E¹ représente -F et E² représente -F, -Cl, -CH₃ ou -CF₃-; ou
E¹ représente -Cl et E² représente -F, -Cl, -CH₃ ou -CF₃ ; ou
E¹ représente -CH₃ et E² représente -F, -Cl, -CH₃, -CF₃ ou -CN ;
R² représente un hydrogène ;
R³ représente un alkyle en C₂ à C₆ substitué par Z¹ ;
chaque Y¹ représente indépendamment un halogène, -CN, -C(O)R^{a}, -C(O)N(R^{b})R^{c}, -C(O)OR^{d}, -N(R^{e})R^{f}, -N(R⁹)C(O)R^{h}, -N(Rⁱ)C(O)OR^{j}, -N(R^{k})C(O)N(R^{l})R^{m}, -NO₂, -N(Rⁿ)S(O)₂R^{o}, -OR^{p}, -OC(O)R^{q}, -OS(O)₂R^{r}, -S(O)ₘR^{s}, -S(O)₂N(R^{t})R^{u}, un hétérocycloalkyle à 3 à 12 chaînons éventuellement substitué par un ou plusieurs substituants choisis parmi W², un aryle à 6 à 14 chaînons éventuellement substitué par un ou plusieurs substituants choisis parmi W³, ou un hétéroaryle à 5 à 10 chaînons contenant un ou plusieurs hétéroatomes choisis parmi l'oxygène, l'azote et/ou le soufre, éventuellement substitué par un ou plusieurs substituants choisis parmi W³ ;
chaque R^{a}, R^{b}, R^{c}, R^{d}, R^{e}, R^{g}, R^{h}, Rⁱ, R^{k}, R^{l}, R^{m}, Rⁿ, R^{p}, R^{q}, R^{s}, R^{t} et R^{u} représente indépendamment un hydrogène, un alkyle en C₁ à C₆ éventuellement substitué par un ou plusieurs substituants choisis parmi W¹, hétérocycloalkyle à 3 à 12 chaînons éventuellement substitué par un ou plusieurs substituants choisis parmi W², aryle à 6 à 14 chaînons éventuellement substitué par un ou plusieurs substituants choisis parmi W³, ou hétéroaryle à 5 à 10 chaînons contenant un ou plusieurs hétéroatomes choisis parmi l'oxygène, l'azote et/ou le soufre, éventuellement substitué par un ou plusieurs substituants choisis parmi W³ ; ou
deux R^{b} et R^{c}, R^{e} et R^{f}, R^{l} et R^{m} et/ou R^{t} et R^{u} quelconques sont liés ensemble pour former, conjointement avec l'atome d'azote auquel ils sont rattachés, un cycle monocyclique ou bicyclique à 3 à 8 chaînons, lequel cycle contient éventuellement un ou deux autres hétéroatomes et lequel cycle est éventuellement substitué par un ou plusieurs substituants choisis parmi W², un alkyle en C₁ à C₃ éventuellement substitué par un ou plusieurs substituants choisis parmi W¹, et =O ;
chaque R^{f}, R^{j}, R^{o} et R^{r} représente indépendamment un alkyle en C₁ à C₆ éventuellement substitué par un ou plusieurs substituants choisis parmi W¹, un hétérocycloalkyle à 3 à 12 chaînons éventuellement substitué par un ou plusieurs substituants choisis parmi W², un aryle à 6 à 14 chaînons éventuellement substitué par un ou plusieurs substituants choisis parmi W³, ou un hétéroaryle à 5 à 10 chaînons contenant un ou plusieurs hétéroatomes choisis parmi l'oxygène, l'azote et/ou le soufre, éventuellement substitué par un ou plusieurs substituants choisis parmi W³ ;
chaque Y² représente indépendamment un halogène, -CN, -C(O)R^{b1}, -C(O)N(R^{c1})R^{d1}, -C(O)OR^{e1}, -N(R^{f1})R^{g1}, -N(R^{h1})C(O)Rⁱ¹, -N(Rⁱ¹)C(O)OR^{k1}, -N(R^{l1})C(O)N(R^{m1})Rⁿ¹, -N(R^{o1})S(O)₂R^{p1}, -OR^{q1}, -OC(O)R^{r1}, -OS(O)₂R^{s1}, -S(O)ₘR^{t1}, -S(O)₂N(R^{u1})R^{v1}, un hétérocycloalkyle à 3 à 12 chaînons éventuellement substitué par un ou plusieurs substituants choisis parmi W¹, un aryle à 6 à 14 chaînons éventuellement substitué par un ou plusieurs substituants choisis parmi W², un hétéroaryle à 5 à 10 chaînons contenant un ou plusieurs hétéroatomes choisis parmi l'oxygène, l'azote et/ou le soufre, éventuellement substitué par un ou plusieurs substituants choisis parmi W² ;
chaque R^{b1}, R^{c1}, R^{d1}, R^{e1}, R^{f1}, R^{h1}, Rⁱ¹, R^{j1}, R^{l1}, R^{m1}, Rⁿ¹, R^{o1}, R^{q1}, R^{r1}, R^{t1}, R^{u1} et R^{v1} représente indépendamment un hydrogène, un alkyle en C₁ à C₆ éventuellement substitué par un ou plusieurs substituants choisis parmi W¹, un hétérocycloalkyle à 3 à 12 chaînons éventuellement substitué par un ou plusieurs substituants choisis parmi W², un aryle à 6 à 14 chaînons éventuellement substitué par un ou plusieurs substituants choisis parmi W³, ou un hétéroaryle à 5 à 10 chaînons contenant un ou plusieurs hétéroatomes choisis parmi l'oxygène, l'azote et/ou le soufre, éventuellement substitué par un ou plusieurs substituants choisis parmi W³ ; ou
deux R^{c1} et R^{d1}, R^{f1} et R^{g1}, R^{m1} et Rⁿ¹ et/ou R^{u1} et R^{v1} quelconques sont liés ensemble pour former, conjointement avec l'atome d'azote auquel ils sont rattachés, un cycle monocyclique ou bicyclique à 3 à 8 chaînons, lequel cycle contient éventuellement un ou deux autres hétéroatomes et lequel cycle est éventuellement substitué par un ou plusieurs substituants choisis parmi W², un alkyle en C₁ à C₃ éventuellement substitué par un ou plusieurs substituants choisis parmi W¹, et =O ;
chaque R^{g1}, R^{k1}, R^{p1} et R^{s1} représente indépendamment un alkyle en C₁ à C₆ éventuellement substitué par un ou plusieurs substituants choisis parmi W¹, un hétérocycloalkyle à 3 à 12 chaînons éventuellement substitué par un ou plusieurs substituants choisis parmi W², un aryle à 6 à 14 chaînons éventuellement substitué par un ou plusieurs substituants choisis parmi W³, ou un hétéroaryle à 5 à 10 chaînons contenant un ou plusieurs hétéroatomes choisis parmi l'oxygène, l'azote et/ou le soufre, éventuellement substitué par un ou plusieurs substituants choisis parmi W³;
Z¹ représente -C(O)N(R^{e2})R^{d2}, -N(R^{f2})R⁹², -N(R^{h2})C(O)Rⁱ², -N(R¹²)C(O)N(R^{m2})Rⁿ², -N(R^{o2})S(O)₂R^{p2}, -OR^{q2}, -S(O)ₘR^{t2}, -S(O)₂N(R^{u2})R^{v2}, ou un aryle représenté par 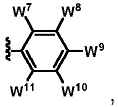
R^{c2}, R^{f2}, R^{h2}, R^{l2}, R^{m2}, R^{o2}, et R^{u2} représentent un hydrogène ; et
R^{d2}, R^{g2}, Rⁱ², Rⁿ² R^{p2}, R^{q2}, R^{t2} et R^{v2} représentent un aryle à 6 à 14 chaînons éventuellement substitué par un ou plusieurs substituants choisis parmi W⁶, ou un hétéroaryle à 5 à 10 chaînons contenant un ou plusieurs hétéroatomes choisis parmi l'oxygène, l'azote et/ou le soufre, éventuellement substitué par un ou plusieurs substituants choisis parmi W⁶ ;
chaque W¹ représente indépendamment un halogène, -CN, -N(R^{f3})R^{g3}, -N(R^{h3})C(O)Rⁱ³, -N(R^{j3})C(O)OR^{k3}, -N(R^{l3})C(O)N(R^{m3})Rⁿ³, -N(R^{o3})S(O)₂R^{p3}, -OR^{q3}, -OC(O)R³, -OS(O)₂R^{s3}, -S(O)ₘR^{t3}, -S(O)₂N(R^{u3})R^{v3}, un hétérocycloalkyle à 3 à 12 chaînons éventuellement substitué par un ou plusieurs substituants choisis parmi G¹, un aryle à 6 à 14 chaînons éventuellement substitué par un ou plusieurs substituants choisis parmi G², un hétéroaryle à 5 à 10 chaînons contenant un ou plusieurs hétéroatomes choisis parmi l'oxygène, l'azote et/ou le soufre, éventuellement substitué par un ou plusieurs substituants choisis parmi G², ou =O ;
chaque W², W³ et W⁶ représente indépendamment un halogène, -R^{a3}, -CN, -C(O)R^{b3}, -C(O)N(R^{c3})R^{d3}, -C(O)OR^{e3}, -N(R^{f3})R^{g3}, -N(Rⁿ³)C(O)Rⁱ³, -N(R^{j3})C(O)OR^{k3}, -N(R^{l3})C(O)N(R^{m3})Rⁿ³, -N(R^{o3})S(O)₂R^{p3}, -OR^{q3}, -OC(O)R³, -OS(O)₂R^{s3}, -S(O)ₘR^{t3}, -S(O)₂N(R^{u3})R^{v3}, un hétérocycloalkyle à 3 à 12 chaînons éventuellement substitué par un ou plusieurs substituants choisis parmi G¹, un aryle à 6 à 14 chaînons éventuellement substitué par un ou plusieurs substituants choisis parmi G², un hétéroaryle à 5 à 10 chaînons contenant un ou plusieurs hétéroatomes choisis parmi l'oxygène, l'azote et/ou le soufre, éventuellement substitué par un ou plusieurs substituants choisis parmi G², ou =O ;
W⁷ et W¹¹ représentent un hydrogène ;
chaque W⁸ et W¹⁰ représente indépendamment un halogène, -R^{a3}, -CN, -C(O)R^{b3} -C(O)N(R^{c3})R^{d3}, -C(O)OR^{e3}, -N(R^{f3})R^{g3}, -N(R^{h3})C(O)Rⁱ³, -N(Rⁱ³)C(O)OR^{k3}, -N(R^{l3})C(O)N(R^{m3})Rⁿ³, -N(R^{e3})S(O)₂R^{b3}, -OR^{q3}, -OC(O)R³, -OS(O)₂R^{s3}, -S(O)ₘR^{t3}, -S(O)₂N(R^{u3})R^{v3}, un hétérocycloalkyle à 3 à 12 chaînons éventuellement substitué par un ou plusieurs substituants choisis parmi G¹, un aryle à 6 à 14 chaînons éventuellement substitué par un ou plusieurs substituants choisis parmi G², ou un hétéroaryle à 5 à 10 chaînons contenant un ou plusieurs hétéroatomes choisis parmi l'oxygène, l'azote et/ou le soufre, éventuellement substitué par un ou plusieurs substituants choisis parmi G² ;
W⁹ représente un halogène, -R^{a3}, -CN, -C(O)R^{b3}, -C(O)N(R^{e3})R^{d3}, -C(O)OR^{e3}, -N(R^{f3})R^{g3}, -N(R^{h3})C(O)Rⁱ³, -N(R^{j3})C(O)OR^{k3}, -N(R^{l3})C(O)N(R^{m3})Rⁿ³, -N(R^{o3})S(O)₂R^{p3}, -OR^{q3x}, -OC(O)R^{r3}, -OS(O)₂R^{s3}, -S(O)ₘR^{t3}, -S(O)₂N(R^{u3})R^{v3}, un hétérocycloalkyle à 3 à 12 chaînons éventuellement substitué par un ou plusieurs substituants choisis parmi G¹, un aryle à 6 à 14 chaînons éventuellement substitué par un ou plusieurs substituants choisis parmi G², ou un hétéroaryle à 5 à 10 chaînons contenant un ou plusieurs hétéroatomes choisis parmi l'oxygène, l'azote et/ou le soufre, éventuellement substitué par un ou plusieurs substituants choisis parmi G² ;
chaque R^{a3}, R^{b3}, R^{c3}, R^{d3}, R^{e3}, R^{f3}, R^{h3}, Rⁱ³, R^{j3}, R^{l3}, R^{m3}, Rⁿ³, R^{o3}, R^{q3}, R^{r3}, R^{t3}, R^{u3} et R^{v3} représente indépendamment un hydrogène ou un alkyle en C₁ à C₆ éventuellement substitué par un ou plusieurs G³ ; ou
deux R^{c3} et R^{d3}, R^{f3} et R^{g3}, R^{m3} et Rⁿ³ et/ou R^{u3} et R^{v3} quelconques sont liés ensemble pour former, conjointement avec l'atome d'azote auquel ils sont rattachés, un cycle à 4 à 6 chaînons, lequel cycle contient éventuellement un autre hétéroatome et lequel cycle est éventuellement substitué par un ou plusieurs G² ;
chaque R^{g3}, R^{k3}, R^{p3} et R^{s3} représente indépendamment un alkyle en C₁ à C₆ éventuellement substitué par un ou plusieurs G³ ;
R^{q3x} représente un alkyle en C₂ à C₆ éventuellement substitué par un ou plusieurs G³;
chaque G¹ et G² représente indépendamment un halogène, -R^{a4}, -CN, -C(O)R^{b4}, -C(O)N(R^{c4})R^{d4}, -C(O)OR^{e4}, -N(R^{f4})R^{g4}, -N(R^{h4})C(O)Rⁱ⁴, -N(R^{j4})C(O)OR^{k4}, -N(R^{l4})C(O)N(R^{m4})Rⁿ⁴, -N(R^{o4})S(O)₂R^{p4}, -OR^{q4}, -OC(O)R^{r4}, -OS(O)₂R^{s4}, -S(O)ₘR^{t4}, -S(O)₂N(R^{u4})R^{v4}, ou =O ;
G³ représente un halogène, -CN, -C(O)R^{b4}, -C(O)N(R⁴)R^{d4} -C(O)OR^{e4}, -N(R^{f4})R^{g4}, -N(R^{h4})C(O)Rⁱ⁴, -N(R^{j4})C(O)OR^{k4}, -N(R^{l4})C(O)N(R^{m4})Rⁿ⁴, -N(R^{o4})S(O)₂R^{p4}, -OR^{q4}, -OC(O)R^{r4}, -OS(O)₂R^{s4}, -S(O)ₘR^{t4}, -S(O)₂N(R^{u4})R^{v4}, ou =O ;
chaque R^{a4}, R^{b4}, R^{c4}, R^{d4}, R^{e4}, R^{f4}, R^{h4}, Rⁱ⁴, R^{j4}, R^{l4}, R^{m4}, Rⁿ⁴, R^{o4}, R^{q4}, R^{r4}, R^{t4}, R^{u4} et R^{v4} représente indépendamment un hydrogène ou un alkyle en C₁ à C₆ éventuellement substitué par un ou plusieurs -F ; ou
deux R^{c4} et R^{d4}, R^{f4} et R^{g4}, R^{m4} et Rⁿ⁴ et/ou R^{u4} et R^{v4} quelconques sont liés ensemble pour former, conjointement avec l'atome d'azote auquel ils sont rattachés, un cycle à 3 à 6 chaînons, lequel cycle est éventuellement substitué par un ou plusieurs -F, -CH₃, -CH₂CH₃, -CHF₂, -CF₃, -CH₂CF₃, ou =O ;
chaque R^{g4}, R^{k4}, R^{p4} et R^{s4} représente indépendamment un alkyle en C₁ à C₆ éventuellement substitué par un ou plusieurs -F ; et
chaque m vaut indépendamment 0, 1 ou 2.

2. Composé selon la revendication 1, dans lequel R¹ représente un hétéroaryle.

3. Composé selon la revendication 1, dans lequel R¹ représente 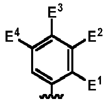 où :
E³ et E⁴ représentent un hydrogène ; et
E¹ représente -F et E² représente -F, -Cl, -CH₃ ou -CF₃ ; ou
E¹ représente -Cl et E² représente -F, -Cl, -CH₃ ou -CF₃ ; ou
E¹ représente -CH₃ et E² représente -F, -Cl, -CH₃, -CF₃ ou -CN.

4. Composé selon l'une quelconque des revendications 1 à 3, dans lequel :
Z¹ représente 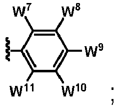 et
(a) W⁸ et W¹⁰ sont indépendamment choisis parmi un hydrogène, -F, -Cl, -CH₃, -CN et -CF₃ ; et
W⁹ est choisi parmi -R^{a3}, -CN, -C(O)R^{B3}, -C(O)N(R^{e3})R^{d3}, -C(O)OR^{e3}, -N/R^{f3})R^{g3}, -N(R^{h3})C(O)Rⁱ³, -N/R^{j3})C/O)OR^{k3}, -N(R¹³)C(O)N(R^{m3})Rⁿ³, -N(R^{o3})S(O)₂R^{p3}, -OR^{q3x}, -OC(O)R^{r3}, -OS(O)₂R^{s3}, -S(O)ₘR^{t3}, -S(O)₂N(R^{u3})R^{v3}, un hétérocycloalkyle à 3 à 12 chaînons éventuellement substitué par un ou plusieurs substituants choisis parmi G¹, un aryle à 6 à 14 chaînons éventuellement substitué par un ou plusieurs substituants choisis parmi G², ou un hétéroaryle à 5 à 10 chaînons contenant un ou plusieurs hétéroatomes choisis parmi l'oxygène, l'azote et/ou le soufre, éventuellement substitué par un ou plusieurs substituants choisis parmi G² ; ou
(b) W⁹ et W¹⁰ sont indépendamment choisis parmi un hydrogène, -F, -Cl, -CH₃, -CN, and -CF₃ ; et W⁸ est choisi parmi -R^{a3}, -CN, -C(O)R^{b3}, -C(O)N(R^{c3})R^{d3}, -C(O)OR^{e3}, -N(R^{f3})R^{g3}, -N(R^{h3})C(O)Rⁱ³, -N(R^{j3})C(O)OR^{k3} -N(R^{l3})C(O)N(R^{m3})Rⁿ³, -N(R^{o3})S(O)₂R^{p3}, -OR^{q3x}, -OC(O)R^{r3}, -OS(O)₂R^{s3}, -S(O)ₘR^{t3}, -S(O)₂N(R^{u3})R^{v3}, un hétérocycloalkyle à 3 à 12 chaînons éventuellement substitué par un ou plusieurs substituants choisis parmi G¹, un aryle à 6 à 14 chaînons éventuellement substitué par un ou plusieurs substituants choisis parmi G², ou un hétéroaryle à 5 à 10 chaînons contenant un ou plusieurs hétéroatomes choisis parmi l'oxygène, l'azote et/ou le soufre, éventuellement substitué par un ou plusieurs substituants choisis parmi G².

5. Composé selon l'une quelconque des revendications 1 à 4, dans lequel :
W⁸ et W¹⁰ représentent un hydrogène et W⁹ représente -F, -Cl, - CH₃, cyclopropyle, -CF₃, -CN, -NH₂, -N(CH₃)₂, -N(H)C(O)CH₃, -N(H)C(O)OC(CH₃)₃, -SO₂CH₃, -SO₂NH₂, -S(O)₂N(CH₃)₂, -S(O)₂-4-morpholinyle, 4-méthylpipérazin-1-yle, 4-méthylpipéridin-1-ylméthyle ou 1,2,3-thiadiazol-4-yle.

6. Composé selon l'une quelconque des revendications 1 à 3, dans lequel :
Z¹ représente -N(R^{f2})R^{g2}, R^{f2} représente un hydrogène et R^{g2} représente un aryle à 6 à 14 chaînons éventuellement substitué par un ou plusieurs substituants choisis parmi W⁶, ou un hétéroaryle à 5 à 10 chaînons contenant un ou plusieurs hétéroatomes choisis parmi l'oxygène, l'azote et/ou le soufre, éventuellement substitué par un ou plusieurs substituants choisis parmi W⁶.

7. Composé selon l'une quelconque des revendications 1 à 3, dans lequel :
Z¹ représente -OR^{q2} et R^{q2} représente un aryle à 6 à 14 chaînons éventuellement substitué par un ou plusieurs substituants choisis parmi W⁶, ou un hétéroaryle à 5 à 10 chaînons contenant un ou plusieurs hétéroatomes choisis parmi l'oxygène, l'azote et/ou le soufre, éventuellement substitué par un ou plusieurs substituants choisis parmi W⁶.

8. Composé selon l'une quelconque des revendications 1, 2, 3, 6 ou 7, dans lequel :
W⁶ représente -F, -Cl, -Br, -CH₃, cyclopropyle, -CF₃, -CN, -N(CH₃)₂, -SO₂CH₃, -SO₂NH₂ ou -SO₂N(CH₃)₂.

9. Composé selon la revendication 1, choisi parmi :
6-(2,3-diméthylphényl)-4-N-(2-phényléthyl)pyrimidine-2,4-diamine ;
6-(2,3-diméthylphényl)-4-N-[2-(4-méthoxyphényl)éthyl]pyrimidine-2,4-diamine ;
6-(2,3-diméthylphényl)-4-N-[2-(4-méthylphényl)éthyl]pyrimidine-2,4-diamine ;
4-N-[2-(4-chlorophényl)éthyl]-6-(2,3-diméthylphényl)pyrimidine-2,4-diamine ;
6-(2,3-diméthylphényl)-4-N-(2-phénylpropyl)pyrimidine-2,4-diamine ;
6-(2,3-diméthylphényl)-4-N-(3-phénylpropyl)pyrimidine-2,4-diamine ;
6-(2,3-diméthylphényl)-4-N-(2-phénoxyéthyl)pyrimidine-2,4-diamine ;
6-(2,3-diméthylphényl)-4-N-[2-(phénylamino)éthyl]pyrimidine-2,4-diamine ;
6-(2-chloro-3-méthylphényl)-4-N-[2-(4-chlorophényl)éthyl]pyrimidine-2,4-diamine ;
4-N-[2-(4-chlorophényl)éthyl]-6-(1H-indol-4-yl)pyrimidine-2,4-diamine ;
6-(3-chloro-2-méthylphényl)-4-N-[(1R)-1-phényléthyl]pyrimidine-2,4-diamine ;
6-(3-chloro-2-méthylphényl)-4-N-(2-phénylpropan-2-yl)pyrimidine-2,4-diamine ;
4-N-[2-(2-chlorophénoxy)éthyl]-6-(2,3-diméthylphényl)pyrimidine-2,4-diamine ;
6-(2,3-diméthylphényl)-4-N-[2-(pyridin-3-yloxy)propyl]pyrimidine-2,4-diamine ;
4-N-[2-(4-chlorophényl)éthyl]-6-(2,3-dichlorophényl)pyrimidine-2,4-diamine ;
4-N-[2-(4-chlorophényl)éthyl]-6-(quinolin-5-yl)pyrimidine-2,4-diamine ;
4-N-[2-(4-chlorophényl)cyclopropyl]-6-(2,3-diméthylphényl)pyrimidine-2,4-diamine ;
3-(2-{[2-amino-6-(2,3-diméthylphényl)pyrimidin-4-yl]amino}éthyl)phénol ;
4-N-{[1-(4-chlorophényl)cyclopropyl]méthyl}-6-(2,3-diméthylphényl)pyrimidine-2,4-diamine ;
4-(2-{[2-amino-6-(2,3-dichlorophényl)pyrimidin-4-yl]amino}éthyl)benzène-1-sulfonamide ;
4-(2-{[2-amino-6-(3-chloro-2-méthylphényl)pyrimidin-4-yl]amino}éthyl)benzène-1-sulfonamide ;
4-(2-{[2-amino-6-(2,3-dichlorophényl)pyrimidin-4-yl]amino}éthyl)phénol ;
4-(2-{[2-amino-6-(2,3-diméthylphényl)pyrimidin-4-yl]amino}éthyl)benzène-1-sulfonamide ;
6-(2,3-diméthylphényl)-4-N-[2-(4-méthanesulfonylphényl)éthyl]pyrimidine-2,4-diamine ;
4-(2-{[2-amino-6-(2,3-diméthylphényl)pyrimidin-4-yl]amino}éthyl)benzonitrile ;
4-(2-{[2-amino-6-(2,3-diméthylphényl)pyrimidin-4-yl]amino}éthoxy)benzonitrile ;
4-N-[2-(3-chlorophényl)éthyl]-6-(2,3-diméthylphényl)pyrimidine-2,4-diamine ;
4-N-[2-(4-chlorophényl)propyl]-6-(2,3-diméthylphényl)pyrimidine-2,4-diamine ;
6-(2,3-diméthylphényl)-4-N-[2-(4-fluorophényl)éthyl]pyrimidine-2,4-diamine ;
4-N-[2-(benzènesulfonyl)éthyl]-6-(2,3-diméthylphényl)pyrimidine-2,4-diamine ;
N-(3-{[2-amino-6-(2,3-diméthylphényl)pyrimidin-4-yl]amino}propyl)-4-chlorobenzène-1-sulfonamide ;
3-(3-{[2-amino-6-(2,3-diméthylphényl)pyrimidin-4-yl]amino}propyl)-1-(4-chlorophényl)urée ;
N-(3-{[2-amino-6-(2,3-diméthylphényl)pyrimidin-4-yl]amino}propyl)-3,5-diméthyl-1,2-oxazole-4-sulfonamide ;
3-(3-{[2-amino-6-(2,3-diméthylphényl)pyrimidin-4-yl]amino}propyl)-1-(2,6-dichloropyridin-4-yl)urée ;
3-(3-{[2-amino-6-(2,3-diméthylphényl)pyrimidin-4-yl]amino}propyl)-1-(3,4-difluorophényl)urée ;
N-[3-(3-{[2-amino-6-(2,3-diméthylphényl)pyrimidin-4-yl]amino}propoxy)phényl]acétamide ;
N-(3-{[2-amino-6-(2,3-diméthylphényl)pyrimidin-4-yl]amino}propyl)-3-bromobenzamide ;
N-(3-{[2-amino-6-(2,3-diméthylphényl)pyrimidin-4-yl]amino}propyl)benzanide ;
N-(3-{[2-amino-6-(2,3-diméthylphényl)pyrimidin-4-yl]aminolpropyl)-3-cyanobenzène-1-sulfonamide ;
N-(3-{[2-amino-6-(2,3-diméthylphényl)pyrimidin-4-yl]amino}propyl)-4-méthanesulfonylbenzène-1-sulfonamide ;
N-(3-{[2-amino-6-(2,3-diméthylphényl)pyrimidin-4-yl]amino}propyl)benzènesulfonamide ;
N-(3-{[2-amino-6-(2,3-diméthylphényl)pyrimidin-4-yl]aminol-2,2-diméthylpropyl)-3-fluorobenzène-1-sulfonamide ;
N-(3-{[2-amino-6-(2,3-diméthylphényl)pyrimidin-4-yl]aminol-2,2-diméthylpropyl)-4-(morpholine-4-sulfonyl)benzène-1-sulfonamide ;
N-(3-{[2-amino-6-(2,3-diméthylphényl)pyrimidin-4-yl]amino}propyl)-3-fluorobenzène-1-sulfonamide ;
N-{4-[(3-{[2-amino-6-(2,3-diméthylphényl)pyrimidin-4-yl]amino}propyl)sulfamoyl]phényl}acétamide ;
N-(2-{[2-amino-6-(2,3-diméthylphényl)pyrimidin-4-yl]aminoléthyl)-3-fluorobenzène-1-sulfonamide ;
N-(2-{[2-amino-6-(2,3-diméthylphényl)pyrimidin-4-yl]aminoléthyl)-4-méthoxybenzène-1-sulfonamide ;
N-(3-{[2-amino-6-(2,3-diméthylphényl)pyrimidin-4-yl]amino}propyl)-4,5-dichlorothiophène-2-sulfonamide;
N-(3-{[2-amino-6-(2,3-diméthylphényl)pyrimidin-4-yl]amino}propyl)-5-chloro-1,3-diméthyl-1H-pyrazole-4-sulfonamide ;
N-(3-{[2-amino-6-(2,3-diméthylphényl)pyrimidin-4-yl]amino}propyl)-1,2-diméthyl-1H-imidazole-4-sulfonamide ;
N-(2-{[2-amino-6-(2,3-diméthylphényl)pyrimidin-4-yl]amino}éthyl)-5-chloro-1,3-diméthyl-1H-pyrazole-4-sulfonamide ;
4-N-{2-[(1,3-benzoxazol-2-yl)amino]éthyl}-6-(2,3-diméthylphényl)pyrimidine-2,4-diamine ;
6-(2,3-diméthylphényl)-4-N-(4-phénylbutan-2-yl)pyrimidine-2,4-diamine ;
6-[(2-{[2-amino-6-(2,3-diméthylphényl)pyrimidin-4-yl]amino}éthyl)amino]pyridine-3-carbonitrile,
4-N-{2-[(3-bromo-1,2,4-thiadiazol-5-yl)amino]éthyl}-6-(2,3-diméthylphényl)pyrimidine-2,4-diamine ;
N-(2-{[2-amino-6-(2,3-diméthylphényl)pyrimidin-4-yl]aminoléthyl)-4-méthanesulfonylbenzène-1-sulfonamide ;
N-(3-{[2-amino-6-(2,3-diméthylphényl)pyrimidin-4-yl]amino}propyl)-3-méthanesulfonylbenzène-1-sulfonamide ;
N-(3-{[2-amino-6-(2,3-diméthylphényl)pyrimidin-4-yl]aminolpropyl)-2-méthanesulfonylbenzène-1-sulfonamide ;
4-(2-{[2-amino-6-(3-chloro-2-méthylphényl)pyrimidin-4-yl]amino}éthyl)-N,N-diméthylbenzène-1-sulfonamide ;
4-(2-{[2-amino-6-(2,3-dichlorophényl)pyrimidin-4-yl]amino}éthyl)-N,N-diméthylbenzène-1-sulfonamide ;
4-(2-{[2-amino-6-(2,3-diméthylphényl)pyrimidin-4-yl]amino}éthyl)-N,N-diméthylbenzène-1-sulfonamide ;
4-N-{1-[(4-chlorophényl)méthyl]cyclopropyl}-6-(2,3-diméthylphényl)pyrimidine-2,4-diamine ;
4-N-[2-(4-chlorophényl)éthyl]-6-(1H-pyrrol-2-yl)pyrimidine-2,4-diamine ;
N-(4-{[2-amino-6-(2,3-diméthylphényl)pyrimidin-4-yl]amino}butyl)-4-méthanesulfonylbenzène-1-sulfonamide ;
4-(2-{[2-amino-6-(3-cyano-2-méthylphényl)pyrimidin-4-yl]amino}éthyl)benzène-1-sulfonamide ;
4-N-{2-[(6-chloropyridazin-3-yl)amino]éthyl}-6-(2,3-diméthylphényl)pyrimidine-2,4-diamine ;
2-[(2-{[2-amino-6-(2,3-diméthylphényl)pyrimidin-4-yl]amino}éthyl)amino]pyridine-4-carbonitrile,
6-[(2-{[2-amino-6-(2,3-diméthylphényl)pyrimidin-4-yl]amino}éthyl)amino]pyridine-3-sulfonamide ;
1-N-(3-{[2-amino-6-(2,3-diméthylphényl)pyrimidin-4-yl]amino}propyl)benzène-1,4-disulfonamide ;
6-(2,3-dichlorophényl)-4-N-[2-(4-méthanesulfonylphényl)éthyl]pyrimidine-2,4-diamine ;
6-(2,3-diméthylphényl)-4-N-{2-[(3-méthoxyphényl)amino]éthyl}pyrimidine-2,4-diamine ;
6-(2,3-diméthylphényl)-4-N-{2-[(3-fluoro-4-méthylphényl)amino]éthyl}pyrimidine-2,4-diamine ;
4-N-{2-[(3,4-dichlorophényl)amino]éthyl}-6-(2,3-diméthylphényl)pyrimidine-2,4-diamine ;
4-N-{2-[(5-chloropyridin-2-yl)amino]éthyl}-6-(2,3-diméthylphényl)pyrimidine-2,4-diamine ;
4-N-{2-[(5-bromopyridin-2-yl)amino]éthyl}-6-(2,3-diméthylphényl)pyrimidine-2,4-diamine ;
4-[(2-{[2-amino-6-(2,3-diméthylphényl)pyrimidin-4-yl]amino}éthyl)amino]benzène-1-sulfonamide ; et
6-(2,3-diméthylphényl)-4-N-{2-[(pyridin-3-yl)amino]éthyl}pyrimidine-2,4-diamine ;
ou un sel pharmaceutiquement acceptable de celui-ci.

10. Composé selon la revendication 1, choisi parmi :
4-N-[2-(4-chlorophényl)éthyl]-6-(2,3-dichlorophényl)pyrimidine-2,4-diamine ;
4-(2-{[2-amino-6-(2,3-dichlorophényl)pyrimidin-4-yl]amino}éthyl)benzène-1-sulfonamide ;
4-(2-{[2-amino-6-(3-chloro-2-méthylphényl)pyrimidin-4-yl]amino}éthyl)benzène-1-sulfonamide ;
4-(2-{[2-amino-6-(2,3-diméthylphényl)pyrimidin-4-yl]amino}éthyl)benzène-1-sulfonamide ;
6-(2,3-diméthylphényl)-4-N-[2-(4-méthanesulfonylphényl)éthyl]pyrimidine-2,4-diamine ;
4-(2-{[2-amino-6-(3-chloro-2-méthylphényl)pyrimidin-4-yl]amino}éthyl)-N,N-diméthylbenzène-1-sulfonamide ;
4-(2-{[2-amino-6-(2,3-dichlorophényl)pyrimidin-4-yl]amino}éthyl)-N,N-diméthylbenzène-1-sulfonamide ;
4-(2-{[2-amino-6-(2,3-diméthylphényl)pyrimidin-4-yl]amino}éthyl)-N,N-diméthylbenzène-1-sulfonamide ;
6-[(2-{[2-amino-6-(2,3-diméthylphényl)pyrimidin-4-yl]amino}éthyl)amino]pyridine-3-sulfonam ide ;
6-(2,3-dichlorophényl)-4-N-[2-(4-méthanesulfonylphényl)éthyl]pyrimidine-2,4-diamine ; et
4-[(2-{[2-amino-6-(2,3-diméthylphényl)pyrimidin-4-yl]amino}éthyl)amino]benzène-1-sulfonamide,
ou un sel pharmaceutiquement acceptable de celui-ci.

11. Formulation pharmaceutique comprenant un composé selon l'une quelconque des revendications 1 à 10, ou un sel pharmaceutiquement acceptable de celui-ci, en mélange avec un adjuvant, diluant ou véhicule pharmaceutiquement acceptable.

12. Composé selon l'une quelconque des revendications 1 à 10, ou un sel pharmaceutiquement acceptable de celui-ci, pour une utilisation dans le traitement d'un cancer.

13. Composé pour une utilisation selon la revendication 12, dans lequel le cancer est choisi parmi les cancers des tissus mous suivants : sarcome (angiosarcome, fibrosarcome, rhabdomyosarcome, liposarcome), myxome, rhabdomyome, fibrome, lipome et tératome ; cancers/maladies des poumons : carcinome bronchique (à cellules squameuses, à petites cellules indifférenciées, à grosses cellules indifférenciées, adénocarcinome), carcinome alvéolaire (bronchiolaire), adénome bronchique, sarcome, lymphome, hamartome chondromateux, mésothéliome ; cancers/maladies gastro-intestinaux/nales : œsophage (carcinome à cellules squameuses, adénocarcinome, léiomyosarcome, lymphome), estomac (carcinome, lymphome, léiomyosarcome), pancréas (adénocarcinome canalaire, insulinome, glucagonome, gastrinome, tumeurs carcinoïdes, vipome), intestin grêle (adénocarcinome, lymphome, tumeurs carcinoïdes, sarcome de Kaposi, léiomyome, hémangiome, lipome, neurofibrome, fibrome), gros intestin (adénocarcinome, adénome tubulaire, adénome villeux, hamartome, léiomyome) ; cancers/maladies des voies génito-urinaires : reins (adénocarcinome, tumeur de Wilm [néphroblastome], lymphome, leucémie), vessie et urètre (carcinome à cellules squameuses, carcinome à cellules de transition, adénocarcinome), prostate (adénocarcinome, sarcome), testicules (séminome, tératome, carcinome embryonnaire, tératocarcinome, choriocarcinome, sarcome, carcinome à cellules interstitielles, fibrome, fibroadénome, tumeurs adénomatoïdes, lipome) ; cancers/maladies du foie : hépatome (carcinome hépatocellulaire), cholangiocarcinome, hépatoblastome, angiosarcome, adénome hépatocellulaire, hémangiome ; cancers/maladies des os : sarcome ostéogénique (ostéosarcome), fibrosarcome, histiocytome fibreux malin, chondrosarcome, sarcome d'Ewing, lymphome malin (réticulosarcome), myélome multiple, chordome tumoral à cellules géantes malignes, ostéochondrome (exostoses ostéo-cartilagineuses), chondrome bénin, chondroblastome, chondromyxofibrome, ostéome ostéoïde et tumeurs à cellules géantes ; cancers/maladies du système nerveux : crâne (ostéome, hémangiome, granulome, xanthome, ostéite déformante), méninges (méningiome, méningiosarcome, gliomatose), cerveau (astrocytome, médulloblastome, gliome, épendymome, germinome [pinéalome], glioblastome multiforme, oligodendrogliome, schwannome, rétinoblastome, tumeurs congénitales), neurofibrome de la moelle épinière, méningiome, gliome, sarcome) ; cancers/maladies gynécologiques : utérus (carcinome endométrial), col (carcinome cervical, dysplasie cervicale prétumorale), ovaires (carcinome ovarien [cystadénocarcinome séreux, cystadénocarcinome colloïde, carcinome non classé], folliculothécomes, tumeurs à cellules de Sertoli-Leydig, dysgerminome, tératome malin), vulve (carcinome à cellules squameuses, carcinome intraépithélial, adénocarcinome, fibrosarcome, mélanome), vagin (carcinome à cellules claires, carcinome à cellules squameuses, sarcome botryoïde (rhabdomyosarcome embryonnaire], trompes de Fallope (carcinome) ; cancers/maladies hématologiques : sang et moelle osseuse (leucémie myéloïde [aiguë et chronique], leucémie lymphoblastique aiguë, leucémie lymphocytaire chronique, maladies myéloprolifératives, myélome multiple, syndrome myélodysplasique), maladie de Hodgkin, lymphome non-Hodgkinien [lymphome malin] ; cancers/maladies de la peau : mélanome malin, carcinome à cellules basales, carcinome à cellules squameuses, sarcome de Kaposi, naevus dysplasique de grain de beauté, lipome, angiome, dermatofibrome, chéloïdes ; cancers/maladies des glandes surrénales, neuroblastome, neurofibromatose et cancers de la tête et du cou.

14. Composé pour une utilisation selon la revendication 12, dans lequel le cancer est choisi dans le groupe constitué par les cancers du sein, les cancers de la prostate, les cancers du poumon, les cancers de l'œsophage, les cancers du côlon, les cancers du cerveau, les cancers de la peau, les cancers ovariens, les cancers testiculaires, les neurofibromatoses et les leucémies.

15. Composé selon l'une quelconque des revendications 1 à 10, ou un sel pharmaceutiquement acceptable de celui-ci, pour une utilisation en tant qu'agent pharmaceutique.

16. Produit combiné comprenant :
(A) un composé selon l'une quelconque des revendications 1 à 10, ou un sel pharmaceutiquement acceptable de celui-ci ; et
(B) un ou plusieurs autres agents thérapeutiques qui sont utiles dans le traitement d'une maladie telle que définie dans la revendication 13 ou 14,
dans lequel chacun des composants (A) et (B) est formulé en mélange avec un adjuvant, diluant ou véhicule pharmaceutiquement acceptable.

17. Produit combiné selon la revendication 16, dans lequel le composant (B) est choisi dans le groupe constitué par les agents anti-microtubules, les complexes de coordination du platine, les agents d'alkylation, les agents antibiotiques, les inhibiteurs de topoisomérase II, les antimétabolites, les inhibiteurs de topoisomérase I, les hormones et analogues hormonaux, les inhibiteurs de voie de transduction de signal ; les inhibiteurs de kinase ; les inhibiteurs d'angiogenèse ; les agents immunothérapeutiques ; les agents pro-apoptotiques ; et les inhibiteurs de signalisation de cycle cellulaire.
